# EUROPÄISCHE PATENTANMELDUNG

(11) **EP 0 845 532 A2**
(43) Veröffentlichungstag der Anmeldung: **03.06.1998**
(21) Anmeldenummer: 97120058.9
(22) Anmeldetag: 17.11.1997
(51) Int. Cl.: C12N 15/53, C12N 9/02, C12P 7/22, C12N 9/80

(54) **Syntheseenzyme für die Herstellung von Coniferylalkohol, Coniferylaldehyd, Ferulasäure, Vanillin und Vanillinsäure und deren Verwendung**

(30) Priorität: 29.11.1996 DE 19649655
(71) Anmelder: HAARMANN & REIMER GMBH, D-37601 Holzminden (DE)
(72) Erfinder: Steinbüchel, Alexander, Prof Dr., 48341 Altenberge (DE); Priefert, Horst, Dr., 48291 Telgte (DE); Rabenhorst, Jürgen, Dr., 37671 Höxter (DE)
(74) Vertreter: Petrovicki, Wolfgang, Dr.

(57) **Zusammenfassung**

Die vorliegende Erfindung betrifft Syntheseenzyme für die Herstellung von Coniferylalkohol, Coniferylaldehyd, Ferulasäure, Vanillin und Vanillinsäure, deren Verwendung bei der Herstellung von Coniferylalkohol, Coniferylaldehyd, Ferulasäure, Vanillin und Vanillinsäure, für diese Enzyme codierende DNA sowie mit dieser DNA transformierte Mikroorganismen.

## Beschreibung

Die vorliegende Erfindung betrifft Syntheseenzyme für die Herstellung von Coniferylalkohol, Coniferylaldehyd, Ferulasäure, Vanillin und Vanillinsäure, deren Verwendung bei der Herstellung von Coniferylalkohol, Coniferylaldehyd, Ferulasäure, Vanillin und Vanillinsäure, für diese Enzyme codierende DNA sowie mit dieser DNA transformierte Mikroorganismen.

Der erste Artikel der sich mit dem Abbau von Eugenol befaßt, stammt von Tadasa 1977 (Degradation of eugenol by a microorganism. Agric. Biol. Chem. 41, 925-929). In ihm wird der Abbau von Eugenol mit einem Bodenisolat, vermutlich Corynebacterium sp., beschrieben. Es wurden dabei Ferulasäure und Vanillin als intermediäre Abbauprodukte gefunden und der weitere Abbau über Vanillinsäure und Protocatechusäure postuliert.

1983 erschien von Tadasa und Kyahara (Initial Steps of Eugenol Degradation Pathway of a Microorganism. Agric. Biol. Chem. 47, 2639-2640) ein weiterer Artikel, über die ersten Schritte des Eugenolabbaus; diesmal mit einem Bodenisolat, das als Pseudomonas sp. identifiziert wurde. In ihm wurden Eugenoloxid, Coniferyalkohol und Coniferylaldehyd als Zwischenstufen zur Bildung von Ferulasäure beschrieben.

Ebenfalls 1983 erschien von Sutherland et al. (Metabolism of cinnamic, p-coumaric, and ferulic acids by Streptomyces setonii. Can. J. Microbiol. 29, 1253-1257) ein Bericht über den Metabolismus von Zimtsäure, p-Coumarsäure und Ferulasäure in Streptomyces setonii. Dabei wird Ferulasäure über Vanillin, Vanillinsaure und Protocatechusäure abgebaut. Dabei wurden die ringspaltenden Enzyme Catechol 1,2-Dioxygenase und Protocatechuat 3,4-Dioxygenase im zellfreien Extrakt indirekt nachgewiesen.

Ötük (Degradation of Ferulic Acid by Escherichia coli. J. Ferment. Technol. 63, 501-506) berichtete 1985 über den Abbau von Ferulasäure mit einem Escherichia coli Stamm, der von verrottender Rinde isoliert wurde. Auch hier wurden Vanillin, Vanillinsaure und Protocatechusäure als Abbauprodukte nachgewiesen.

1987 erschien eine deutsche Patentanmeldung der BASF (Verfahren zur Gewinnung von Coniferylaldehyd und Mikroorganismus dafür; DE-A 3 606 398) für ein Verfahren zur Herstellung von Coniferylaldehyd aus Eugenol mit einer Arthrobacter globiformis Mutante. Dabei war das Ziel die Gewinnung von natürlichem Vanillin.

Abraham et al. (Microbial transformations of some terpenoids and natural compounds. in: Bioflavour '87, pp 399-413) berichten auf der Bioflavor '87 über die Verstoffwechslung von Eugenol mit verschiedenen Mikroorganismen. Dabei wurden bei der Verwendung von Pilzen vor allem Dimere gefunden, und nur bei Verwendung von Isoeugenol bildet Aspergillus niger ATCC 9142 auch Vanillin.

1988 wurde von Omori et al. (Protocatechuic acid production from trans-ferulic acid by Pseudomonas sp. HF-1 mutants defective in protocatechuic acid catabolism. Appl. Microbiol. Biotechnol. 29, 497-500) ein Verfahren zur Gewinnung von Protocatechusäure mit einer Mutante einer Pseudomonas sp. HF-1 beschrieben. Als Zwischenprodukt wird nur Vanillinsäure erwähnt.

Der Metabolismus von Ferulasäure mit zwei Pilzen, Paecilomyces variotii und Pestalotia palmarum wurde 1989 von Rahouti et al. (Metabolism of ferulic acid by Paecilomyces variotii and Pestalotia palmarum. Appl. Environ. Microbiol. 55, 2391-2398) beschrieben. Dabei wurde der Abbau über 4-Vinylguajacol und Vanillin zur Vanillinsäure postuliert.

1990 erscheinen zwei japanische Patentanmeldung von Hasegawa über eine neue Pseudomonas sp. und ein Dioxygenase Enzym (Novel Pseudomonas sp. and dioxygenase enzyme. JP 2195-871:25.10.88-JP-267 284 (02.08.90) 09.03.89 as 055111), und über eine neue Methode zur Herstellung eines Aldehyds, wie z.B. Vanillin (A new method for the preparation of aldehyde e.g. vanillin. JP 2200-192:25.10.88-JP-267 285 (08.08.90) 09.03.89 as 055112). Dabei wird aber nicht von Eugenol ausgegangen, sondern von verschiedenen Edukten wie Isoeugenol und Coniferylalkohol. Es besteht auch keine Übereinstimmung zwischen der dort beanspruchten Dioxygenase und den hier beanspruchten Enzymen.

In (Production of natural vanillin by microbial oxidation of eugenol or isoeugenol. EP-A 405 197) wurden Bakterien der Gattungen Serratia, Enterobacter oder Klebsiella zur mikrobiellen Oxidation von Eugenol und Isoeugenol verwendet. Der Prozeß brachte aber nur mit Isoeugenol gute Umsetzungen, mit Eugenol lief er nur sehr schlecht.

1991 erschien die EP-A 453 368 (Production de vanilline par bioconversion de précurseurs benzeniques), bei der mit einem Basidiomyceten, Pycnoporus cinnabarinus CNCM I-937 und I-938, mit Vanillinsäure und Ferulasäure die Umsetzung zu Vanillin beobachtet wurde.

Takasago Perfumery Company erhielt 1992 ein japanisches Patent (Preparation of vanillin, coniferyl-alcohol and - aldehyde, fenilic acid and vanillyl alcohol - by culturing mutant belonging to Pseudomonas genus in presence of eugenol which is oxidatively decomposed; JP 05 227 980 21.02.1992) für die Herstellung von Vanillin, Coniferylalkohol, Coniferylaldehyd, Ferulasäure und Vanilylalkohol aus Eugenol mit einer Pseudomonas Mutante.

Ebenfalls 1992 wurde das US-Patent 5 128 253 von Labuda et al. (Kraft-Generals Foods) (Bioconversion Process for the production of vanillin) erteilt, in dem eine Biotransformation zur Herstellung von Vanillin beschrieben wird. Ausgangsmaterial ist auch hier Ferulasäure, verwendete Organismen sind Aspergillus niger, Rhodotorula glutinis und Corynebacterium glutamicum. Entscheidend dabei ist die Verwendung von Sulfhydryl-Komponenten (z.B. Dithiothreitol) im Medium. 1993 erscheint der Inhalt des Patents auch als Publikation (Microbial bioconversion process for the production of vanillin; Prog. Flavour Precursor Stud. Proc. Int. Conf. 1992, 477-482).

Die EP-A 542 348 (Process of preparation of phenylaldehydes) beschreibt ein Verfahren zur Herstellung von Phenylaldehyden mit dem Enzym Lipoxygenase. Substrate sind u.a. Eugenol und Isoeugenol. Wir haben versucht, das Verfahren mit Eugenol nachzuarbeiten, konnten aber die Umsetzungen nicht bestätigen.

Die DE-A 4 227 076 (Verfahren zur Herstellung substituierter Methoxyphenole und dafür geeigneter Mikroorganismus) beschreibt die Herstellung substituierter Methoxyphenole mit einer neuen Pseudomonas sp. Ausgangsmaterial ist hier Eugenol und die Produkte sind Ferulasäure, Vanillinsäure, Coniferylalkohol und Coniferylaldehyd.

Ebenfalls 1995 erscheint ein umfangreiches Review über die Biotransformationsmöglichkeiten mit Ferulasäure von Rosazza et al. (Biocatalytic transformation of ferulic acid: an abundant aromatic natural product; J. Ind. Microbiol 15, 457-471).

Die vorliegende Erfindung betrifft nun Syntheseenzyme für Coniferylalkohol, Coniferylaldehyd, Ferulasäure, Vanillin und Vanillinsäure aus Eugenol.

Syntheseenzyme gemäß der Erfindung sind beispielsweise die
a) Eugenol-Hydroxylase,
b) Coniferylalkohol-Dehydrogenase,
c) Coniferylaldehyd-Dehydrogenase,
d) Ferylasäuredeacylase und die
e) Vanillin-Dehydrogenase.

Weiterhin betrifft die Erfindung DNA codierend für die genannten Enzyme und Cosmidklone enthaltend diese DNA sowie Vektoren enthaltend diese DNA und Mikroorganismen transformiert mit der DNA bzw. den Vektoren. Sie betrifft auch die Verwendung der DNA zur Transformation von Mikroorganismen zur Herstellung von Coniferylalkohol, Coniferylaldehyd, Ferulasäure, Vanillin und Vanillinsäure. Die Erfindung betrifft auch Teilsequenzen dieser DNA sowie funktionelle Äquivalente. Unter funktionellen Äquivalenten sind solche Derivate zu verstehen, bei denen einzelne Nucleobasen ausgetauscht wurden (Wobbelaustausche), ohne die Funktion zu ändern. Auch auf Proteinebene können Aminosäuren ausgetauscht werden, ohne daß es eine Veränderung der Funktion zur Folge hat.

Ebenso betrifft die Erfindung die einzelnen Herstellungsschritte der Herstellung von Coniferylalkohol, Coniferylaldehyd, Ferulasäure, Vanillin und Vanillinsäure aus Eugenol, also konkret:
a) das Verfahren zur Herstellung von Coniferylalkohol aus Eugenol, das in Anwesenheit von Eugenolhydroxylase stattfindet;
b) das Verfahren zur Herstellung von Coniferylaldehyd aus Coniferylalkohol, das in Anwesenheit von Coniferylalkohol-Dehydrogenase stattfindet;
c) das Verfahren zur Herstellung von Ferulasäure aus Coniferylaldehyd, das in Anwesenheit von Coniferylaldehyd-Dehydrogenase stattfindet;
d) das Verfahren zur Herstellung von Vanillin aus Ferulasäure, das in Anwesenheit von Ferulasäuredeacylase stattfindet;
e) das Verfahren zur Herstellung von Vanillinsäure aus Vanillin, das in Anwesenheit von Vanillin-Dehydrogenase stattfindet.

Von dem Eugenol verwertenden Stamm Pseudomonas sp. HR 199 (DSM 7063) wurden nach NMG-Mutagenese Mutanten erhalten, die Defekte in einzelnen Schritten des Eugenol-Katabolismus aufweisen. Ausgehend von partiell EcoRI-verdauter Gesamt-DNA des Pseudomonas sp. HR 199 Wildtyps wurde eine Genbank in dem Cosmid pVK100 angelegt, welches über ein breites Wirtsspektrum verfügt und auch in Pseudomonaden stabil repliziert wird. Die Hybridcosmide wurden nach Verpackung in λ-Phagenpartikel nach E. coli S17-1 transduziert. Die Genbank umfaßte 1330 rekombinante E. coli S17-1 Klone. Das Hybridcosmid eines jeden Klons wurde konjugativ in zwei Eugenol-negative Mutanten (Mutanten 6164 und 6165) des Stammes Pseudomonas sp. HR 199 übertragen und auf eine mögliche Komplementationseigenschaft überprüft. Dabei wurden zwei Hybridcosmide (pE207 und pE115) identifiziert, deren Erhalt die Mutante 6165 wieder in die Lage versetzten, Eugenol zu verwerten. Ein Hybridcosmid (pE5-1) führte zur Komplementation der Mutante 6164.

Die komplementierende Eigenschaft der Plasmide pE207 und pE115 konnte auf ein 23 kbp EcoRI-Fragment (E230) zurückgeführt werden. Von diesem Fragment wurde eine physikalische Karte angefertigt und das Fragment wurde vollständig sequenziert. Auf einem 11,2 kbp HindIII-Subfragment (H110) wurden die Gene vanA und vanB lokalisiert, die für die Vanillat-Demethylase codieren. Ein weiterer offener Leserahmen (ORF) wies Homologie zur γ-Glutamylcystein Synthetase aus Escherichia coli auf. Zwischen diesem ORF und dem vanB-Gen wurde ein weiterer ORF identifiziert, der Homologie zu Formaldehyd-Dehydrogenasen aufwies. Zwei weitere ORF wiesen Homologien zur Cytochrom C- bzw. Flavoprotein-Untereinheit der p-Cresol Methylhydroxylase aus Pseudomonas putida auf und codieren im Stamm Pseudomonas sp. HR 199 für eine bisher noch nicht beschriebene Eugenol Hydroxylase, welche Eugenol, in Analogie zum Reaktionsmechanismus der p-Cresol Methylhydroxylase, über ein Chinon-Methid-Derivat zu Coniferylalkohol umsetzt. Zwischen den Genen der beiden Untereinheiten der Eugenol Hydroxylase wurde ein weiterer ORF unbekannter Funktion identifiziert. Auf einem 5.0 kbp HindIII-Subfragment (H50) wurde ein ORF identifiziert, der Homologie zur Lignostilben-α,β-Dioxygenase aufwies. Daneben wurde ein ORF identifiziert, welcher Homologie zu Alkohol-Dehydrogenasen aufwies. Auf einem 3,8 kbp HindIII/EcoRI-Subfragment wurde das Strukturgen vdh der Vanillin Dehydrogenase identifiziert. Stromaufwärts von diesem Gen wurde ein ORF mit Homologie zu Enoyl-CoA Hydratasen aus unterschiedlichen Organismen lokalisiert.

Die komplementierende Eigenschaft des Plasmids pE5-1 konnte auf den gemeinsamen Erhalt der 1.2 und 1.8 kbp EcoRI-Fragmente (E12 und E18) zurückgeführt werden. Fragment E 12 wurde vollständig, Fragment E 18 wurde teilweise sequenziert. Auf diesen Fragmenten wurde das Strukturgen cadh der Coniferylalkohol Dehydrogenase lokalisiert, welches eine EcoRI-Schnittstelle aufwies. Das Enzym wurde mittels chromatographischer Methoden aus der löslichen Fraktion des Rohextraktes auf Eugenol gewachsener Zellen von Pseudomonas sp. HR 199 isoliert. Von der bestimmten N-terminalen Aminosäuresequenz wurde eine Oligonukleotidsequenz abgeleitet. Eine entsprechende DNA-Sonde hybridisierte mit Fragment E12, auf welchem der den N-Terminus codierende Bereich des cadh Gens lokalisiert war.

Eine Eugenol- und Ferulasäure-negative Mutante (Mutante 6167) ließ sich durch den Erhalt eines 9,4 kbp EcoRI-Fragments (E 94) des Hybridcosmids pE5-1 komplementieren. Von diesem Fragment wurde eine physikalische Karte angefertigt. Die komplementierende Eigenschaft ließ sich auf ein 1,9 kbp EcoRI/HindIII-Subfragment eingrenzen. Dieses Fragment wies unvollständige ORF (erstreckten sich über die EcoRI- bzw. HindIII-Schnittstelle) mit Homologien zu Acetyl-CoA Acetyltransferasen unterschiedlicher Organismen bzw. mit der "Medium-chain acyl-CoA Synthetase" aus Pseudomonas oleovorans auf. Das Fragment E 94 wurde vollständig sequenziert. Stromabwärts des zuvor genannten ORFs befand sich ein ORF mit Homologie zu β-Ketothiolasen. In zentraler Lage auf Fragment E 94 wurde das Strukturgen der Coniferylaldehyd-Dehydrogenase (caldh) lokalisiert. Das Enzym wurde mittels chromatographischer Methoden aus der löslichen Fraktion des Rohextraktes auf Eugenol gewachsener Zellen von Pseudomonas sp. HR 199 isoliert.

Die konjugative Übertragung des Hybridcosmids pE207 in eine Vielzahl von Pseudomonas-Stämmen führte zur heterologen Expression der Gene vanA, vanB, vdh und der Eugenol-Hydroxylase-Gene in den erhaltenen Transkonjuganten. Ein Stamm wurde durch den Erhalt des Plasmids zum Wachstum mit Eugenol als C- und Energiequelle befähigt.

### Material und Methoden

**Wachstumsbedingungen der Bakterien.** Stämme von Escherichia coli wurden bei 37°C in Luria-Bertani (LB) oder M9-Mineralmedium (Sambrook, J.E.F. Fritsch und T. Maniatis. 1989. Molecular cloning: a laboratory manual. 2. Aufl., Cold Spring Harbor Laboratory Press, Cold Spring Harbor, New York) angezogen. Stamme von Pseudomonas sp. und Alcaligenes eutrophus wurden bei 30°C in Nutrient Broth (NB, 0,8 Gew.- %) oder in Mineralmedium (MM) (Schlegel, H.G. et al. 1961. Arch. Mikrobiol. 38: 209-222) angezogen. Ferulasäure, Vanillin, Vanillinsäure und Protocatechusäure wurden in Dimethylsulfoxid gelöst, und dem jeweiligen Medium in einer Endkonzentration von 0,1 Gew.-% zugesetzt. Eugenol wurde dem Medium direkt in einer Endkonzentration von 0,1Vol.-% zugesetzt, bzw. in den Deckel von MM-Agarplatten auf Filterpapier (Rundfilter 595, Schleicher & Schuell, Dassel, Deutschland) appliziert. Bei der Anzucht von Transkonjuganten von Pseudomonas sp. wurde Tetracyclin und Kanamycin in Endkonzentrationen von 25 µg/ml bzw. 300 µg/ml eingesetzt.

**Nitrosoguanidin-Mutagenese.** Die Nitrosoguanidin-Mutagenese von Pseudomonas sp. HR 199 wurde mit Modifikationen nach Miller (Miller, J.H. 1972. Experiments in molecular genetics. Cold Spring Harbor Laboratory, Cold Spring Harbor, New York) durchgeführt. An Stelle des Citrat-Puffers kam Kalium-Phosphat (KP)-Puffer (100 mM, pH 7,0) zum Einsatz. Die Endkonzentration von N-Methyl-N'-Nitro-N-Nitrosoguanidin betrug 200 µg/ml. Die erhaltenen Mutanten wurden hinsichtlich des Verlustes der Fähigkeit, Eugenol, Ferulasäure, Vanillin und Vanillinsäure als Wachstumssubstrate nutzen zu können, gescreent.

**Qualitativer und quantitativer Nachweis von Stoffwechselintermediaten in Kulturüberständen.** Kulturüberstände wurden direkt bzw. nach Verdünnung mit zweifach destilliertem Wasser mittels Hochdruck-Flüssigkeits-Chromatographie (Knauer-HPLC) analysiert. Die Chromatographie erfolgte an Nucleosil-100 C18 (7 µm, 250 x 4 mm). Als Lösungsmittel diente 0,1 Vol.-% Ameisensaure und Acetonitril.

**Reinigung der Coniferylalkohol-Dehydrogenase und der Coniferylaldehyd-Dehydrogenase.** Die Aufreinigungen erfolgten bei 4°C.

**Rohextrakt.** Auf Eugenol angezogene Zellen von Pseudomonas sp. HR 199 wurden in 10 mM Natriumphosphat-Puffer, pH 7,5 gewaschen, im gleichen Puffer resuspendiert und durch zweimalige Passage einer French-Presse (Amicon, Silver Spring, Maryland, USA) bei einem Druck von 1 000 psi aufgeschlossen. Das Zellhomogenat wurde einer Ultrazentrifugation (1 h, 100 000 x g, 4°C) unterzogen, wodurch die lösliche Fraktion des Rohextraktes als Überstand erhalten wurde.

**Anionenaustauschchromatographie an DEAE-Sephacel.** Die lösliche Fraktion des Rohextraktes wurde über Nacht gegen 10 mM Natriumphosphat-Puffer, pH 7,5 mit 100 mM NaCl dialysiert. Das Dialysat wurde auf eine mit 10 mM Natriumphosphat-Puffer, pH 7,5 mit 100 mM NaCl äquilibrierte DEAE-Sephacel-Säule (2,6 cm x 35 cm, Bettvolumen [BV]: 186 ml) mit einer Durchflußrate von 0,8 ml/min aufgetragen. Die Säule wurde mit zwei BV 10 mM Natriumphosphat-Puffer, pH 7,5 mit 100 mM NaCl gespült. Die Elution der Coniferylalkohol-Dehydrogenase (CADH) und der Coniferylaldehyd-Dehydrogenase (CALDH) erfolgte mit einem linearen Salzgradient von 100 bis 500 mM NaCl in 10 mM Natriumphosphat-Puffer, pH 7,5 (2 x 150 ml). Es wurden 5 ml-Fraktionen aufgefangen. Fraktionen mit hoher CADH- bzw. CALDH-Aktivität wurden zum jeweiigen DEAE-Pool vereinigt.

**Gelfiltrationschromatographie an Sephadex G200.** Der CADH-DEAE-Pool wurde in einer 50 ml Amicon Ultrafiltrationskammer über eine Diaflo Ultrafiltrationsmembran PM 30 (beide Fa. AMICON CORP., Lexington, USA) bei einem Druck von 290 kPa auf ein Volumen eingeengt, welches ca. 2 % des Sephadex G200-BV entsprach. Die eingeengte Proteinlösung wurde auf eine mit 10 mM Natriumphosphat-Puffer, pH 7,5 mit 100 mM NaCl äquilibrierte Sephadex G200-Säule (BV: 138 ml) aufgetragen und mit einer Flußrate von 0,2 ml/min mit dem gleichen Puffer eluiert. Es wurden 2 ml-Fraktionen aufgefangen. Fraktionen mit hoher CADH-Aktivität wurden zum Sephadex-G200-Pool vereinigt.

**Hydrophobe Interaktionschromatographie an Butyl-Sepharose 4B.** Der CADH-Sephadex-G200-Pool wurde auf 3 M NaCl eingestellt und anschließend auf eine mit 10 mM Natriumphosphat-Puffer, pH 7,5 mit 3 M NaCl äquilibrierte Butyl-Sepharose 4B-Säule (BV: 48 ml) aufgetragen (Flußrate: 0,5 ml/min). Die Säule wurde anschließend mit 2 BV 10 mM Natriumphosphat-Puffer, pH 7,5 mit 3 M NaCl gewaschen (Flußrate: 1,0 ml/min). Die Elution der CADH erfolgte mit einem linearen abfallenden NaCl-Gradienten von 3 bis 0 M NaCl in 10 mM Natriumphosphat-Puffer, pH 7,5 (2 x 50 ml). Es wurden 4 ml-Fraktionen aufgefangen. Fraktionen mit hoher CADH-Aktivität wurden zum HIC-Pool vereinigt und wie oben beschrieben eingeengt.

**Chromatographie an Hydroxylapatit.** Der CALDH-DEAE-Pool wurde in einer 50 ml Amicon Ultrafiltrationskammer über eine Diaflo Ultrafiltrationsmembran PM 30 (beide Fa. AMICON CORP., Lexington, USA) bei einem Druck von 290 kPa auf 10 ml eingeengt. Die eingeengte Proteinlösung wurde auf eine mit Puffer (10 mM NaCL in 10 mM Natriumphosphat-Puffer, pH 7,0) äquilibrierte Hydroxylapatit-Säule (BV: 80 ml) aufgetragen (Flußrate: 2 ml/min). Die Säule wurde anschließend mit 2,5 BV Puffer gewaschen (Flußrate: 2 ml/min). Die Elution der CALDH erfolgte mit einem linearen ansteigenden Natriumphosphat-Gradienten von 10 bis 400 mM NaP (jeweils mit 10 mM NaCL) (2 x 100 ml). Es wurden 10 ml-Fraktionen aufgefangen. Fraktionen mit hoher CALDH-Aktivität wurden zum CALDH-HA-Pool vereinigt.

**Gelfiltrationschromatographie an Superdex HR 200 10/30.** Der CALDH-HA-Pool wurde auf 200 µl eingeengt (Amicon Ultrariltrationskammer, Ultrafiltrationsmembran PM 30), und auf eine mit 10 mM Natriumphosphat-Puffer, pH 7,0 äquilibrierte Superdex HR 200 10/30-Säule (BV: 23,6 ml) aufgetragen. Die CALDH wurde mit einer Flußrate von 0,5 ml/min mit dem gleichen Puffer eluiert. Es wurden 250 µl-Fraktionen aufgefangen. Fraktionen mit hoher CALDH-Aktivität wurden zum CALDH-Superdex-Pool vereinigt.

**Bestimmung der Coniferylalkohol-Dehydrogenase-Aktivität.** Die Bestimmung der CADH-Aktivität erfolgte bei 30°C durch einen optisch enzymatischen Test nach Jaeger et al. (Jaeger, E., L. Eggeling und H. Sahm. 1982. Current Microbiology. 6: 333-336) mit Hilfe eines ZEISS PM 4 Spektralphotometers mit angeschlossenem TE-Wandler (beide Fa. ZEISS, Oberkochen, Deutschland) und Schreiber. Der Reaktionsansatz mit einem Volumen von 1 ml enthielt 0,2 mmol Tris/HCl (pH 9,0), 0,4 µmol Coniferylalkohol, 2 µmol NAD, 0,1 mmol Semicarbazid und Enzymlösung ("Tris"=Tris(hydroxymethyl)-aminomethan). Die Reduktion von NAD wurde bei λ = 340 nm verfolgt (ε = 6,3 cm²/µmol). Die Enzymaktivität wurde in Einheiten (U) angegeben, wobei 1 U der Enzymmenge entspricht, die 1 µmol Substrat pro Minute umsetzt. Die Proteinkonzentrationen in den Proben wurden nach Lowry et al. (Lowry, O.H., N.J. Rosebrough, A.L. Farr und R. J. Randall. 1951. J. Biol. Chem. 193: 265-275) bestimmt.

**Bestimmung der Coniferylaldehyd-Dehydrogenase-Aktivität.** Die Bestimmung der CALDH-Aktivität erfolgte bei 30°C durch einen optisch enzymatischen Test mit Hilfe eines ZEISS PM 4 Spektralphotometers mit angeschlossenem TE-Wandler (beide Fa. ZEISS, Oberkochen, Deutschland) und Schreiber. Der Reaktionsanstatz mit einem Volumen von 1 ml enthielt 10 mM Tris/HCl-Puffer (pH 8,8), 5,6 mM Coniferylaldehyd, 3 mM NAD und Enzymlösung. Die Oxidation von Coniferylaldehyd zu Ferulasäure wurde bei λ = 400 nm verfolgt (ε = 34 cm²/µmol). Die Enzymaktivität wurde in Einheiten (U) angegeben, wobei 1 U der Enzymmenge entspricht, die 1 µmol Substrat pro Minute umsetzt. Die Proteinkonzentration in den Proben wurden nach Lowry et al. (Lowry, O. H., N.J. Rosebrough, A.L. Farr und R.J. Randall. 1951, J. Biol. Chem. **193**:265-275) bestimmt.

**Electrophoretische Methoden.** Die Auftrennung von proteinhaltigen Extrakten erfolgte in 7,4 Gew.-% Polyacrylamidgelen unter nativen Bedingungen nach der Methode von Stegemann et al. (Stegemann et al. 1973. Z. Naturforsch. 28c: 722-732) und unter denaturierenden Bedingungen in 11,5 Gew.-% Polyacrylamidgelen nach der Methode von Laemmli (Laemmli, U.K. 1970. Nature (London) 227: 680-685). Zur unspezifischen Proteinfärbung wurde Serva Blue R verwendet. Zur spezifischen Anfärbung der Coniferylalkohol-, Coniferylaldehyd- und Vanillin-Dehydrogenase wurden die Gele für 20 min in 100 mM KP-Puffer (pH 7,0) umgepuffert und anschließend bei 30°C im gleichen Puffer, dem 0,08 Gew.-% NAD, 0,04 Gew.-% p-Nitroblau-Tetrazoliumchlorid, 0,003 Gew.-% Phenazine-Methosulfat und 1 mM des jeweiligen Substrates zugesetzt worden war, inkubiert, bis entsprechende Farbbanden sichtbar wurden.

**Transfer von Proteinen aus Polyacrylamidgelen auf PVDF-Membranen.** Proteine wurden aus SDS-Polyacrylamidgelen mit Hilfe eines Semidry-Fastblot Gerätes (B32/33, Biometra, Göttingen, Deutschland) nach Herstellerangaben auf PVDF-Membranen (Waters-Milipore, Bedford, Mass., USA) übertragen.

**Bestimmung von N-terminalen Aminosäuresequenzen.** Die Bestimmung von N-terminalen Aminosäuresequenzen erfolgte mit Hilfe eines Protein Peptide Sequenzers (Typ 477 A, Applied Biosystems, Foster City, USA) und eines PTH-Analysers nach Herstellerangaben.

**Isolierung und Manipulation von DNA.** Die Isolierung von genomischer DNA erfolgte nach der Methode von Marmur (Marmur, J. 1961. J. Mol. Biol. 3: 208-218). Megaplasmid-DNA wurde nach der Methode von Nies et al. (Nies, D., et al. 1987. J. Bacteriol. 169: 4865-4868) isoliert. Die Isolierung und Analyse von anderer Plasmid-DNA bzw. von DNA-Restriktionsfragmenten, die Verpackung von Hybridcosmiden in λ-Phagenpartikel und die Transduktion von E. coli erfolgte nach Standardmethoden (Sambrook, J.E.F. Fritsch und T. Maniatis. 1989. Molecular cloning: a laboratory manual. 2. Aufl., Cold Spring Harbor Laboratory Press, Cold Spring Habor, New York).

**Transfer von DNA.** Die Präparation und Transformation von kompetenten Escherichia coli-Zellen erfolgte nach der Methode von Hanahan (Hanahan, D. 1983. J. Mol. Biol. 166: 557-580). Konjugativer Plasmidtransfer zwischen Plasmid-tragenden Escherichia coli S17-1-Stämmen (Donor) und Pseudomonas sp.-Stämmen (Rezipient) bzw. Alcaligenes eutrophus (Rezipient) erfolgte auf NB-Agarplatten nach der Methode von Friedrich et al. (Friedrich, B. et al. 1981. J. Bacteriol. 147: 198-205) oder durch eine "Minikomplementations-Methode" auf MM-Agarplatten mit 0,5 Gew.-% Gluconat als C-Quelle und 25 µg/ml Tetracyclin oder 300 µg/ml Kanamycin. Dabei wurden Zellen des Rezipienten in einer Richtung als Impfstrich aufgetragen. Nach 5 min wurden dann Zellen der Donor-Stämme als Impfstriche aufgetragen, wobei der Rezipienten-Impfstrich gekreuzt wurde. Nach einer Inkubation für 48 h bei 30°C wuchsen die Transkonjuganten direkt hinter der Kreuzungsstelle, wohingegen weder Donor- noch Rezipienten-Stamm zum Wachstum in der Lage war.

**Hybridisierungsexperimente.** DNA-Restriktionsfragmente wurden in einem 0,8 Gew.-% Agarose-Gel in 50 mM Tris- 50 mM Borsäure- 1,25 mM EDTA-Puffer (pH 8,5) elektrophoretisch aufgetrennt (Sambrook, J.E.F. Fritsch und T. Maniatis. 1989. Molecular cloning: a laboratory manual. 2. Aufl., Cold Spring Harbor Laboratory Press, Cold Spring Habor, New York). Die Übertragung der denaturierten DNA aus dem Gel auf eine positiv geladene Nylonmembran (Porengröße: 0,45 µm, Pall Filtrationstechnik, Dreieich, Deutschland), die anschließende Hybridisierung mit biotinylierten bzw. ³²P-markierten DNA-Sonden und die Herstellung dieser DNA-Sonden erfolgten nach Standardmethoden (Sambrook, J.E.F. Fritsch und T. Maniatis. 1989. Molecular cloning: a laboratory manual. 2. Aufl. Cold Spring Harbor Laboratory Press, Cold Spring Habor, New York).

**Synthese von Oligonukleotiden.** Ausgehend von Desoxynukleosid-Phosphoramiditen wurden Oligonukleotide im 0,2 µmol-Maßstab synthetisiert (Beaucage, S. L., and M.H. Caruthers. 1981. Tetrahedron Lett. 22: 1859-1862). Die Synthese erfolgte in einem Gene Assembler Plus nach Herstellerangaben (Pharmacia-LKB, Uppsala, Schweden). Die Abspaltung von Schutzgruppen erfolgte durch eine 15 h Inkubation bei 55°C in 25 Vol.-% wäßriger Ammoniak-Lösung. Die Oligonukleotide wurden abschließend durch Chromatographie an einer NAP-5-Säule (Pharmacia-LKB, Uppsala, Schweden) gereinigt.

**DNA-Sequenzierung.** Die Bestimmung von Nukleotidsequenzen erfolgte nach der Didesoxy-Kettenabbruch-Methode von Sanger et al. (Sanger et al. 1977. Proc. Natl. Acad. Sci. USA 74: 5463-5467) mit [α-³⁵S]dATP und einem T7-Polymerase-Sequencing-Kit (Pharmacia-LKB). Dabei wurde 7-Desazaguanosin-5'-Triphosphat an Stelle von dGTP verwendet (Mizusawa, S. et al. 1986. Nucleic Acids Res. 14: 1319-1324). Die Produkte der Sequenzierreaktionen wurden in einem 6 Gew.-% Polyacrylamid-Gel in 100 mM Tris/HCl-, 83 mM Borsäure-, 1 mM EDTA-Puffer (pH 8,3) mit 42 Gew.-% Harnstoff aufgetrennt, wobei eine S2-Sequenzier-Apparatur (GIBCO/BRL, Bethesda Research Laboratories GmbH, Eggenstein, Deutschland) nach Vorschrift des Herstellers zum Einsatz kam. Nach der Elektrophorese wurden die Gele 30 min in 10 Vol.-% Essigsäure inkubiert und nach kurzem Spülen in Wasser für 2 h bei 80°C getrocknet. Für die Autoradiographie der getrockneten Gele fanden Kodak X-OMAT AR-Röntgenfilme (Eastman Kodak Company, Rochester, NY, USA) Verwendung. Daneben wurden DNA-Sequenzen auch "nicht-radioaktiv" mit einem "LI-COR DNA-Sequencer Modell 4000L" (LI-COR Inc., Biotechnology Division, Lincoln, NE-USA) unter Verwendung eines "Thermo Sequenase fluorescent labelled primer cycle sequencing kit with 7-deaza-dGTP" (Amersham Life Science, Amersham International plc, Little Chalfont, Buckinghamshire, England) jeweils nach Vorschrift des Herstellers bestimmt.

Es kamen unterschiedliche Sequenzierungsstrategien zur Anwendung: Mit Hilfe von synthetischen Oligonukleotiden wurde nach der "Primer-hopping Strategie" von Strauss et al. (Strauss, E. C. et al. 1986. Anal. Biochem. 154: 353-360) sequenziert. Bei ausschließlicher Verwendung von "Universal-" und "Reverse-Primer" kamen Hybridplasmide als "Template-DNA" zum Einsatz, deren Insert-DNA-Fragmente mit Hilfe eines "Exo III/Mung Bean Nuklease Deletions"-Kits (Stratagene Cloning Systems, La Jolla, Cal., USA) nach Herstellerangaben unidirektional verkürzt worden waren.

**Chemikalien, Biochemikalien und Enzyme.** Restriktionsenzyme, T4 DNA-Ligase, Lambda-DNA und Enzyme bzw. Substrate für die optisch enzymatischen Tests wurden von C. F. Boehringer & Söhne (Mannheim, Deutschland) oder von GIBCO/BRL (Eggenstein, Deutschland) bezogen. [α-³⁵S]dATP und [γ-³²P]ATP kam von Amersham/Buchler (Braunschweig, Deutschland). Agarose vom Typ NA wurde von Pharmacia-LKB (Uppsala, Schweden) bezogen. Alle anderen Chemikalien waren von Haarmann & Reimer (Holzminden, Deutschland), E. Merck AG (Darmstadt, Deutschland), Fluka Chemie (Buchs, Schweiz), Serva Feinbiochemica (Heidelberg, Deutschland) oder Sigma Chemie (Deisenhofen, Deutschland).

### Beispiele

### Beispiel 1

### Isolierung von Mutanten des Stammes Pseudomonas sp. HR 199 mit Defekten im Eugenol-Katabolismus

Der Stamm Pseudomonas sp. HR 199 wurde einer Nitrosoguanidin-Mutagenese unterzogen mit dem Ziel, Mutanten mit Defekten im Eugenol-Katabolismus zu isolieren. Die erhaltenen Mutanten wurden bezüglich ihres Vermögens, Eugenol, Ferulasäure und Vanillin als C- und Energiequelle nutzen zu können, klassifiziert. Die Mutanten 6164 und 6165 waren nicht mehr in der Lage, Eugenol als C- und Energiequelle zu nutzen, vermochten jedoch wie der Wildtyp, Ferulasäure und Vanillin zu verwerten. Die Mutanten 6167 und 6202 waren nicht mehr in der Lage, Eugenol und Ferulasäure als C- und Energiequelle zu nutzen, vermochten jedoch wie der Wildtyp, Vanillin zu verwerten. Die obengenannten Mutanten kamen bei den weiteren molekularbiologischen Analysen zum Einsatz.

### Beispiel 2

### Anlegen einer Pseudomonas sp. HR 199 Genbank im Cosmidvektor pVK100

Die genomische DNA des Stammes Pseudomonas sp. HR 199 wurde isoliert und einer partiellen Restriktionsverdauung mit EcoRI unterzogen. Die so erhaltene DNA-Präparation wurde mit EcoRI-geschnittenem Vektor pVK100 ligiert. Die DNA-Konzentrationen lagen dabei relativ hoch, um die Entstehung konkatemerer Ligationsprodukte zu forcieren. Die Ligationsansätze wurden in λ-Phagenpartikel verpackt, mit denen anschließend E. coli S17-1 transduziert wurde. Die Selektion der Transduktanten erfolgte auf Tetracyclin-haltigen LB-Agarplatten. Auf diese Weise wurden 1330 Transduktanten erhalten, die über unterschiedliche Hybridcosmide verfügten.

### Beispiel 3

### Identifizierung von Hybridcosmiden, die essentielle Gene des Eugenol-Katabolismus beherbergen

Die Hybridcosmide der 1330 Transduktanten wurden durch ein Minikomplementations-Verfahren konjugativ in die Mutanten 6164 und 6165 übertragen. Die erhaltenen Transkonjuganten wurden auf MM-Platten mit Eugenol bezüglich ihres Vermögens, wieder auf Eugenol wachsen zu können (Komplementation der jeweiligen Mutante), untersucht. Die Mutante 6164 wurde durch den Erhalt des Hybridcosmids pE5-1 komplementiert, in welchem ein 1,2 kbp, ein 1,8 kbp, ein 3 kbp, ein 5,8 kbp und ein 9,4 kbp EcoRI-Fragment kloniert vorlag. Der dieses Hybridcosmid pE5-1 tragende E. coli S17-1-Stamm wurde bei der Deutschen Sammlung von Mikroorganismen und Zellkulturen GmbH (DSM) unter der Nummer DSM 10440 hinterlegt. Die Mutante 6165 wurde jeweils durch den Erhalt der Hybridcosmide pE207 und pE115 komplementiert. Die komplementierende Eigenschaft war auf ein 23 kbp EcoRI-Fragment zurückzuführen, welches in dem Hybridcosmid pE207 als alleiniges EcoRI-Fragment kloniert vorlag, wohingegen in dem Hybridcosmid pE115 zusätzlich noch ein 3 kbp und ein 6 kbp EcoRI-Fragment enthalten war. Der das Hybridcosmid pE207 tragende E. coli S17-1-Stamm wurde bei der DSM unter der Nummer DSM 10439 hinterlegt.

### Beispiel 4

### Analyse des 23 kbp EcoRI-Fragments (E230) des Hybridcosmids pE207

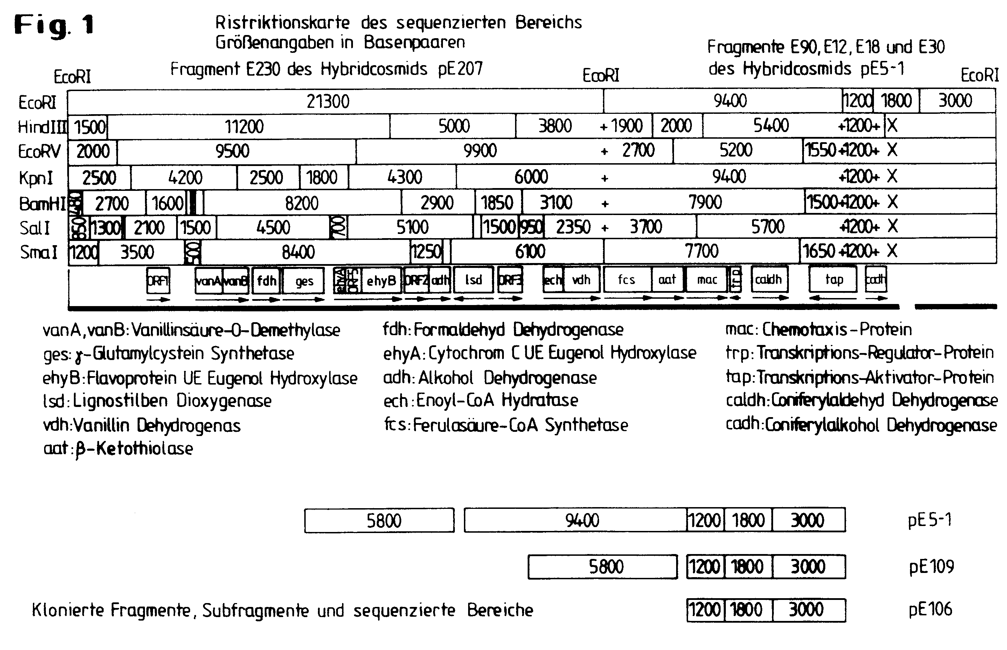

Das Fragment E230 wurde präparativ aus dem mit EcoRI-verdautem Hybridcosmid pE207 isoliert und mit EcoRI-verdauter pBluescript SK⁻-DNA ligiert. Mit dem Ligationsansatz wurde E. coli XL1-Blue transformiert. Nach "Blau-Weiß"-Selektion auf X-Gal und IPTG enthaltenden LB-Tc-Amp-Agarplatten wurden "weiße" Transformanden erhalten, deren Hybridplasmide pSKE230 das Fragment E230 kloniert enthielten. Mit Hilfe dieses Plasmids und durch Einsatz unterschiedlicher Restriktionsenzyme wurde eine physikalische Karte des Fragments E230 angefertigt (Abb. 1).

Der die Mutante 6165 komplementierende Bereich wurde durch Klonierung von Subfragmenten von E230 in den Vektoren pVK101 und pMP92, die beide über ein weites Wirtsspektrum verfügen und auch in Pseudomonaden stabil sind, mit anschließender konjugativer Übertragung in die Mutante 6165 auf ein 1,8 kbp KpnI-Fragment (K18) eingegrenzt. Nach Klonierung dieses Fragments in pBluescript SK⁻ wurde die Nukleotidsequenz bestimmt, wobei das Gen der Cytochrom C-Untereinheit der Eugenol-Hydroxylase identifiziert wurde. Das Genprodukt von 117 Aminosäuren besaß N-terminal ein Leader-Peptid (MMNVNYKAVGASLLLAFISQGAWA) und wies eine 32,9 %ige Identität (über einen Bereich von 82 Aminosäuren) mit der Cytochrom C-Untereinheit der p-Cresol Methylhydroxylase aus Pseudomonas putida (McIntire et al. 1986. Biochemistry 25: 5975-5981) auf.

Durch Klonierung der an K18 angrenzenden KpnI-Subfragmente von E230 in pBluescript SK⁻ und Sequenzierung wurden weitere offene Leserahmen (ORF) identifiziert, wobei einer dieser ORFs für die Flavoprotein-Untereinheit der Eugenol-Hydroxylase codiert und hohe Homologie zur Flavoprotein-Untereinheit der p-Cresol Methylhydroxylase aus Pseudomonas putida aufwies. Ein weiterer ORF wies hohe Homologien zur γ-Glutamylcystein Synthetase (erstes Enzym in der Glutathion-Biosynthese) aus Escherichia coli (Watanabe et al. 1986. Nucleic Acids Res. 14: 4393-4400) auf.

In der löslichen Fraktion des Rohextraktes von E. coli (pSKE230) konnte durch spezifische Aktivitätsfärbung im Polyacrylamid-Gel Vanillin-Dehydrogenase nachgewiesen werden. Durch Subklonierung in pBluescript SK⁻ und entsprechender Analyse löslicher Fraktionen der Rohextrakte von erhaltenen Transformanden konnte das Vanillin-Dehydrogenase-Gen (vdh) auf einem 3,8 kbp HindIII/EcoRI-Subfragment von E230 lokalisiert werden. Die Nukleotidsequenz dieses Fragments wurde vollständig bestimmt. Das Molekulargewicht der Vanillin-Dehydrogenase betrug 50 779, was durch SDS-Polyacrylamid-Gelektrophorese bestätigt wurde. Die Aminosäuresequenz wies hohe Homologien zu anderen Aldehyd-Dehydrogenasen unterschiedlicher Herkunft auf.

Stromaufwärts des vdh-Gens wurde ein weiterer ORF identifiziert, der Homologien zu Enoyl-CoA Hydratasen aufwies. Das errechnete Molekulargewicht von 27 297 wurde durch SDS-Polyacrylamid-Gelelektrophorese bestätigt.

Durch Sequenzierung des 5,0 kbp HindIII-Subfragments von E230, welches ebenfalls in pBluescript SK⁻ kloniert worden war, wurde ein ORF mit hoher Homologie zur Lignostilben-α,β-Dioxygenase aus Pseudomonas paucimobilis identifiziert. Durch vollständige Sequenzierung des Fragments E 230 wurden zwei weitere ORFs identifiziert, die Homologien zu Formaldehyd-Dehydrogenasen (fdh) bzw. zu Alkohol-Dehydrogenasen (adh) aufwiesen (s. Abb. 1).

### Beispiel 5

Analyse des die Mutante 6164 komplementierenden Bereichs des Hybridcosmids pE5-1

Die Mutante 6164 wurde durch den Erhalt des Hybridcosmids pE5-1 komplementiert, welches ein 1,2 kbp (E12), ein 1,8 kbp (E18), ein 3 kbp (E30), ein 5,8 kbp (E58) und ein 9,4 kbp (E94) EcoRI-Fragment kloniert enthielt (Abb. 1). Durch Verdauung von pE5-1 mit EcoRI und anschließender Religation wurde ein Derivat (pE106) dieses Hybridcosmids erhalten, welches nur noch über die Fragmente E12, E18 und E30 verfügte. Dieses Plasmid war jedoch nach konjugativer Übertragung in die Mutante 6164 in der Lage, diese zu komplementieren, wodurch entsprechende Transkonjuganten wieder auf Eugenol als C- und Energiequelle wachsen konnten.

Nach Verdauung des Plasmids pE106 mit EcoRI, gelelektrophoretischer Auftrennung des Verdauungsansatzes in einem 0,8 Gew.-% Agarose-Gel und Übertragung der DNA auf eine Nylonmembran erfolgte eine Hybridisierung mit einer mit ³²P-markierten Oligonukleotid-Sonde mit der folgenden Sequenz:

5'-ATG CAA CTC ACC AAC AAA AAA ATC GT-3'
G G C T G G T
G G C G G
G T G G G
G G G
T G G

Die Sequenz dieser Gensonde war aus der N-terminalen Aminosäuresequenz der aus Pseudomonas sp. HR 199 aufgereinigten Coniferylalkohol-Dehydrogenase (CADH) (s.u.) abgeleitet worden. Mit Hilfe dieser Sonde wurde der den N-Terminus der CADH codierende Bereich des cadh-Gens auf Fragment E12 lokalisiert. Dieses Fragment und Teile des angrenzenden Fragments E 18 wurden ebenfalls sequenziert und somit die vollständige Sequenz des cadh-Gens bestimmt. Die Von cadh abgeleitete Aminosäuresequenz wies Homologien zu anderen Alkohol-Dehydrogenasen der Klasse I, Gruppe II (nach Matthew und Fewson. 1994. Critical Rev. Microbiol. 20(1): 13-56) auf.

### Beispiel 6

### Reinigung und Charakterisierung der Coniferylalkohol-Dehydrogenase

Pseudomonas sp. HR 199 wurde auf Eugenol angezogen. Die Zellen wurden geerntet, gewaschen und mit Hilfe einer French-Presse aufgeschlossen. Die nach Ultrazentrifugation erhaltene lösliche Fraktion des Rohextraktes wies eine spezifische Aktivität von 0,24 U/mg Protein auf. Durch Chromatographie an DEAE-Sephacel wurde eine 11,7 fache Anreicherung der CADH bei einer Ausbeute von 83,7 % erzielt. Durch Chromatographie an Sephadex G200 wurde eine 6,8fache Anreicherung der CADH bei einer Ausbeute von 11,2 % erzielt. Durch Chromatographie an Butyl-Sepharose 4B wurde eine 70,6 fache Anreicherung der CADH bei einer Ausbeute von 7,8 % erzielt.

Durch diese Methode wurde ein Präparat erhalten, welches nach SDS-Polyacrylamid-Gelelektrophorese eine Bande bei 27 kDa ergab. Der Aufreinigungsfaktor betrug 64 bei einer Ausbeute von 0,8 %.

### Temperatur-Optimum und -Stabilität

Das Temperatur-Optimum der von der CADH katalysierten Reaktion lag bei 42°C. Das Enzym war jedoch wärmeempfindlich. Die Halbwertszeiten waren wie folgt: T_{1/2} (34°C) = 5 min, T_{1/2} (39°C) = 1 min, T_{1/2} (42°C) <1 min.

### pH-Optimum

Das pH-Optimum für die von der CADH katalysierten Reaktion lag bei pH 10,9 in 25 mM MOPS-Puffer. Bei höheren pH-Werten wurde ein Aktivitätsverlust durch Denaturierung beobachtet.

### Apparentes Molekulargewicht

Das native Molekulargewicht der CADH wurde mit Hilfe der FPLC durch Gelfiltration an Superdex 200HR 10/30 mit 54,9 kDa ermittelt, was eine α₂-Untereinheitenstruktur nahelegt.

### N-terminale Aminosäuresequenz

Die N-terminale Aminosäuresequenz-Bestimmung des gereinigten Proteins ergab folgendes Ergebnis: 
1                5                10                15                20
M Q L T N K K I V V V (G) V (S) ? (R) (I) ? (A) (E)
                                        (V)      (V)
 (Sequenz im Ein-Buchstaben-Code; ?: keine Angabe möglich; (): unsicher; in der zweiten Zeile wurde eine ebenfalls mögliche Aminosäure angegeben)

### Beispiel 7

### Reinigung und Charakterisierung der Coniferylaldehyd-Dehydrogenase

Pseudomonas sp. HR199 wurde auf Eugenol angezogen. Die Zellen wurden geerntet, gewaschen und mit Hilfe einer French-Presse aufgeschlossen. Die nach Ultrazentrifugation erhaltene lösliche Fraktion des Rohextraktes wies eine spezifische Aktivität von 0,43 U/mg Protein auf. Durch Chromatographie an DEAE-Sephacel wurde eine 6,6-fache Anreicherung der CALDH, bei einer Ausbeute von 65,3 % erzielt. Durch Chromatographie an Hydroxylapatit wurde eine 63-fache Anreicherung der CALDH, bei einer Ausbeute von 33 % erzielt. Durch Chromatographie an Superdex HR 200 wurde eine 81-fache Anreicherung der CALDH, bei einer Ausbeute von 13 % erzielt. Durch diese Methode wurde ein Präparat erhalten, welches nach SDS-Polyacrylamid-Gelelektrophorese eine Bande bei ca. 49 kDa ergab.

### Temperatur-Optimum und -Stabilität

Das Temperatur-Optimum der von der CALDH katalysierten Reaktion lag bei 26°C. Das Enzym war warmeempfindlich. Die Halbwertzeiten waren wie folgt: T_{1/2} (31°C) = 5 min, T_{1/2} (34°C) = 2,5 min, T_{1/2} (38°C) = 1 min.

### pH-Optimum

Das pH-Optimum für die von der CALDH katalysierten Reaktion lag bei pH 8,8 in 100 mM Tris/HCl-Puffer. Bei diesem pH-Wert ist das Enzym jedoch schon instabil (87 % Aktivitätsverlust innerhalb von 5 min). Bei niedrigen pH-Werten ist das Enzym stabiler (z.B. pH 6,0: 50 % Aktivitätsverlust innerhalb von 4 h).

### Substratspezifität

Das Enzym setzt neben Coniferylaldehyd (100 %) auch trans-Zimtaldehyd (96,7 %), Sinapylaldehyd (76,7 %), p-Anisaldehyd (23,1 %), Benzaldehyd (17,8), 3,5-Dimethoxy-Benzaldehyd (7,6 %) und 3-Hydroxybenzaldehyd (1,7 %) um. Der K_{M}-Wert der CALDH für Coniferylaldehyd liegt im Bereich zwischen 0,007 und 0,012 mM, bei einer Vₘₐₓ von ca. 9 bis 15 U/ml. Der K_{M}-Wert der CALDH für NAD liegt bei 0,334 mM, bei einer Vₘₐₓ von 14,2 U/ml. NADP wird mit einer Rate von 4,3 % verglichen mit NAD umgesetzt.

### N-terminale Aminosäuresequenz

Die N-terminale Aminosäuresequenz-Bestimmung des gereinigten Proteins ergab folgendes Ergebnis:
1 S I L G L N G A P V G A E Q L G S A L (D) 20
(Sequenz im Ein-Buchstaben-Code; (): unsicher).

### Beispiel 8

### Lokalisierung und Sequenzierung des Coniferylaldehyd-Dehydrogenase Gens (caldh)

Die N-terminale Aminosäuresequenz konnte eindeutig einer von der DNA-Sequenz des Fragmentes E94 des Plasmides pE5-1 abgeleiteten Aminosäuresequenz zugeordnet werden. Somit ist das CALDH-Strukturen caldh auf E94 lokalisiert. Die von caldh abgeleitete Aminosäuresequenz wies Homologien zu anderen Aldehyd-Dehydrogenasen auf.

### Beispiel 9

### Komplementierung weiterer, im Eugenol-Katabolismus defekter Mutanten durch die Hybridcosmide pE207 und pE5-1

Nach NMG-Mutagenese waren die Mutanten 6167 und 6202 erhalten worden, die nicht mehr in der Lage waren, Eugenol und Ferulasäure als C- und Energiequelle zu nutzen (s.o.). Die Mutante 6202 war durch Erhalt des Plasmids pE207 nach konjugativem Transfer wieder in der Lage, diese Substrate zu nutzen. Diese Mutante wird durch das Enoyl-CoA Hydratase-homologe Gen komplementiert.

Die Mutante 6167 war durch Erhalt des Plasmids pE5-1 nach konjugativem Transfer wieder in der Lage, diese Substrate zu nutzen. Die komplementierende Eigenschaft konnte durch einzelne Klonierung der EcoRI-Fragmente von pE5-1 in pHP1014 und konjugativer Übertragung dieser Plasmide in die Mutante 6167 auf das Fragment E94 eingegrenzt werden. Von Fragment E94 wurde nach Klonierung in pBluescript SK⁻ und Verdauung mit unterschiedlichen Restriktionsenzymen eine physikalische Karte angefertigt. Der die Mutante 6167 komplementierende Bereich wurde durch Klonierung von Subfragmenten von E94 in den Vektoren pVK101 und pMP92 mit anschließender konjugativer Übertragung in die Mutante 6167 auf ein 1,9 kbp EcoRI/HindIII-Fragment (EK19) eingegrenzt. Nach Klonierung dieses Fragments in pBluescript SK⁻ und Sequenzierung wurden 2 ORFs identifiziert, die Homologien zu Acetyl-CoA Acetyltransferasen bzw. zur "Medium-chain acyl-CoA Synthetase" aus Pseudomonas oleovorans aufwiesen. Durch vollständige Sequenzierung des Fragments E94 wurden weitere ORFs identifiziert, die Homologien zu Regulator-Proteinen und einem Chenotaxis-Protein aufwiesen (s. Abb. 1).

### Beispiel 10

### Nachweis der chromosomalen Codierung der Gene des Eugenol-Katabolismus in Pseudomonas sp. HR 199

Da Pseudomonas sp. HR 199 ein Megaplasmid mit einer Größe von ca. 350 kbp besitzt, wurde in einem Hybridisierungsexperiment überprüft, ob die Gene des Eugenol-Katabolismus auf diesem Megaplasmid oder auf dem Chromosom lokalisiert waren. Dazu wurden Megaplasmidpräparationen des Wildtyps und der Mutanten im 0,8 Gew.-% Agarose-Gel aufgetrennt. Die chromosomale und megaplasmidäre DNA wurde auf eine Nylonmembran geblottet und anschließend gegen eine biotinylierte HE38-DNA-Sonde hybridisiert. Dabei wurde nur mit der chromosomalen DNA, nicht jedoch mit der Megaplasmid-DNA ein Hybridisierungssignal erhalten. Somit liegen die Gene des Eugenol-Katabolismus in Pseudomonas sp. HR 199 chromosomal codiert vor.

### Beispiel 11

### Heterologe Expression von Genen des Eugenol-Katabolismus aus Pseudomonas sp. HR 199 in anderen Pseudomonas-Stämmen und in Alcaligenes eutrophus.

Das Plasmid pE207 und ein pVK101-Hybridplasmid mit Fragment H110 (pVKH110) wurden konjugativ nach A. eutrophus und in Pseudomonas-Stämme übertragen, die nicht in der Lage waren, Eugenol, Vanillin oder Vanillinsäure zu verstoffwechseln. Die erhaltenen Transkonjuganten wurden zum einen auf ihr Vermögen überprüft, auf MM-Agarplatten mit Eugenol, Vanillin oder Vanillinsäure wachsen zu können. Zum anderen wurden einige Transkonjuganten in MM-Flüssigmedium mit Eugenol inkubiert. Mittels HPLC-Analyse der Kulturüberstände wurde eine Umsetzung von Eugenol durch einige der Transkonjuganten beobachtet.

Auf diese Weise wurde eine funktionelle Expression des vdh-Gens in Transkonjuganten von P. stutzeri, P. asplenii, Pseudomonas sp. DSM13, Pseudomonas sp. DSM15a und Pseudomonas sp. D1 nachgewiesen.

Transkonjuganten des Stammes Pseudomonas sp. D1, die das Plasmid pE207 erhalten hatten, waren in der Lage, mit Eugenol als C- und Energiequelle zu wachsen. Auch in entsprechenden Transkonjuganten von P. testosteroni LMD3324, P. fluorescens TypB, P. stutzeri DSM50027, Pseudomonas sp. DSM1455 und P. fragi DSM3456 wurde eine funktionelle Expression der Eugenol-Hydroxylase-Gene beobachtet, was zu einer Ausscheidung von Intermediaten des Eugenol-Katabolismus (Coniferylalkohol, Coniferylaldehyd, Ferulasäure, Vanillin, Vanillinsäure) in das Kulturmedium führte. Ein Wachstum dieser Transkonjuganten auf Eugenol wurde hingegen nicht beobachtet.

SEQUENZPROTOKOLL

(1) ALLGEMEINE ANGABEN:

(i) ANMELDER:
(A) NAME: Haarmann & Reimer GmbH
(B) STRASSE: Rumohrtalstrasse 1
(C) ORT: Holzminden
(E) LAND: Deutschland
(F) POSTLEITZAHL: 37603
(G) TELEFON: 0214-3067988
(H) TELEFAX: 0214-303482

(ii) BEZEICHNUNG DER ERFINDUNG: Syntheseenzyme fuer die Herstellung von
Coniferylalkohol, Coniferylaldehyd, Ferulasaeure, Vanillin
und Vanillinsaeure und deren Verwendung

(iii) ANZAHL DER SEQUENZEN: 42

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 32679 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Pseudomonas sp.
(B) STAMM: HR199

(ix) MERKMAL:
(A) NAME/SCHLÜSSEL: CDS
(B) LAGE:3146..3997
(D) SONSTIGE ANGABEN:/gene= "ORF1"

(xi) SEQUENZBESCHREIBUNG: SEQ ID NO: 1:

GAATTCATCC TCATGGAGCA CTTCTACAAG CAGCAGGCAG GCCACCCTCC CCAGACCGAT 60

GACGTGCATA TTATCGCGAT CGGCGGAACG AGCTTTAAAC GCTACCTGGA GCTCGGAAAG 120

CTCCTGAACA TCAGAGTTGC CGCAATTCGA GATAACGACG GTGACTATCA GCAGAACTGT 180

GTAGCGAACT ACGAAGGCTA CCTGTACGAG TCGGCCAAGA TTTTCGCCGC CCCAGATCCT 240

GACCGAAGCA CCTTCGAAAT AGGGCTGTAC CGTGACAACC AGAAAGCCTG TGACGATCTC 300

TTTGTTGCGG GTCGCAAAAA ACTGACCGTG CAAGAGTACA TGCTCAAAAA TAAAGCGGAT 360

GCCGCTTTCG AGCTGCTGAC CAAGAAGTCC GCTGAACTGA TCGCCCCGAA GTACATACAG 420

GAAGCGATCG AATGGATAAG AGCGTAATTT TCTCCGTCGC AGGATCCGGG AAAACCAGCC 480

TGATCATCGA GCGTCTCAGC CTTGATCAGC GGGCATTGGT CATCACTTAC ACGGACAACA 540

ATCACCGGCA CCTGCGCAAC AGGATCATTC AGAGATTCGG GGTGATCCCA TCCAACATCA 600

CGCTCATGAC GTACTTCTCG TTCCTGCATG GGTTCTGCTA TCGGCCCTTG ATGCAATTGC 660

AGCTAGGAAC ACGAGGCCTA AATTTCAGAC GTCCGCCCAA CAGGCAGTAC CCCCTGAACG 720

ATCTCAATCG GTATCGCGAT GGAAGCGGCA GGCTCTATCA CTGCCGCCTC GCGAAACTGC 780

TGGACGTTGC GCAGGCCTTA CCGGATGTGC GTGCCCGCCT GGAGCGCTTT TACGACTGCC 840

TGTACGTCGA CGAGGTACAG GATTTCGCGG GTCACGACTT CAACCTCCTG CTGGAGGTTT 900

CACGGGCGAA GATCGGCATG ACGTTCGTCG GTGATTTCCA CCAGCACACC TTCGATACCA 960

GCCGAGACGG AGCGGTAAAC AAAACCCTTC ACGACGATGC CGTTCGCTAC GAGAAGCGCT 1020

TTCGTGATGC CGGCATTTCG GTGGACAAGC AAACGTTGAA CCGCAGCTGG CGATGCGCCA 1080

AAACGGTCTG TGACTTCATC AGCGCAAAGC TGAAAATTGG CGATGGACGC TCACGAGGAG 1140

CGGGGCAGCC GGATCATTAG AGTTGATGAC CAAGAGCAGG CCAACTTGTT GCACGTTGAC 1200

CCAACCATCG TGAAGCTGTT TTTGAGCGAA CACTACAAGT ACGGCTGCCA CTCCGAAAAC 1260

TGGGGGGCAA GCAAGGCATG GATCACTTTA ACGATGTCTG CGTTGTGATG GGCCCGGGTA 1320

TCTGGAAAGA CTATGTGGCT GAGAGGTTAC ACCAGGCCAA CCCGCAAACC CGAAACAAGC 1380

TGTACGTGGC CTGCACTAGG GCGCGGGGTG ATCTGTATTT CGTGCCTGAG AAGCTCTTGA 1440

GGGCCTTCAA ACAGGGAAAT TAGGCGATAA AGCTGAAAAA GGATTTTCAA GTAAAGACCA 1500

CTCCTTCCTT ACTCGATGTC CGCTTTTGGC CGATTTCTGC CAGTCACGAC CGGCAAAGAA 1560

CGGCCAAAAG CGGACTGATG CGGTTACTAA GCCTGCCTCT TATTGAAGCT TGGTGGGCTT 1620

TAAGAATGTG GTGCGATCCA GCCTGATGAT GTTCCGCTTT ATGCACGCAG CCAAGCCTAT 1680

CGACCGCCGT CTGCACGTTG TAACCGACTA CGCCTGTGCC TTTGCCGCTG GTGGCCATGG 1740

AGCGTGCATC CGGATCGGTG AGTGAGACTT GCCCATCCGG TGCTTCACGT AGCTGCTGCT 1800

CCATCTCCTT GAGCGCCTGC ATCTGCTGGC GGAGTTTCTC GATTTTATCC TGGAGGCGGC 1860

TGGCTTTGGC TTCGGCGACA TCGGATTGAG TTCTGTCGGC GGTGTCCATC GCTGCCAGAT 1920

AGCGGTCGAT GATTTTATCA ATCTGGTCCA TCCGGGCGCG CACCCGCTAT GATCCGGAGT 1980

CCTCCGATAT CGATGAGGCC TATCTGGGCT GGAAGAGCGG TTCGGTGTTC TCAGACCTTG 2040

GCGAGAACGC GGTCAAGCTC AGCTTCGGGC GCCAAGCCTT CAAGATCGGC AACGGCTTCC 2100

TGATCGGCGA AGGCCACGTC GACCAAGGTA ACGATGCGGG CTACTGGCTG GCCCCTACCT 2160

AGGCGTTCGA CAACACCGTC CTAGCCCAAC TGGACACCGG CAAGCTGCAT GTCGACCTGT 2220

TCGACCTCCA GGCGGGCATG GATCTGGACG TCGCCGACAT CAAGGAGAAA GTCCGGGTGC 2280

GCGGGGGCAA CGTCGAGTGG CGCGACGAGA CCTACGGCAC GGTAGGGTTC ACCGGCTTCC 2340

ATACGCTGGA CGCTGACAAT CCGCTGCGCG ACGGCATGAA TGTCTACGAC GTACGCGCAT 2400

CGGGCAGCCC GATCCGAGCC CTGCCGCAGG TGGCCCTGGC GGCGGAGTAC GCCTGGCAGC 2460

GCGGCGGCGA GGCGGACAAG ACGAGTGAGG CCTGGTACCT ACAGGGCAGC TACACCTTTC 2520

GGGATGCCCC CTGGACGCCA GTGCTGATGT ACCGTCACGC GGTCTTCTCC GACGACTACG 2580

ACTCCCTGCT GTACGGCTAA GGGGGCAACA ACATGGGCTG GAAAGGAGCA TTGCGTTGAA 2640

ACGATGCTGA AGGGCGTCAC TCTTTTACTG CTGTCCGCTC ACGTCGAAAC TGCATGATTT 2700

CGGGCAGCCT TTCTTCTATC CAGTCGGCCA GCACCTGAAC ATGAGCCGCT ACTTCCTGGC 2760

CAAGCGGCGT CAGGCTGTAC TCGACATGTG GGGGAACGAC CGGGAGCGAA TGTCGAGCTA 2820

TGAAACCGTC TCCCTCCAGG CCTTGTAGGG TCTGCGCAAG CATTCTTTTC GCTGACACCG 2880

CCGATTCTTC CGACGCAGGT CGCTGAATCG ATGGACACCG TCCACCAAGA TGATCAGCAC 2940

GAGCACGCCC AGCGGCTTGT CACGTGCTTG AGCACGTCCC GCGACGGCAT TCAGCACTCA 3000

GCAATTCCCG CGCCGTGCTT GCATGGAGAG ACTGGTAAGG GCGGCCAGCG TGAGTTTCAT 3060

GGCACTAACC TTTATGTATG TACTTACTTT TAGTTGCTAG TAGGGATATG GTGACGCCTT 3120

CATCCTACGA AACAAGTGAA GACTG ATG ATC GCC ATC ACA GGT GCC TCC GGA 3172
Met Ile Ala Ile Thr Gly Ala Ser Gly
1               5

CAA CTT GGT CGG TTG ACT ATA GAG GCG CTA CTG AAG CGC CTG CCA GCA 3220
Gln Leu Gly Arg Leu Thr Ile Glu Ala Leu Leu Lys Arg Leu Pro Ala
10                  15                  20                  25

TCC GAA ATT ATT GCC CTC GTC CGG GAT CCG AAT AAG GCC GGA GAC CTT 3268

Ser Glu Ile Ile Ala Leu Val Arg Asp Pro Asn Lys Ala Gly Asp Leu
                30                  35                  40

ACC GCA CGT GGC ATC GTG GTG CGC CAG GCC GAT TAC AAC CGG CCG GAA 3316
Thr Ala Arg Gly Ile Val Val Arg Gln Ala Asp Tyr Asn Arg Pro Glu
            45                  50                  55

ACA CTC CAC CGG GCC CTG ATT GGG GTC AAC CGG TTG CTG TTG ATT TCC 3364
Thr Leu His Arg Ala Leu Ile Gly Val Asn Arg Leu Leu Leu Ile Ser
        60                  65                  70

TCC AGT GAG GTG GGT CAA CGA ACT GCG CAA CAC CGG GCA GTG ATC GAC 3412
Ser Ser Glu Val Gly Gln Arg Thr Ala Gln His Arg Ala Val Ile Asp
    75                  80                  85

GCT GCG AAG CAA GAA GGT ATC GAG TTG CTG GCT TAT ACG AGT CTG CTT 3460
Ala Ala Lys Gln Glu Gly Ile Glu Leu Leu Ala Tyr Thr Ser Leu Leu
90                  95                  100                 105

CAT GCC GAT AAA TCG GCG CTG GGC CTA GCG ACT GAA CAC CGA GAC ACG 3508
His Ala Asp Lys Ser Ala Leu Gly Leu Ala Thr Glu His Arg Asp Thr
                110                 115                 120

GAA CAG GCC CTG ACA GAG TCC GGT ATT CCT CAT GTC CTG TTG CGC AAC 3556
Glu Gln Ala Leu Thr Glu Ser Gly Ile Pro His Val Leu Leu Arg Asn
            125                 130                 135

GGT TGG TAT CAC GAG AAC TAC ACG GCG GGC ATC CCA GTC GCG CTG GTT 3604
Gly Trp Tyr His Glu Asn Tyr Thr Ala Gly Ile Pro Val Ala Leu Val
        140                 145                 150

CAT GGC GTG TTG CTG GGC TGT GCC CAG GAT GGC TTG ATT GCT TCT GCT 3652
His Gly Val Leu Leu Gly Cys Ala Gln Asp Gly Leu Ile Ala Ser Ala
    155                 160                 165

GCA CGT GCT GAC TAC GCC GAA GCA GCG GCT GTG GTG CTC ACC GGT GAG 3700
Ala Arg Ala Asp Tyr Ala Glu Ala Ala Ala Val Val Leu Thr Gly Glu
170                 175                 180                 185

AAT CAG GCA GGT CGC GTC TAC GAG CTG GCC GGT GAA CCG GCA TAT ACG 3748
Asn Gln Ala Gly Arg Val Tyr Glu Leu Ala Gly Glu Pro Ala Tyr Thr
                190                 195                 200

CTC ACC GAA CTG GCA GCT GAG GTG GCG CCG CAA GCA GGA AAG ACC GTC 3796
Leu Thr Glu Leu Ala Ala Glu Val Ala Pro Gln Ala Gly Lys Thr Val
            205                 210                 215

GTG TAT TCG AAC CTA TCC GAG AGC GAT TAC CGA TCT GCG TTG ATC AGT 3844
Val Tyr Ser Asn Leu Ser Glu Ser Asp Tyr Arg Ser Ala Leu Ile Ser
        220                 225                 230

GCG GGC CTT CCC GAT GGT TTT GCG GCA TTG CTC GCA GAC TCT GAT GCA 3892
Ala Gly Leu Pro Asp Gly Phe Ala Ala Leu Leu Ala Asp Ser Asp Ala
    235                 240                 245

GGC GCA GCC AAG GGG TAT TTG TTT GAT TCC AGT GGA GAC AGT CGC AAG 3940
Gly Ala Ala Lys Gly Tyr Leu Phe Asp Ser Ser Gly Asp Ser Arg Lys
250                 255                 260                 265

CTG ATC GGT CGC CCA ACC ACT CCG ATG TCG GAA GCC ATC GCG GCA GCA 3988
Leu Ile Gly Arg Pro Thr Thr Pro Met Ser Glu Ala Ile Ala Ala Ala
                270                 275                 280

ATT GGC CGC TAAAACTGCA TTTTCGCGAC TTGAGTGACA CCTGGGTTAG 4037
Ile Gly Arg

ATAACCCAGG TGTCTCGCAC CGCTTTGGGT TAGTGGTGGG CAATAGCGGT GTCTGGTCAC 4097

CGCTTGCCCG GCGGCGCGCC CGCTATTGGA TGATTCTCAA CTTCCTGGTG CCGGCGTCTT 4157

GTTGGGGCCC AAACAGGCGG GCATAACGCA ATGTGGCATT TGCACTGTCG CGCATGATGG 4217

CTTCTGCTCG AGCACCTTGC CCGCTAATCA GCGCGTCTAC CACAGCATGA TGCTGCATGT 4277

TGGCAAAATT GAACCGGCGG TACTCTTGGG GAGGTTGCTA CCGTCGACGG CCAGTGAACT 4337

GACAGAGGCA AAGGGCAGGT GTTCATTCCG AGCCAATGCT TCACCTATGG CAGCGTTACC 4397

GCTGGCATCC ACGATAGCTT GATGGAAGCG CTTGTTGATG TCGTGGTATT CGGCGAGGTC 4457

GTCTTCGCTG ACATAACCTT TCTCAAATAG GGCATCGCCC TGGGCCAAGC ACTGCAAGAG 4517

GATCTCTTGC GTTTCACTGG ATAGCCCTCG CTCGGCAGCC TGCCTTGCGG CCAGTCCTTC 4577

AAGTACCCCT CGAACCTCCA CCGCGCCTGC CAGGTCATTT GGGGTCATTT GCCGCACTGC 4637

ATAGCCACGT GCGCCTTGGC GATCAGTAAC CCTTCCTGTT CTAGCGCTCG GAACGCAATG 4697

CGGATAGGTG TGCGCCGACA CTCCCAGGCG CTCGGCAGTG GGGATTTCGG CGATGCGCTC 4757

TCCTGCCGGG AGTTCGCCAT CCACAATCAT TTTGCGCAGT AGATTGAGTA CTCGCTGCCC 4817

GGGCCCGCTC ATTTCAGCCT CCGATTGGAT CCAGTAATGG TTTGAGAGAA TTTTACTCGC 4877

AAGGGATTTC TGGGCAATAG CCCCGCTGAT TGCTGGTTTT TGTATGTGGC GTGCGACTAT 4937

CGCACAGAAT TGGATCCACC TTGGCGCAAA AAAACTGGAG CTACCTCATC GGTCGTGGTT 4997

ATATTGGATC CCATAAGGTC AAGTTCATAG CTGATTTTGG CTTTAGATGT CCATTGTGGA 5057

TCCAAAAACA AGATCGCCAT TGAGGAACGC GCCATGTTTC CGAAAAACGC CTGGTATGTC 5117

GCTTGCACTC CGGATGAAAT CGCAGATAAG CCGCTAGGCC GTCAGATCTG CAACGAAAAG 5177

ATTGTCTTCT ATCGGGGGCC GGAAGGACGT GTTGCCGCGG TAGAGGATTT CTGCCCTCAT 5237

CGCGGGGCAC CGTTGTCCCT GGGTTTCGTT CGCGACGGTA AGCTGATTTG CGGCTACCAC 5297

GGTTTGGAAA TGGGCTGCGA GGGCAAAACG CTCGCGATGC CCGGGCAGCG CGTTCAAGGC 5357

TTCCCTTGCA TCAAAAGCTA CGCGGTAGAA GAGCGATACG GCTTTATCTG GGTATGGCCT 5417

GGTGATCGCG AGCTGGCGGA TCCGGCGCTT ATTCACCACC TGGAGTGGGC CGATAATCCG 5477

GAGTGGGCCT ATGGTGGCGG TCTCTACCAC ATCGCTTGTG ATTACCGCCT GATGATCGAC 5537

AACCTCATGG ATCTCACCCA TGAGACCTAT GTGCATGCCT CCAGCATCGG TCAAAAGGAA 5597

ATTGACGAGG CACCGGTCAG TACTCGTGTC GAGGGCGACA CCGTGATTAC CAGCCGGTAC 5657

ATGGATAACG TCATGGCCCC TCCGTTCTGG CGTGCTGCGC TTCGTGGCAA CGGCTTGGCC 5717

GACGATGTAC CGGTTGATCG CTGGCAGATC TGCCGATTCG CTCCTCCGAG TCACGTACTG 5777

ATCGAAGTAG GTGTGGCTCA TGCGGGCAAA GGCGGATATG ACGCGCCGGC GGAATACAAG 5837

GCCGGCAGCA TAGTGGTCGA CTTCATCACG CCGGAGAGTG ATACCTCGAT TTGGTACTTC 5897

TGGGGCATGG CTCGCAACTT CCGTCCGCAG GGCACGGAGC TGACTGAAAC CATTCGTGTT 5957

GGTCAGGGCA AGATTTTTGC CGAGGACCTG GACATGCTGG AGCAGCAGCA GCGCAATCTG 6017

CTGGCCTACC CGGAGCGCCA GTTGCTCAAG CTGAATATCG ATGCCGGCGG GGTTCAGTCA 6077

CGGCGCGTCA TTGATCGGAT TCTCGCAGCT GAACAAGAGG CCGCAGACGC AGCGCTGATC 6137

GCGAGAAGTG CATCATGATT GAGGTAATCA TTTCGGCGAT GCGCTTGGTT GCTCAGGACA 6197

TCATTAGCCT TGAGTTTGTC CGGGCTGACG GTGGCTTGCT TCCGCCTGTC GAGGCCGGCG 6257

CCCACGTCGA TGTGCATCTT CCTGGCGGCC TGATTCGGCA GTACTCGCTC TGGAATCAAC 6317

CAGGGGCGCA GAGCCATTAC TGCATCGGTG TTCTGAAGGA CCCGGCGTCT CGTGGTGGTT 6377

CGAAGGCGGT GCACGAGAAT CTTCGCGTCG GGATGCGCGT GCAAATTAGC GAGCCGAGGA 6437

ACCTATTCCC ATTGGAAGAG GGGGTGGAGC GGAGTCTGCT GTTCGCGGGC GGGATTGGCA 6497

TTACGCCGAT TCTGTGTATG GCTCAAGAAT TAGCAGCACG CGAGCAAGAT TTCGAGTTGC 6557

ATTATTGCGC GCGTTCGACC GACCGAGCGG CGTTCGTTGA ATGGCTTAAG GTTTGCGACT 6617

TTGCTGATCA CGTACGTTTC CACTTTGACA ATGGCCCGGA TCAGCAAAAA CTGAATGCCG 6677

CAGCGCTGCT AGCGGCCGAG GCCGAAGGTA CCCACCTTTA TGTCTGTGGG CCCGGCGGGT 6737

TCATGGGGCA TGTGCTTGAT ACCGCGAAGG AGCAGGGCTG GGCTGACAAT CGACTGCATC 6797

GAGAGTATTT CGCCGCGGCG CCGAATGTGA GTGCTGACGA TGGCAGTTTC GAGGTGCGGA 6857

TTCACAGCAC CGGACAAGTG CTTCAGGTCC CCGCGGATCA AACGGTCTCC CAGGTGCTCG 6917

ATGCGGCCGG AATTATCGTT CCCGTTTCTT GTGAGCAGGG CATCTGCGGT ACTTGCATCA 6977

CTCGGGTGGT AGACGGAGAG CCTGATCATC GTGACTTCTT CCTCACGGAT GCGGAGAAGG 7037

CAAAGAACGA CCAGTTCACC CCCTGTTGCT CGCGAGCCAA GAGCGCCTGT TTGGTCTTGG 7097

ATCTCTAACT CATCCCCGTG TCCGGTCCCC TGCTTTGGTG CGGCGGACTG TGCGCGGGTA 7157

AGTAAACAGG CTCAACCGTT TTTAGCGGGA TAACCATTCT TGAGGATGAA GGAGGGTTAT 7217

CCCGCTCTTT TCATGCACCA AGCCATTCAT AGTCACCAGC TGCTTCTACG TGCTGCTGCG 7277

TTACAAGTTT ATTCAGAAGG AAATCGGAAT GATCAAATCC CGCGCCGCTG TGGCGTTCGC 7337

ACCCAATCAG CCATTGCAGA TCGTCGAAGT GGACGTGGCT CCGCCCAAGG CCGGTGAAGT 7397

CCTGGTGCGG GTCGTGGCCA CCGGCGTTTG CCACACCGAT GCCTACACCC TGTCCGGCGC 7457

TGATTCCGAG GGCGTTTTCC CCTGCATCCT TGGTCACGAA GGCGGCGGCA TTGTCGAAGC 7517

GGTGGGCGAG GGCGTCACCT CGCTGGCGGT CGGCGACCAC GTGATCCCGC TCTACACGGC 7577

CGAATGCCGT GAGTGCAAGT TCTTCAAGTC CGGCAAGACC AACCTGTGCC AGAAAGTGCG 7637

TGCTACTCAG GGCAAGGGTC TGATGCCGGA CGGCACCTCC CGCTTCAGCT ACAACGGTCA 7697

GCCGATCTAC CACTACATGG GCTGCTCGAC CTTCTCCGAG TACACCGTGC TGCCGGAAAT 7757

CTCCCTGGCG AAGATTCCCA AGAATGCGCC GCTGGAGAAA GTCTGCCTGC TGGGCTGCGG 7817

CGTGACCACC GGCATTGGCG CGGTGCTGAA CACTGCCAAG GTGGAGGAGG GTGCTACCGT 7877

GGCCATCTTC GGCCTGGGCG GCATCGGCTT GGCGGCGATC ATCGGCGCGA AGATGGCCAA 7937

GGCCTCGCGC ATCATCGCCA TCGACATCAA TCCGTCCAAG TTCGATGTGG CTCGCGAGCT 7997

GGGCGCCACT GACTTCGTCA ATCCGAACGA TCACGCGAAG CCGATCCAGG ATGTCATCGT 8057

CGAGATGACT GATGGCGGTG TGGACTACAG CTTCGAGTGC ATCGGCAACG TTCGACTCAT 8117

GCGCGCAGCA CTCGAGTGCT GCCACAAGGG CTGGGGCGAA TCCGTGATCA TCGGCGTGGC 8177

GCCGGCGGGG GCCGAAATCA ACACCCGTCC GTTCCACCTG GTGACCGGTC GCGTCTGGCG 8237

GGGTTCGGCG TTCGGTGGCG TAAAGGGCCG CACCGAACTG CCGAGCTACG TGGAGAAGGC 8297

ACAGCAGGGC GAGATCCCGC TGGACACCTT CATCACTCAC ACCATGGGCC TGGACGACAT 8357

CAACACGGCC TTCGACCTGA TGGACGAAGG GAAGAGCATC CGCTCTGTTG TTCAATTGAG 8417

TCGCTAGTGA AGTGGGGTGA GGAAATTGGA TTAGGAGGCG GATGGTTCCT GCCGCTTAAC 8477

CACCTTGTCC CAGCTTCTGG CTGAGATTTC CAAGATTCGG TGAAATTTGC CATGCCGCAA 8537

ACTCTTGCTG GACGGTTGAG TCTGTTATCC GGCACCGACG AATTAACCCT GCTTCTTCGG 8597

GGTGGTCGGG GCATTGAGCG TGAAGCCTTG CGGGTCGATG TTCAAGGTGA ACTGGCGCTG 8657

ACGCCTCACC CGGCGGCGCT TGGCTCTGCG TTGACCCATC CGACAATTAC TACGGATTAC 8717

GCCGAGGCCC TGCTTGAGTT GATCACTCGG CCGGCAACCG ATTGTGCGCA AGCCTTGGCT 8777

GAGCTGGAGG AGCTTCACCG TTTCGTTCAT TCGAGACTTG AGGGGGAGTA TCTCTGGAAT 8837

CTGTCCATGC CTGGCAGATT GCCGGTTGAT GAGCAAATCC CGATTGCTTG GTATGGACCA 8897

TCAAATCCAG GCATGTTGCG CCACGTTTAT CGCCGTGGCC TAGCTCTGCG TTATGGCAAG 8957

CGAATGCAAT GCATCGCAGG GATTCACTAC AACTACTCAC TGCCGCCAGA GCTTTTCGCT 9017

GTCCTGACCA AGGCAGAGGT CGGGTCTCCC AAGTTACTGG AGCGCCAGTC AGCAGCTTAC 9077

ATGCGCCAAA TTCGCAACCT TCGGCAATAC GGTTGGTTGC TGGCCTACTT GTTCGGCGCT 9137

TCCCCCGCCA TCTGCAAGAG CTTCTTGGGG GGCGAGAGAG ATGAGCTAGC TCGCATGGGG 9197

GGCGATACGC TTTACATGCC CTATGCAACC AGCTTGCGCA TGAGTGACAT CGGGTACCGC 9257

AACCGTGCCA TGGATGATCT ATCTCCCAGC CTGAATGATC TGGGTGCCTA TATTCGCGAT 9317

ATTTGCCGTG CTCTTCACAC TCCCGATGCC CAGTACCAGG CGCTGGGTGT GTTTGCACAG 9377

GGCGAGTGGC GGCAGTTAAA CGCCAATCTA TTGCAGTTGG ATAGTGAGTA CTACGCACTG 9437

GCGCGACCGA AGTCAGCGCC CGAGCGGGGG GAGCGAAACC TGGATGCTCT CGCTAGGCGT 9497

GGAGTCCAGT ATGTGGAGCT GCGCGCACTG GATCTCGATC CATTCTCCCC GTTAGGCATT 9557

GGCCTGACCT GCGCCAAGTT CCTCGATGGC TTTTTGCTTT TCTGCTTGTT GTCTGAGGCG 9617

CCGGTTGATG ATCGAAATGC CCAGCGTTCA AGACCGGGAA AATCTGAGCC TGGCCGGCAA 9677

GTACGGGCGT CACCTGGCTT AAAGCTGCAT CGGAATGGTC AGTCCATTCT CCTCAAGGAT 9737

TGGGCGCAGG AAGTGTTGAC GGAGGTTCAG GCCTGTGTGG AATTGCTCGA CAGTGCAAAT 9797

GGGGGCTCAT CTCACGCATT GGCTTGGTCA GCACAGGAGG AAAAGGTGCT TAATCCGGAT 9857

TGTGCGCCAT CAGCTCAGGT GCTCGCAGAG ATACACAGAC ACGGTGGGAG CTTCACGGCA 9917

TTTGGTCGCC AATTAGCTAT CGACCATGCA AAACACTTCA GTGCCTCCTC GCTTGAGGCT 9977

GGCGTAGCCA AAGCGCTTGA CCTCCAGGCG ACGTCGTCTC TGCGCGAGCA GCATCAATTG 10037

GAGGCCAACG ACCGTGCGCC ATTTTCTGAC TACCTTCAGC AATTCTCCCT GGCTTTCGGT 10097

CAATCCGTCG GCGCCTCTCG TGCGCCCAAC CCTACCGCGC ACCTCATCGA TCTGACCCCT 10157

CCTGTCTAAG GTTGTCGTGG GAGCAGATCC GTGGGCCGAG CTTCCTCCAG GGCCTGGCCG 10217

CAGCGATCCA GTTGCTAGGT CCCTATGCTC TTGCATAGGG TAAAAATTAG TTATTGTGTT 10277

TAACGAAACG TCTGGCATAC TGGCTTTAGG CACGAGCTTC CACGCCGAAG TTGAGAGCGT 10337

CATGAACGAT TTTTCGTGTG GAGAGACGAT GCCCGATGCG GTCGACGAGG TTCAGGTCCT 10397

AATGGCAGTG CCGGCGGCTA AACGGAACGT GCCGTATTTT GAGGCTTGGA GCGTGGTGAA 10457

GCAGCTTGGC TGCTCCCTGG GCCTGTCAGG ATCACGCTGT GTCGGCAGTG ACACTTCAAA 10517

ACAAGAAGGG CATTAAGATG ATGAATGTTA ATTATAAGGC TGTCGGGGCG AGCCTACTCC 10577

TCGCCTTCAT CTCTCAGGGA GCTTGGGCAG AGAGCCCCGC AGCCTCTGGC AATACCCCTG 10637

ACATTTATCG AAAGACCTGC ACCTACTGCC ATGAGCCTAC TGTCAACAAT GGCCGGGTCA 10697

TTGCCCGAAG CCTCGGGCCG ACTCTGCGAG GGCGCCAGAT CCCTCCACAG TACACGGAGT 10757

ACATGGTGCG TCATGGACGC GGGGCAATGC CTGCATTCTC TGAAGCAGAA GTGCCTCCGG 10817

CGGAGCTGAA AGTTCTGGGC GATTGGATTC AGCAAAGCAG TGCTCCCAAA GACGCTGGAG 10877

TCGCGCCATG ACTACCCGTC GCAACTTTCT AATAGGCGCG TCGCAGGTGG GGGCATTGGT 10937

GATGATGTCG CCGAAATTGG TCTTCCGTAC GCCGCTCAAG CAGAAGCCCG TGCGCATCCT 10997

GTCGACCGGG CTGGCCGGTG AGCAAGAGTT TCACTCGATG CTTCGCGCGC GATTGACCCA 11057

TACGGGTCAG GTCGACATCG CGTCGGTACC GCTGGACGCA GCTATTTGGG CTTCTCCCGC 11117

TCGACTTGCC CAGGCAATGG ATGCGTTGAA TGGTACGCGT CTGATCGCTT TTGTTGAGCC 11177

CAGGAACGAA TTGATACTGA TGCAATTCTT GATGGATCGC GGGGCTGCGG TGCTTATTCA 11237

AGGTGAGCAT GCGGTGGACA GCAAGGGGGT CTCTCGGCAC GACTTTCTGA GTACCCCATC 11297

CAGTGCGGGA ATTGGAGGGG CGCTAGCCGA CAGCCTGGCA AAGGGGGGCT CGCCGTTCTC 11357

TATTTCCGTC CGAGCGCTTG GCTCGGTAAC TGCTCAGCCA AGAAGTAATC AGAGTGAGGT 11417

GGCCACCCAC TGGACGACCG CTCTGGGGAC CTATTATGCC GATATCGCAG TGGGGCGCTG 11477

GGAGCCGCAG CGCGAAGTGG CCAGCTATGG AAGTGGACTA ATCATGGCGG AACGGCTTGA 11537

TCGTGTTGCC TCAACCTTCA TTGCAGATCT CTGAGTCAGG GTATTGATAT GGAAAGCACC 11597

GTAGTTCTTC CCGAGGGTGT CACCCCGGAG CAGTTCACCA AAGCCATCAG CGAGTTCCGT 11657

CAGGTATTGG GTGAGGACAG TGTTCTTGTC ACTGCTGAAC GAGTTGTTCC CTATACGAAA 11717

CTCCTCATTC CTACACAGGA TGATGCCCAG TACACCCCGG CCGGTGCCTT GACTCCTTCT 11777

TCGGTGGAGC AGGTCCAGAA AGTCATGGGG ATCTGCAATA AGTACAAGAT CCCGGTATGG 11837

CCAATCTCTA CCGGTCGGAA CTGGGGGTAT GGGTCCGCTT CGCCTGCAAC TCCTGGGCAG 11897

ATGATTCTTG ACCTTCGCAA GATGAACAAG ATCATTGAGA TCGATGTTGA GGGGTGTACT 11957

GCCCTGCTCG AGCCGGGCGT TACCTACCAG CAGCTTCACG ATTACATCAA GGAGCACAAT 12017

CTGCCCTTGA TGCTGGATGT GCCGACTATT GGGCCTATGG TTGGCCCGGT GGGTAACACG 12077

CTGGATCGAG GCGTTGGTTA TACGCCGTAC GGCGAGCACT TCATGATGCA GTGTGGTATG 12137

GAAGTCGTCA TGGCCGATGG CGAAATCCTC CGTACTGGTA TGGGCTCGGT GCCCAAAGCC 12197

AAGACTTGGC AGGCATTCAA ATGGGGCTAT GGTCCATATC TGGACGGTAT CTTTACCCAG 12257

TCCAACTTTG GTGTTGTGAC AAAGCTCGGG ATTTGGTTGA TGCCCAAGCC GCCAGTGATC 12317

AAGTCGTTTA TGATCCGTTA TCCCAATGAA GCTGATGTGG TTAAGGCAAT TGATGCTTTT 12377

CGCCCGCTGC GTATTACTCA GCTGATTCCT AACGTCGTTT TGTTCATGCA CGGCATGTAC 12437

GAAACGGCAA TCTGCCGGAC GCGTGCTGAG GTTACTTCGG ACCCAGGTCC TATTTCTGAA 12497

GCGGACGCCC GCAAAGCATT CAAAGAGCTA GGCGTTGGCT ACTGGAACGT TTACTTCGCG 12557

CTTTACGGCA CAGAAGAGCA GATAGCCGTC AATGAAAAGA TCGTCCGCGG CATCCTCGAA 12617

CCGACGGGGG GTGAGATCCT CACCGAAGAG GAGGCTGGAG ATAACATTCT TTTCCATCAC 12677

CATAAGCAGC TCATGAACGG CGAGATGACA TTGGAGGAAA TGAATATCTA CCAGTGGCGC 12737

GGAGCAGGTG GCGGTGCTTG CTGGTTTGCA CCGGTTGCTC AGGTCAAGGG GCATGAGGCA 12797

GAGCAGCAGG TCAAGCTTGC TCAGAAGGTG CTTGCAAAGC ATGGGTTCGA TTACACGGCG 12857

GGCTTTGCGA TTGGTTGGCG CGATCTTCAC CATGTGATCG ATGTGCTGTA CGACCGTAGC 12917

AATGCCGACG AGAAAAAGCG CGCTTACGCT TGCTTTGATG AATTGATCGA CGTCTTTGCG 12977

GCCGAAGGCT TTGCAAGTTA CAGGACCAAT ATTGCCTTTA TGGACAAAGT CGCCTCTAAG 13037

TTCGGCGCTG AGAATAAGAG GGTCAATCAG AAGATCAAGG CTGCCCTTGA TCCAAACGGC 13097

ATCATCGCTC CCGGCAAGTC GGGCATTCAT CTTCCCAAAT AATGCGTGTT CGTGAGGCGG 13157

CTGCTAGCCG CCTCATTTGA AGAAAGAGTC GTATCGGCGA TGCATGATGC GTCGTTCGCT 13217

CTCGGCTGTT GATTCTTCGA AAGAAGCGTA TGGGGGGGGA ATGATTGCAA TCACTGCGGG 13277

CACCGGAAGT CTTGGTCGGG CTATCGTTGA GCGACTAGGG GACTGCGGTC TTATCGGTCA 13337

AGTTCGATTG ACGGCTCGCG ATCCTAAAAG GCTTCGTGCC GCTGCCGAGG AAGGGTTTCA 13397

GGTCGCTAAG GCGGATTACG CCGATATTGG GAGTCTTGAC CAGGCATTAC AGGGGGTAGA 13457

CGTATTACTC CTGATTTCTG GTACTGCACC CAATGAAATA AGGATCCAAC AGCATAAGTC 13517

GGTCATCGAC GCGGCAAAAC GAAACGGCGT GTCGCGTATT GTGTATACCA GCTTCATAAA 13577

TCCAAGTACT CGCAGCAGGT CTATTTGGGC CTCCATTCAT CGTGAAACTG AGACTTACCT 13637

CAGGCAGTCT GGGGTGAAGT TTACGATTGT CCGAAATAAT CAGTATGCGT CTAACCTGGA 13697

TCTGTTGCTG CTGAGGGCTC AAGACAGCGG AATATTTGCC ATTCCCGGGG CGAAGGGGCG 13757

GGTGGCGTAC GTCTCTCATC GCGACGTTGC CGCTGCCATC TGTAGTGTCC TGACGACCGC 13817

CGGACACGAT AACAGGATCT ACCAGCTCAC AGGCTCTGAG GCTCTCAATG GGCTCGAGAT 13877

CGCGGAGATT CTTGGTGGGG TGCTCGGGCG TCCAGTGCGC GCGATGGATG CCTCGCCTGA 13937

CGAGTTTGCT GCCAGCTTTC GCGAGGCTGG ATTCCCTGAG TTTATGGTTG AAGGCCTACT 13997

AAGCATTTAT GCCGCTTCAG GTGCTGGGGA GTACCAATCC GTCAGTCCTG ATGTTGGGTT 14057

GTTGACGGGA CGACGTGCCG AATCGATGCG AACTTACATA CAGCGTCTAG TTTGGCCTTG 14117

AGGGAGGTGA CCGACGTATG AAGGCTTATG AGCTTCACAA GATTTCGGAA CAGGTAGAGG 14177

TCAGGCTCCA GCCAACTCGG CCCCGCCCGC AGTTGAATCA TGGCGAGGTC CTCATCAGGG 14237

TCCATGCAGC CTCGCTCAAC TTTCGCGATT TGATGATCTT GGCCGGTCGC TATCCGGGTC 14297

AAATGAAACC CGATGTGATC CCGCTGTCCG ATGGTGCTGG CGAGATTGTG GAGGTCGGGC 14357

CTGGCGTATC TTCGGAGGTG CAGGGTCAGC GCGTAGCCAG CACCTTTTTC CCTAACTGGC 14417

GGGCCGGAAA GATTACCGAG CCGGCTATTG AGGTGTCGTT GGGCTTCGGT ATGGACGGGA 14477

TGCTCGCGGA ATACGTTGCT CTGCCCTATG AGGCAACGAT ACCGATACCG GAGCACCTGT 14537

CGTACGAGGA GGCTGCAACA TTGCCTTGCG CGGCGCTAAC CGCTTGGAAT GCGTTGACCG 14597

AAGTGGGGCG TGTCAAGGCC GGTGATACGG TCTTGTTGCT TGGCACTGGC GGTGTCTCGA 14657

TGTTCGCGTT GCAGTTCGCC AAGCTCTTGG GGGCGACGGT CATTCACACC TCGAGCAGTG 14717

AACAAAAGCT GGAGAGGGTG AAAGCGATGG GGGCTGATCA TCTGATCAAC TACCGCAATT 14777

CGCCAGGGTG GGACCGTACT GTCCTGGATC TCACCGCGGG GCGAGGGGTT GACCTGGTAG 14837

TCGAGGTAGG GGGGGCGGGG ACCTTGGAGC GCTCACTTCG TGCGGTCAAG GTAGGCGGTA 14897

TTGTCGCCAC GATTGGGCTA GTGGCTGGCG TTGGCCCGAT TGACCCATTG CCGCTTATCT 14957

CCAGGGCTAT TCAGCTCTCG GGCGTCTATG TCGGTTCCCG GGAAATGTTT CTCTCAATGA 15017

ACAAAGCCAT TGCATCAGCC GAAATCAAGC CAGTGATCGA TTGCTGCTTC CCCATCGACG 15077

AGGTTGGAGA TGCTTATGAG TACATGCGTA GCGGCAATCA CCTTGGCAAA GTAGTTATCA 15137

CGATCTAACT GCCGCTAAAC CCGTTGTGCG GCAATTTGCG GGAGCTAGTA CCGGGCTTTC 15197

GGTTTGGCTC TTGGATGGTC TTCGCATGCA CGCTTTACGA AGGGGGCCAG GGACAGACGC 15257

CCCGGGGCGT AATCAATGGC CTTGCGTGCA GGCTCTCACC GTCGTGATCG GGATTGGAAA 15317

TTCGTGCGAG GACAGCGGCC ACGTACCGGC GCCCTGAAGG GCTGGAAGGT TGGAGTTTCG 15377

TTAAGGTCTG GTACCCAGCA GCCATGGAGA GCGGCCCTTA GCCGGAATGG CAGCTTGATG 15437

GTTGCCACGG GACCAGACTG GATGTCTTGA GTGTCGAGAA TTACCAGATC GCTGCGATTT 15497

TCATCGAGGC GACCAACCAC GGTCAGCAAG TACCCGTCAC CTTCGGCGGC GGTCGGACTT 15557

CTAGGGACGA AGGCCGGCTC CTGGGCCGCC GAGGCTTCGC CGGAGTACCA GAGGTCGTAG 15617

TCACCTCGGT GGTTGTCCCA GATGCCGAGT GAGTTGTACG CGAATATCTT CTCGGCCTGC 15677

TGATGCGCAA GTGGTTTGCG TGGATCGTCC ACCCCCATAA AGCCATAGCG GTTGCATTGC 15737

AGGGCGAACG AAGAATCCAT GATTGGCATT TCCGCAAAGA AATCGTGTAG CCGGGTTCGC 15797

TTGATCTCGT CGCTGCTGCT ATCGAGGTCA ATTTCCCAAC GAGTCAGGCG TGGTACGGCT 15857

TTCTCAGGGG CGAAGGGTTG GTTTTGTGAG TTGGGGAAGG GGAACGGCAG GATTTCACTT 15917

TCCATAAGGT CGATATAAAT CTTGGTTCCG ACTTCCCAAG CATTCACAAC ATGAAATACC 15977

CAGAGCGCCG GTGCCTTGAG CCAGCGAATC AGACTGCCCT GGCGCGGCGC GAGTACGCCA 16037

ATGTAGCTGC CCAGTTCCGG CTCCCACATA TAAATTGGCT GTTTCGCCTT GAGGCGGGAC 16097

AGGCTGTTGG TGGCCGGCAT AATTGGGAAA ATGGACCAAT TTCGGGTAAT GGCAAAGTCG 16157

TGCATGAATG CGCCATAGGG CTGCTCAAAC CAAGTTTCAT GTGTCACCTT GCCGTGCTTG 16217

TCGACAATGT AATAGGCCAT GTCTGGAGTT GCTTCGCCCT TAGCTGCCGA ACCGAAGAAC 16277

AACAAGTCAC CCGTTTCCGG GTCATATTTT GGATGGGCGG TGTGGGTTTG GCTGGTAACT 16337

TGGCCGTCGT AGTCGAAGTG TCCGCGAGTT TCAAGTGTAC GAGGATCCAG TTCGTACGGT 16397

AGGCCGTCTT CCTTCACCGC CAGCACCTTG CCGTGATGGC TAATGATGCT TGTATTGGCA 16457

ACGGTGCGGT CTAGTCCTTT TACACTGGTG TCGTCGGTAT AGGGGTTTCT GTACATGCCA 16517

AATAGCGATT TTCGCGCTAG TCGTTCGGCC GTGAATCGAG CGGTTTTAAC CCAGCGACTG 16577

ATGAAGTCGA CATGACCATC TTCGAAGTGG AAGGCAGAGG CCATTCCATC TCCATCTATG 16637

AAGGTGTGGA ATTTTTGTGG GGTAACTTGA GGCTCTGGCG TATTACGGTA GAACGTTCCA 16697

TTTATTGATT TTGGGATTTC GCCGTCAACC TCTAGATCGA ACAAGTCTGC CTCTATACGG 16757

GTGGGGAGAA GTGTTCCTAC TAATTGCGGG TCGTTGCGGT TGAATCTCGC CATGGCACGG 16817

TCTCCTTTGT TGTTCTGAAT GGCCTAAATG CGCGGCTTGC CGGGTTGGAG TTTATGTTTA 16877

GGACTGACCG GATTTCATGT GTGCCGGTGA AGTGAAGATG TCTGTGAGTG CAATGGTGGT 16937

GGTATTGAAA ATGGGCCGAG GCTGGCCTAT TGTTTAGAAT TTCAAGAATG ACAACTATTC 16997

GGTGGCGGCG TATGTCCATT CACTCTGAGG GGATCACTCT CGCGGATTCG CCGCTGCATT 17057

GGGCGCATAC CCTGAATGGA TCAATGCGTA CTCATTTCGA AGTCCAGCGT CTTGAGCGGG 17117

GTAGAGGTGC CTCCCTTGCC CGATCTAGAT TTGGCGCGGG TGAGCTGTAC AGTGCCATTG 17177

CACCAAGCCA GGTACTTCGC CACTTCAACG ACCAGCGAAA TGCTGATGAG GCTGAGCACA 17237

GCTATTTGAT TCAGATACGA AGTGGCGCTT TGGGCGTTGC ATCCGGCGGA AGAAAGGTGA 17297

TCTTGGCAAA TGGTGATTGC TCCATAGTTG ATAGTCGCCA AGACTTCACA CTTTCCTCGA 17357

ACTCTTCGAC CCAAGGTGTC GTAATACGCT TTCCGGTGAG TTGGCTGGGA GCGTGGGTGT 17417

CCAATCCGGA GGATCTTATC GCCCGACGAG TTGATGCTGA GGTAGGGTGG GGTAGGGCGC 17477

TAAGCGCATC GGTTTCTAAT CTAGATCCAT TGCGCATCGA CGATTTAGGT AGCAATGTAA 17537

ATGGCATTGC AGAGCATGTT GCTATGTTAA TTTCACTAGC AAGTTCTGCG GTTAGTTCTG 17597

AAGATGGGGG TGTGGCTCTT CGGAAAATGA GGGAAGTGAA GAGAGTACTC GAGCAGAGTT 17657

TCGCAGACGC TAATCTCGGG CCGGAAAGTG TTTCAAGTCA ATTAGGAATT TCGAAACGCT 17717

ATTTGCATTA TGTCTTTGCT GCGTGCGGTA CGACCTTTGG TCGCGAGCTG TTGGAAATAC 17777

GCCTGGGCAA AGCTTATCGA ATGCTCTGTG CGGCGAGTGA CTCGGGTGCT GTGCTGAAGG 17837

TGGCCATGTC CTCAGGTTTT TCGGATTCAA GCCATTTCAG CAAGAAATTT AAGGAAAGAT 17897

ACGGTGTTTC GCCTGTCTCC TTGGTGAGGC AGGCTTGATT TCCCATAGCG TTATTGCGGT 17957

CGTCGTTGCA AATGCGGACC TGCGTGATCA TCAAGGCTAA GACTGCCACA TTAGGTGTCG 18017

ACTCGAGCGT CCCTCTATCC GCCTGACCGC GCTCCGTCCC TAGTACCTAG GAAATTGAGT 18077

GGGCCTACTT GCCAGGGCCA GTTGGATTCG GTGCTGGTGA GCGCTGCGGG TGACAGAATC 18137

CTGATCGTGG CGATCACGAT GGCGATAAAG TTGCCCGGTG TCGTAGATCG CAGGGTGACC 18197

AAGACGGGGA CTCATGGCGC GGATCCCGCC AGTGATGCCT TCGCATGACG CCACCTCTCT 18257

CCTCCGCTCA GCCTTCATGC CTGACTAATT AAGTCGTATA TCAATCTGGC TCTGTGCCGC 18317

ATTCAGTTCC TCCAGCTGCA TTGTCTCTCG GCGGGAGGGC ATTCCCCTGC ATTGGCCAAA 18377

TGGGTCCCCT TGTTCACGAC CGGACAAGCG CACCGTGCTG CCCGTTCGTC GTGTGCCCTG 18437

TCAAAAAGCC TGGCGACGAA AGGGCGGCAG GCCGCATGGC CACGGCTGGG CGGTAACTGA 18497

TGCTTGCGTT AATCGTTAAC CGTTTGAAAT TCCTTGCCAA ATTTCGGCGA GAGAATCATG 18557

CGGGTACGCC TTTCCGTGCG CTTTGATCTG CGCTTCCGTG CCTTGAATCA GAAAAATAGT 18617

TAATTGACAG AACTATAGGT TCGCAGTAGC TTTTGCTCAC CCACCAAATC CACAGCACTG 18677

GGGTGCACGA TGAATAGCTA CGATGGCCGT TGGTCTACCG TTGATGTGAA GGTTGAAGAA 18737

GGTATCGCTT GGGTCACGCT GAACCGCCCG GAGAAGCGCA ACGCAATGAG CCCAACTCTC 18797

AATCGAGAGA TGGTCGAGGT TCTGGAGGTG CTGGAGCAGG ACGCAGATGC TCGCGTGCTT 18857

GTTCTGACTG GTGCAGGCGA ATCCTGGACC GCGGGCATGG ACCTGAAGGA GTATTTCCGC 18917

GAGACCGATG CTGGCCCCGA AATTCTGCAA GAGAAGATTC GTCGCGAAGC GTCGACCTGG 18977

CAGTGGAAGC TCCTGCGGAT GTACACCAAG CCGACCATCG CGATGGTCAA TGGCTGGTGC 19037

TTCGGCGGCG GCTTCAGCCC GCTGGTGGCC TGTGATCTGG CCATCTGTGC CGACGAGGCC 19097

ACCTTTGGCC TGTCCGAGAT CAACTGGGGC ATCCCGCCGG GCAACCTGGT GAGTAAGGCT 19157

ATGGCCGACA CCGTGGGTCA CCGCGAGTCC CTTTACTACA TCATGACTGG CAAGACATTT 19217

GGCGGTCAGC AGGCCGCCAA GATGGGGCTT GTGAACCAGA GTGTTCCGCT GGCCGAGCTG 19277

CGCAGTGTCA CTGTAGAGCT GGCTCAGAAC CTGCTGGACA AGAACCCCGT AGTGCTGCGT 19337

GCCGCCAAAA TAGGCTTCAA GCGTTGCCGC GAGCTGACTT GGGAGCAGAA CGAGGACTAC 19397

CTGTACGCCA AGCTCGACCA ATCCCGTTTG CTCGATCCGG AAGGCGGTCG CGAGCAGGGC 19457

ATGAAGCAGT TCCTTGACGA GAAAAGCATC AAGCCGGGCT TGCAGACCTA CAAGCGCTGA 19517

TAAATGCGCC GGGGCCCTCG CTGCGCCCCC GGCCTTCCAA TAATGACAAT AATGAGGAGT 19577

GCCCAATGTT TCACGTGCCC CTGCTTATTG GTGGTAAGCC TTGTTCAGCA TCTGATGAGC 19637

GCACCTTCGA GCGTCGTAGC CCGCTGACCG GAGAAGTGGT ATCGCGCGTC GCTGCTGCCA 19697

GTTTGGAAGA TGCGGACGCC GCAGTGGCCG CTGCACAGGC TGCGTTTCCT GAATGGGCGG 19757

CGCTTGCTCC GAGCGAACGC CGTGCCCGAC TGCTGCGAGC GGCGGATCTT CTAGAGGACC 19817

GTTCTTCCGA GTTCACCGCC GCAGCGAGTG AAACTGGCGC AGCGGGAAAC TGGTATGGGT 19877

TTAACGTTTA CCTGGCGGCG GGCATGTTGC GGGAAGCCGC GGCCATGACC ACACAGATTC 19937

AGGGCGATGT CATTCCGTCC AATGTGCCCG GTAGCTTTGC CATGGCGGTT CGACAGCCAT 19997

GTGGCGTGGT GCTCGGTATT GCGCCTTGGA ATGCTCCGGT AATCCTTGGC GTACGGGCTG 20057

TTGCGATGCC GTTGGCATGC GGCAATACCG TGGTGTTGAA AAGCTCTGAG CTGAGTCCCT 20117

TTACCCATCG CCTGATTGGT CAGGTGTTGC ATGATGCTGG TCTGGGGGAT GGCGTGGTGA 20177

ATGTCATCAG CAATGCCCCG CAAGACGCTC CTGCGGTGGT GGAGCGACTG ATTGCAAATC 20237

CTGCGGTACG TCGAGTGAAC TTCACCGGTT CGACCCACGT TGGACGGATC ATTGGTGAGC 20297

TGTCTGCGCG TCATCTGAAG CCTGCTGTGC TGGAATTAGG TGGTAAGGCT CCGTTCTTGG 20357

TCTTGGACGA TGCCGACCTC GATGCGGCGG TCGAAGCGGC GGCCTTTGGT GCCTACTTCA 20417

ATCAGGGTCA AATCTGCATG TCCACTGAGC GTCTGATTGT GACAGCAGTC GCAGACGCCT 20477

TTGTTGAAAA GCTGGCGAGG AAGGTCGCCA CACTGCGTGC TGGCGATCCT AATGATCCGC 20537

AATCGGTCTT GGGTTCGTTG ATTGATGCCA ATGCAGGTCA ACGCATCCAG GTTCTGGTCG 20597

ATGATGCGCT CGCAAAAGGC GCGCGGCAGG TCGTCGGTGG TGGCTTAGAT GGCAGCATCA 20657

TGCAGCCGAT GCTGCTTGAT CAGGTCACTG AAGAGATGCG GCTCTACCGT GAGGAGTCCT 20717

TTGGCCCTGT TGCCGTTGTC TTGCGCGGCG ATGGTGATGA AGAACTGCTG CGTCTTGCCA 20777

ACGATTCGGA GTTTGGTCTT TCGGCCGCCA TTTTCAGCCG TGACGTCTCG CGCGCAATGG 20837

AATTGGCCCA GCGCGTCGAT TCGGGCATTT GCCATATCAA TGGACCGACT GTGCATGACG 20897

AGGCTCAGAT GCCATTCGGT GGGGTGAAGT CCAGCGGCTA CGGCAGCTTC GGCAGTCGAG 20957

CATCGATTGA GCACTTTACC CAGCTGCGCT GGCTGACCAT TCAGAATGGC CCGCGGCACT 21017

ATCCAATCTA AATCGATCTT CGGGCGCCGC GGGCATCATG CCCGCGGCGC TCGCCTCATT 21077

TCAATCTCTA ACTTGATAAA AACAGAGCTG TTCTCCGGTC TTGGTGGATC AAGGCCAGTC 21137

GCGGAGAGTC TCGAAGAGGA GAGTACAGTG AACGCCGAGT CCACATTGCA ACCGCAGGCA 21197

TCATCATGCT CTGCTCAGCC ACGCTACCGC AGTGTGTCGA TTGGTCATCC TCCGGTTGAG 21257

GTTACGCAAG ACGCTGGAGG TATTGTCCGG ATGCGTTCTC TCGAGGCGCT TCTTCCCTTC 21317

CCGGGTCGAA TTCTTGAGCG TCTCGAGCAT TGGGCTAAGA CCCGTCCAGA ACAAACCTGC 21377

GTTGCTGCCA GGGCGGCAAA TGGGGAATGG CGTCGTATCA GCTACGCGGA AATGTTCCAC 21437

AACGTCCGCG CCATCGCACA GAGCTTGCTT CCTTACGGAC TATCGGCAGA GCGTCCGCTG 21497

CTTATCGTCT CTGGAAATGA CCTGGAACAT CTTCAGCTGG CATTTGGGGC TATGTATGCG 21557

GGCATTCCCT ATTGCCCGGT GTCTCCTGCT TATTCACTGC TGTCGCAAGA TTTGGCGAAG 21617

CTGCGTCACA TCGTAGGTCT TCTGCAACCG GGACTGGTCT TTGCTGCCGA TGCAGCACCT 21677

TTCCAGCGCG CAATTGAGAC CATTCTGCCG GACGACGTGC CCGCAATCTT CACTCGAGGC 21737

GAATTGGCCG GGCGGCGCAC GGTGAGTTTT GACAGCCTGC TGGAGCAGCC TGGTGGGATT 21797

GAGGCAGATA ATGCCTTTGC GGCAACTGGC CCCGATACGA TTGCCAAGTT CTTGTTCACT 21857

TCTGGCTCTA CCAAACTGCC TAAGGCGGTG CCGACTACTC AGCGAATGCT CTGCGCCAAT 21917

CAGCAGATGC TTCTGCAAAC TTTCCCGGTT TTTGGTGAAG AGCCGCCGGT GCTGGTGGAC 21977

TGGTTGCCGT GGAACCACAC CTTCGGCGGC AGCCACAACA TCGGCATCGT GTTGTACAAC 22037

GGCGGCACGT ACTACCTTGA CGACGGTAAA CCAACCGCCC AAGGGTTCGC CGAGACGCTT 22097

CGCAACTTGA GCGAAATCTC TCCCACTGCG TACCTCACTG TGCCGAAAGG CTGGGAGGAA 22157

TTAGTGGGTG CCCTTGAGCG AGACAGTACC CTGCGCGAAC GCTTCTTCGC TCGCATGAAG 22217

CTGTTCTTCT TCGCGGCGGC TGGGTTGTCG CAAGGGATCT GGGATCGTTT GGACCGGGTC 22277

GCTGAACAGC ACTGTGGTGA GCGCATTCGC ATGATGGCGG GTCTGGGCAT GACGGAGACT 22337

GCTCCTTCCT GCACTTTTAC CACCGGACCG CTGTCGATGG CTGGTTACAT TGGGCTGCCA 22397

GCGCCTGGCT GCGAGGTCAA GCTCGTTCCG GTCGATGGGA AATTGGAAGG GCGTTTCCAT 22457

GGTCCGCACG TCATGAGCGG CTACTGGCGT GCTCCTGAAC AAAATGCCCA AGCGTTCGAC 22517

GAGGAAGGCT ATTACTGCTC CGGTGATGCC ATCAAATTGG CAGATCCTGC CGATCCTCAG 22577

AAAGGTCTGA TGTTTGACGG TCGAATTGCT GAAGACTTCA AGCTGTCCTC AGGGGTATTT 22637

GTCAGCGTTG GGCCATTGCG CACGCGGGCG GTTCTGGAAG GCGGCTCTTA CGTCCTGGAC 22697

GTAGTGGTTG CTGCTCCTGA TCGTGAATGC CTTGGATTGC TCGTGTTTCC GCGTCTTCTC 22757

GACTGCCGTG CCTTGTCGGG GCTAGGAAAA GAGGCGTCGG ACGCCGAGGT GCTTGCCAGT 22817

GAGCCGGTTC GGGCCTGGTT TGCTGACTGG CTCAAACGAC TCAATCGAGA AGCAACTGGC 22877

AATGCCAGTC GCATCATGTG GGTAGGGCTC CTCGATACGC CGCCGTCGAT TGATAAGGGC 22937

GAGGTCACTG ACAAGGGCTC GATCAACCAG CGCGCTGTTT TGCAATGGCG GTCGGCGAAA 22997

GTTGATGCGC TGTATCGTGG TGAAGATCAA TCCATGCTGC GTGACGAGGC CACACTGTGA 23057

GTTGGTCAGG GGGGGCTTAC TCGGCGTTTT CCGACACTGC GTTGGTTGCG GCAGTGCGCA 23117

CCCCCTGGAT TGATTGCGGG GGTGCCCTGT CGCTGGTGTC GCCTATCGAC TTAGGGGTAA 23177

AGGTCGCTCG CGAAGTTCTG ATGCGTGCGT CGCTTGAACC ACAAATGGTC GATAGCGTAC 23237

TCGCAGGCTC TATGGCTCAA GCAAGCTTTG ATGCTTACCT GCTCCCGCGG CACATTGGCT 23297

TGTACAGCGG TGTTCCCAAG TCGGTTCCGG CCTTGGGGGT GCAGCGCATT TGCGGCACAG 23357

GCTTCGAACT GCTTCGGCAG GCCGGCGAGC AGATTTCCCA AGGCGCTGAT CACGTGCTGT 23417

GTGTCGCGGC AGAGTCCATG TCGCGTAACC CCATCGCGTC GTATACACAC CGGGGCGGGT 23477

TCCGCCTCGG TGCGCCCGTT GAGTTCAAGG ATTTTTTGTG GGAGGCATTG TTTGATCCTG 23537

CTCCAGGACT CGACATGATC GCTACCGCAG AAAACCTGGC GCGCCTGTAC GGAATCACCA 23597

GGGGAGAAGC TAATTCCTAC GCGGTAAGCA GCTTCGAGCG CGCATTGAGG GCGCAAGAGG 23657

AGAAATGGAT TGACCAAGAG ATCGTGGCTG TTACGGATGA ACAGTTCGAT TTAGAGGGCT 23717

ACAACAGTCG AGCAATTGAA CTGCCTCGGA AGGCAAAATT GTTGATCGTG ACAGTCATCC 23777

GCGGCCTAGC AGTCTTTGAA GCCCTTTCCC GATTGAAGCC TGTTCATTCT GGCGGGGTGC 23837

AGACTGCGGG CAACAGCTGT GCCGTAGTGG ACGGCGCCGC GGCGGCTTTG GTGGCTCGAG 23897

AGTCGTCTGC GACACAGCCG GTCTTGGCTA GGATACTGGC TACCTCCGTA GTCGGGATCG 23957

AGCCCGAGCA TATGGGGCTC GGCCCTGCGC CCGCGATTCG CCTGCTGCTT GCGCGTAGTG 24017

ATCTTAGTTT GAGGGATATC GACCTCTTTG AGATAAACGA GGCGCAGGCC GCCCAAGTTC 24077

TAGCGGTACA GCATGAATTG GGTATTGAGC ACTCAAAACT TAATATTTGG GGCGGGGCCA 24137

TTGCACTTGG ACACCCGCTT GCCGCGACCG GATTGCGTCT CTGCATGACC CTCGCTCACC 24197

AATTGCAAGC TAATAACTTT CGATATGGAA TTGCCTCGGC ATGCATTGGT GGGGGACAGG 24257

GGATGGCGGT TCTTTTAGAG AATCCCCACT TCGGTTCGTC CTCTGCACGA AGTTCGATGA 24317

TTAACAGAGT TGACCACTAT CCACTGAGCT AACGGGCATC TCCTTTGTTG CTTTGAGGTG 24377

GCGCACGAAG GAGGGCTCGA AAATCTCTGC TAAAAACAAG AAGAAGGAAC AGGGAACATG 24437

ATTAGTTTCG CTCGTATGGC AGAAAGTTTA GGAGTCCAGG CTAAACTTGC CCTTGCCTTC 24497

GCACTCGTAT TATGTGTCGG GCTGATTGTT ACCGGCACGG GTTTCTACAG TGTACATACC 24557

TTGTCAGGGT TGGTGGAAAA GAGCGCGATA GCTGGTGAGT TGCGGGCGAA AATTCAGGAA 24617

CTGAAGGTTC TGGAGCAGCG CGCCTTATTC ATCGCCGATG AAGGGTCGCT GAAGCAGCGC 24677

TCGATCCTCC TAAGTCAGGT GATAGCTGAA GTTAATGATG CTATAGATAT TTTTGACTTT 24737

CAGCGCGGAC GATCTGAGTT ACTTAAATTC GCTGCTTCTT CGCGCGAAGC AAGTTACTCC 24797

ATTGAGGTCG GTAGTAACGC TGCGGCCGAT AAGTTGCAGT CGGGCGAACC AAGTGACGCA 24857

TTGATGGTTG CCGATAAAAA GCTGAATGTT GAGTATGAGC AATTGAGTTC TGCTGTGAAT 24917

GCACTGATGG GGCATTTAAT TGAGGATCAG AATGAAAAAG TTCCACTAAT CTACTATATG 24977

CTTGGCGGCG TAACTTTGTT TACGATGCTC ATGAGTGCTT ATTCGGTCTG GTTCATTTCG 25037

CGTCAGTTAG TTCCGCCATT AAAGTCGACG GTGCAGCTTG CCGAGCGGAT TGCATCAGGC 25097

GACTTGGCTG ATGTCGGGGA CAGCAGGCGC AAGGATGAAA TCGGTCAGTT GCAAAGTGCA 25157

ACTAGGCGGA TGGCGATTGG ACTGCGTAAT CTGGTCGGTG ATATTGGTCA AAGTCGTGCG 25217

CAACTGGTTT CATCGTCCAG CGACCTTTCG GCCATCTGTG CTCAGGCTCA GATTGATGTC 25277

GAGTGCCAGA AGCTTTCGGT CGCCCAGGTC TCTACCGCCG TGAACGAGTT GGTTGAAACC 25337

GTCCAGGCAA TAGCAAAAAG CACCGAAGAG GCAGCAACAG TCGCCGTCTT GGCCGATGAA 25397

AAGGCACGCG GTGGTGAAAG TGTCGTTAAC AAGGCCGTTG ATTTCATTGA GCACCTCTCC 25457

GGAGATATGG CGGAACTGGG AGACGCAATG GAGCGGCTTC AGAACGACAG TGCGCAGATC 25517

AATAAGGTAG TAGACGTCAT TAAGGCTGTG GCGGAGCAGA CCAATCTGCT AGCCCTGAAT 25577

GCGGCGATAG AGGCGGCCCG TGCAGGAGAG CAGGGCAGGG GCTTTGCGGT CGTGGCGGAT 25637

GAGGTTCGTG CTTTGGCGAT GCGCACCCAA CAATCGACCA AAGAAATTGA GAGGCTAGTG 25697

GTTTCATTGC AGCAGGGAAG TGAAGCTGCG GGCGAGTTGA TGCGGCGTGG CAAGGTCCGG 25757

ACGCATGACG TCGTTGGATT GGCCCAGCAA GCCGCGCGCC GCGCTACTCG AAATTACCCA 25817

GCTGTCGCCG GCATCCAAGC GATGAACTAT CAGATCGCCG CTGGAGCAGA GCAGCAAGGG 25877

GCTGCTGTGG TTCAAATCAA CCAGAATATG CTTGAAGTGC ATAAGATGGC TGACGAGTCC 25937

GCCATTAAAG CGGGACAGAC CATGAAGTCA TCGAAGGAGC TTGCTCACCT CGGCAGTGCG 25997

CTACAAAAAT CCGTTGATCG ATTCCAGCTG TAGCGCTCCG GGTGGCTGAA ACGCGCATTT 26057

TCGTTAAGGT CTTCAGCGCG GTCTGCTGGT GCGTGGGCCG CTAGCCTAAC TGTTGCGCTT 26117

CAGGCTCCGC ATGGATCTTG TGCAGCAGCA ATAGCAATTG TTCACGTTCG TCATCACTCA 26177

GCATCGACGT CGCGTCTTGG TCGCTCTGTA CCACGATCTT CTTCAGCTCT TTGAGCTGCG 26237

TCTCCCCAGC TTTGCTGAGA AATATCCCAT AGGAACGCTT GTCCGGCTTG CAGCGCACGC 26297

GCACAGCAAG GCCGAGCTTC TCGAGCTTGT TCAGCAAGGG AACCAGTTGT GGTGGTTCGA 26357

TTGCGAGCAT CCGCGCTAGG TCAGCCTGCA TAAGCCCAGG GCTCGCTTCG ATGATTAGAA 26417

GTGCCGACAG CTGCGCCGGG CGTAGGTCAT ATGGCGTCAG GGCTTCAATC AGGCCCTGAG 26477

CGAGCTTCAG CTGTGAGCCG GCGTAAGGCA TAGCCAATCA ATTGATTCAG GAGCGTATCG 26537

CCCGGTTCTA TCAGCGGGCC GCTTTCGAAA GTCATGGTGT TAGCCGGTAG GGTCTTTTTC 26597

TTGGCCATGC TTGTTGCCTG AACCTTCGTT GACATAGGGC AGAGGTGCGT TTGCCGCTTC 26657

GCTTCGCGAT GAACCGCATC GAGATGCTGA GGTCAGGATT TTTCCTTAAC TCGCGTAAGC 26717

ATTCTGTCAT TTTTTTGGTG GCTTTGAACA GCCTGATGAA AGGTGGTCTC GCCCTTTGAG 26777

GCCGATTCTT GGGCGCTTGG CGGCGTCGAA GCGATGCTCC ACTACCGATT AAGATAATTA 26837

AAATAAGGAA ACCGCATGGT TTCTTATGTG AATTTGTCTG GCATACTCCA GCTCAAGGGC 26897

AATTTTTGGG CTATTGGCTG AGCAGTTGCC TCTATATGGT TATTCAGAAT AACAATTGAC 26957

TCCTCAGGAG GTCAGCGATG AGCATTCTTG GTTTGAATGG TGCCCCGGTC GGAGCTGAGC 27017

AGCTGGGCTC GGCTCTTGAT CGCATGAAGA AGGCGCACCT GGAGCAGGGG CCTGCAAACT 27077

TGGAGCTGCG TCTGAGTAGG CTGGATCGTG CGATTGCAAT GCTTCTGGAA AATCGTGAAG 27137

CAATTGCCGA CGCGGTTTCT GCTGACTTTG GCAATCGCAG CCGTGAGCAA ACACTGCTTT 27197

GCGACATTGC TGGCTCGGTG GCAAGCCTGA AGGATAGCCG CGAGCACGTG GCCAAATGGA 27257

TGGAGCCCGA ACATCACAAG GCGATGTTTC CAGGGGCGGA GGCACGCGTT GAGTTTCAGC 27317

CGCTGGGTGT CGTTGGGGTC ATTAGTCCCT GGAACTTCCC TATCGTACTG GCCTTTGGGC 27377

CGCTGGCCGG CATATTCGCA GCAGGTAATC GCGCCATGCT CAAGCCGTCC GAGCTTACCC 27437

CGCGGACTTC TGCCCTGCTT GCGGAGCTAA TTGCTCGTTA CTTCGATGAA ACTGAGCTGA 27497

CTACAGTGCT GGGCGACGCT GAAGTCGGTG CGCTGTTCAG TGCTCAGCCT TTCGATCATC 27557

TGATCTTCAC CGGCGGCACT GCCGTGGCCA AGCACATCAT GCGTGCCGCG GCGGATAACC 27617

TAGTGCCCGT TACCCTGGAA TTGGGTGGCA AATCGCCGGT GATCGTTTCC CGCAGTGCAG 27677

ATATGGCGGA CGTTGCACAA CGGGTGTTGA CGGTGAAAAC CTTCAATGCC GGGCAAATCT 27737

GTCTGGCACC GGACTATGTG CTGCTGCCGG AAGAATCGCT GGATAGCTTT GTCGCCGAGG 27797

CGACGCGCTT CGTGGCCGCA ATGTATCCCT CGCTTCTAGA TAATCCGGAT TACACGTCGA 27857

TCATCAATGC CCGAAATTTC GACCGTCTGC ATCGCTACCT GACTGATGCG CAGGCAAAGG 27917

GAGGGCGCGT CATTGAAATC AATCCTGCGG CCGAAGAGTT GGGGGATAGT GGTATCAGGA 27977

AGATCGCGCC CACTTTGATC GTGAATGTGT CGGATGAAAT GCTGGTCTTG AACGAGGAGA 28037

TCTTTGGTCC GCTGCTCCCG ATCAAGACTT ATCGTGATTT CGACTCGGCT ATCGACTACG 28097

TCAACAGCAA GCAGCGACCA CTTGCCTCGT ACTTCTTCGG CGAAGATGCG GTTGAGCGTG 28157

AGCAAGTGCT TAAGCGTACG GTTTCGGGCG CCGTGGTCGT GAACGATGTC ATGAGCCATG 28217

TGATGATGGA TACGCTTCCA TTTGGTGGTG TGGGGCACTC GGGGATGGGG GCATATCACG 28277

GCATTTATGG TTTCCGAACC TTCAGCCATG CCAAGCCTGT TCTCGTGCAA AGTCCTGTGG 28337

GTGAGTCGAA CTTGGCGATG CGCGCACCCT ACGGAGAAGC GATCCACGGA CTGCTCTCTG 28397

TCCTCCTTTC AACGGAGTGT TAGAACCGTT GGTAGTGGTT TTGGACGGGC CCAGGAGCAT 28457

GCGCTTCTGG GCCCGTTTCT TGAGTATTCA TTGGATAGTC ACGCGTGGTA GCTTCGAGCC 28517

TGCACAGCTG ATGAGCACCC TGGAAGGCGC GCTGTACGCG GACGACTGGG TTCATCTTCG 28577

CCATTCATGA CGGAACTCCG TTCCCCAGTA CCGCGATGAC TATTTTGCCT CTTCCGATGT 28637

CCGATTCCAC GCCGCCTGAC GCTAAGCGGG GGCGGGGGCG CCCGCATCCC AGCCCAGACA 28697

GCAACAAATG AGTAGGCTCT TGGATGCCGC GGCGGCTGAG ATTGGTAACG GCAATTTCGT 28757

CAATGTGACG ATGGATTCGA TTGCCCGTGC TGCCGGCGTC TCAAAAAAAA CGCTGTACGT 28817

CTTGGTGGCG AGCAAGGAAG AACTCATTTC CCGGTTAGTG GCTCGAGACA TGTCCAACCT 28877

TGAGCTGCTG CTTTGTCACG AGGTTGAGTC TGCGGAGGCC CTTCAGGATG AGTTGCGAAA 28937

CTATCTGCTG CTCTGGGCGC GCTTGACCTT GTCCCCTCTT GCTTTGGGCA TTTTTCTGAT 28997

GGCCGTGCAG GGGCGTGAAA GTGCCCCGGG CCTGGCGAGA ATCTGGTATC GAGAGGGGGC 29057

AGAGCGTTGC CTCAGCTTGC TTCGGGGATG GTTGGCAAGG ATGGCAAGCC GGGAGCTGAT 29117

CGCTCCTGGA GATATCGACT CCGCAGTGGA GCTTATCGAT TCGCTCCTGA TCTCACAGCC 29177

TTTGAAATTA TTTGGCCTGG GGATCCAGAG CGGCTGGACC GATGATCAGA TCAATCAACG 29237

GGTCACAATC GCTCTCGATG CATTCCGTCG GTGCTATGTC GTTTAGCACC GTTCTCGCGG 29297

GCTGTGGCGG CGTGACCTAT TTGTCTAGTG GTCGGCGCGA AATTCGATAA GAAAGCTGGG 29357

CGCGAGTGAG GCCGAGCCGG CGGGCAGCTT CCGAGACATT GCCTTTCACC TGGCCCAGAG 29417

CATGGCTAAT CATCGCGTCC TCCACTTCTT GCAGCGTCAT CGCGCTCAGG TCCTTTGAGT 29477

CAAGCGGCGA GTCGATTGTG CTGGTCGGTT TGGAGAAGGA AGTACTTGGG CTGCCAGTTT 29537

CCTGTGGCTG ATTATCTTGA GCGGTGGCCA GGATGCCGCT GGCCCCAATG GAGAACATCG 29597

GTTGAGTCAG TCGTTCACCG CTAGTGAAGA GGTGGCTCAC GTCAATGGCT CCATCCTCCG 29657

GAGCGCTGAT GACTCCGCGC TCCACCAAAT TTTGAAGCTC CCGGATGTTT CCTGGAAAGT 29717

CGTAGCCAAG CAGGGCATTG GCTGCACGTG GAGTGAATCC GCTGACCACC CGGCTATGAC 29777

GCTGATTGAA GCGGTGCAGG AAATAGGTCA TCAGGAGGGG AATGTCTTCC TTCCTCTCTC 29837

GAAGCGGCGG GAGGTGGATC GGGTAAACAT TGAGGCGGAA AAAAAGGTCC TCGCGGAACT 29897

CGCCGCGCTG GACGCCTGCG CGAAGATCGA CATTGGTTGC GGCTACCACA CGGACGTCAA 29957

CCTTGAGTGT CCTGCTTCCG CCAACCCGTT CGACCTCCGA CTCTTGCAGG GCGCGAAGTA 30017

ACTTCCCTTG GGCCACGAGG CTTAGCGTCC CTATCTCGTC AAGGAATAGT GTGCCGCCCG 30077

AAGCGCGCTC GAACCGTCCT GCTCGAGATT GGGTGGCGCC GGTAAACGCC CCCCGTTCGA 30137

CGCCGAACAA CTCGGACTCC ATCAGGGTTT CGGGAATACG TGCGCAATTG ACCGCAACAA 30197

ACGGGCCGTC GTGTCTGGGG CTGATGCGGT GAAGCATGCG GGCGAACATC TCCTTGCCCA 30257

CACCTGATTC ACCCGTAAAC AGTACCGTCG CCTCCGTGGG TGCTACGCGC TTCAGCATGT 30317

GGCAGGCAGC ATTGAATGCC GAGGAAATTC CCACCATGTC GTGTTCCGAT GCAGTGCTTG 30377

AGTCTGCGGC GGAGTGATGG GGAGTGTTCC TTTGTCCCTG CTGCGTTCTT CGTCTCTGCG 30437

GCGTGCTTGG TTGCCGACAA ATGGTTGCGC TAAGCGCCGC CAAGTCCTCT TCGGCGTCTT 30497

CCCATTCTTC CGCTGGCTTG CCGATCATGC GGCAGATCTG CGAACCCGTG GAGCGGCATT 30557

CCACCTCTCG GTAAAGGATG AGGCGACCAA CCAGCGCGGA CGTATAGCCA ATGGCATAAC 30617

CCGTCTGCGT CCAGCACGCG GGCTCGGTGC CGATGCCGTA GTGCGCAATA TGTTCATCAT 30677

CTTCGCTCGA ATGGTGCCAG AGGAATTCGC CGTAGTAGGT CCCCAAATCC ATGTCGAAGT 30737

CGAAGTGGAT CGGCTCCACG CGTACTGCGC CTTCCAGAGA GTGCAAGTTC GGGCCGGCGG 30797

CAAATAGGGA GAGCGGATCG GCGTTGCTGA AGCGCTCCTT CAGAAGGGCG GCATCTTTGG 30857

CGCCGCAGTG GTAACCGGTT CGCAGCATGA TTCCGCGGGC GCGGGCGAAG CCCACGCTTT 30917

CAATTAATTC GCGTCGCAAT GCACCCAGTC CGCTGCTGTG GAGGAGCAGC ATTCGCGCGC 30977

CGTTCAACCA GATGCGTCCA TCGCCAGGGC TGAAAAGGAG GGATTCAGTG AGGTCATGAA 31037

GGGAGGGGAC GGCGCCTGGC TCCAATTGCT CGATGGCGCC GCGATTGAGT GTCTTGGGCG 31097

CGGTCTTGGA GAGTTCGGCT AGGGAGATAA ATTTGCTGGC CATGGTGGCG GCCCCTGATG 31157

GGTTGGATGA TTTTCTGCAT TCTGCATCAT GAAATTCATG AAATCATCAC TTTTCGGGGG 31217

GTGGGTGCAC GGGATTGAAG GTTGCTAGGA GAGTGCATTG CTCGTAAGCC CAGGAAGCAC 31277

GCGGGTTTCA GGATGGTGCA TGGAAATGGC ATGAGCTTTG CTGGATATGA TTAGAGACAT 31337

TAACTATTTT GGCGGAATGG AAGCACGATT CCTCGCCCGG TAGAGCGGTA ACCGCGACAT 31397

TCAGGACCGT AAAAAGGAAA GAGCATGCAA CTGACCAACA AGAAAATCGT CGTCACCGGA 31457

GTGTCCTCCG GTATCGGTGC CGAAACTGCC CGCGTTCTGC GCTCTCACGG CGCCACAGTG 31517

ATTGGCGTAG ATCGCAACAT GCCGAGCCTG ACTCTGGATG CTTTCGTTCA GGCTGACCTG 31577

AGCCATCCTG AAGGCATCGA TAAGGCCATC TCTCAGCTGC CGGAGAAAAT TGACGGACTC 31637

TGCAATATCG CCGGGGTGCC CGGCACTGCC GATCCTCAGC TCGTCGCAAA CGTGAACTAC 31697

CTGGGTCTAA AGTATCTGAC CGAGGCAGTC CTGTCGCGCA TTCAACCCGG TGGTTCGATT 31757

GTCAACGTGT CCTCTGTGCT TGGCGCCGAG TGGCCGGCCC GCCTTCAGTT GCATAAGGAG 31817

CTGGGGAGTG TTGTTGGATT CTCCGAAGGC CAGGCATGGC TTAAGCAGAA TCCAGTGGCC 31877

CCCGAATTCT GCTACCAGTA TTTCAAAGAA GCACTGATCG TTTGGTCTCA AGTTCAGGCG 31937

CAGGAATGGT TCATGAGGAC GTCTGTACGC ATGAACTGCA TCGCCCCCGG CCCTGTATTC 31997

ACTCCCATTC TCAATGAGTT CGTCACCATG CTGGGTCAAG AGCGGACTCA GGCGGACGCT 32057

CATCGTATTA AGCGCCCAGC ATATGCCGAT GAAGTGGCCG CGGTGATTGC ATTCATGTGT 32117

GCTGAGGAGT CACGTTGGAT CAACGGCATA AATATTCCAG TGGACGGAGG TTTGGCATCG 32177

ACCTACGTGT AAGTTCGTGG ACGCCCTTTG CACGCGCACT ATATCTCTAT GCAGCAGCTG 32237

AAAGCAGCTT TGGTTTTGAT CGGAGGTAGC GGGCGGAAAG GTGCAGAATG TCTAAATAAT 32297

AAAGGATTCT TGTGAAGCTT TAGTTGTCCG TAAACGAAAA TAAAAATAAA GAGGAATGAT 32357

ATGAAAGCAA GTAGATCAGT CTGCACTTTC AAAATAGCTA CCCTGGCAGG CGCCATTTAT 32417

GCAGCGCTGC CAATGTCAGC TGCAAACTCG ATGCAGCTGG ATGTAGGTAG CTCGGATTGG 32477

ACGGTGCGTT GGGGACAACA CCCTCAAGTA TAGCCTTGCC TCTCGCCTGA ATGAGCAAGA 32537

CTCAAGTCTG ACAAATGCGC CGACTGTCAA TGGTTATATC CGGATATTCA AAGTCAGGGT 32597

GATCGTAACT TTGACCGGGG GCTTGGTATC CAATCGTCTC GATATTCTGT CGGAGCTTGA 32657

TGTCAGTCGT GACTGGTTGG TG 32679

(2) ANGABEN ZU SEQ ID NO: 2:

(i) SEQUENZKENNZEICHEN:
(A) LÄNGE: 284 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

Met Ile Ala Ile Thr Gly Ala Ser Gly Gln Leu Gly Arg Leu Thr Ile
1               5                   10                  15

Glu Ala Leu Leu Lys Arg Leu Pro Ala Ser Glu Ile Ile Ala Leu Val
            20                  25                  30

Arg Asp Pro Asn Lys Ala Gly Asp Leu Thr Ala Arg Gly Ile Val Val
        35                  40                  45

Arg Gln Ala Asp Tyr Asn Arg Pro Glu Thr Leu His Arg Ala Leu Ile
    50                  55                  60

Gly Val Asn Arg Leu Leu Leu Ile Ser Ser Ser Glu Val Gly Gln Arg
65                  70                  75                  80

Thr Ala Gln His Arg Ala Val Ile Asp Ala Ala Lys Gln Glu Gly Ile
                85                  90                  95

Glu Leu Leu Ala Tyr Thr Ser Leu Leu His Ala Asp Lys Ser Ala Leu
            100                 105                 110

Gly Leu Ala Thr Glu His Arg Asp Thr Glu Gln Ala Leu Thr Glu Ser
        115                 120                 125

Gly Ile Pro His Val Leu Leu Arg Asn Gly Trp Tyr His Glu Asn Tyr
    130                 135                 140

Thr Ala Gly Ile Pro Val Ala Leu Val His Gly Val Leu Leu Gly Cys

145                 150                 155                 160

Ala Gln Asp Gly Leu Ile Ala Ser Ala Ala Arg Ala Asp Tyr Ala Glu
                165                 170                 175

Ala Ala Ala Val Val Leu Thr Gly Glu Asn Gln Ala Gly Arg Val Tyr
            180                 185                 190

Glu Leu Ala Gly Glu Pro Ala Tyr Thr Leu Thr Glu Leu Ala Ala Glu
        195                 200                 205

Val Ala Pro Gln Ala Gly Lys Thr Val Val Tyr Ser Asn Leu Ser Glu
    210                 215                 220

Ser Asp Tyr Arg Ser Ala Leu Ile Ser Ala Gly Leu Pro Asp Gly Phe
225                 230                 235                 240

Ala Ala Leu Leu Ala Asp Ser Asp Ala Gly Ala Ala Lys Gly Tyr Leu
                245                 250                 255

Phe Asp Ser Ser Gly Asp Ser Arg Lys Leu Ile Gly Arg Pro Thr Thr
            260                 265                 270

Pro Met Ser Glu Ala Ile Ala Ala Ala Ile Gly Arg
        275                 280

(2) ANGABEN ZU SEQ ID NO: 3:

(i) SEQUENZKENNZEICHEN:
(A) LÄNGE: 1065 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1062
(D) SONSTIGE ANGABEN:/product=
"Vanillinsaeure-O-Demethylase"
/gene= "vanA"

(xi) SEQUENZBESCHREIBUNG: SEQ ID NO: 3:

ATG TTT CCG AAA AAC GCC TGG TAT GTC GCT TGC ACT CCG GAT GAA ATC          48
Met Phe Pro Lys Asn Ala Trp Tyr Val Ala Cys Thr Pro Asp Glu Ile
285                 290                 295                 300

GCA GAT AAG CCG CTA GGC CGT CAG ATC TGC AAC GAA AAG ATT GTC TTC 96
Ala Asp Lys Pro Leu Gly Arg Gln Ile Cys Asn Glu Lys Ile Val Phe
305 310 315

TAT CGG GGG CCG GAA GGA CGT GTT GCC GCG GTA GAG GAT TTC TGC CCT 144
Tyr Arg Gly Pro Glu Gly Arg Val Ala Ala Val Glu Asp Phe Cys Pro
320 325 330

CAT CGC GGG GCA CCG TTG TCC CTG GGT TTC GTT CGC GAC GGT AAG CTG 192
His Arg Gly Ala Pro Leu Ser Leu Gly Phe Val Arg Asp Gly Lys Leu
335 340 345

ATT TGC GGC TAC CAC GGT TTG GAA ATG GGC TGC GAG GGC AAA ACG CTC 240
Ile Cys Gly Tyr His Gly Leu Glu Met Gly Cys Glu Gly Lys Thr Leu
350 355 360

GCG ATG CCC GGG CAG CGC GTT CAA GGC TTC CCT TGC ATC AAA AGC TAC 288
Ala Met Pro Gly Gln Arg Val Gln Gly Phe Pro Cys Ile Lys Ser Tyr
365 370 375 380

GCG GTA GAA GAG CGA TAC GGC TTT ATC TGG GTA TGG CCT GGT GAT CGC 336
Ala Val Glu Glu Arg Tyr Gly Phe Ile Trp Val Trp Pro Gly Asp Arg
385 390 395

GAG CTG GCG GAT CCG GCG CTT ATT CAC CAC CTG GAG TGG GCC GAT AAT 384
Glu Leu Ala Asp Pro Ala Leu Ile His His Leu Glu Trp Ala Asp Asn
400 405 410

CCG GAG TGG GCC TAT GGT GGC GGT CTC TAC CAC ATC GCT TGT GAT TAC 432
Pro Glu Trp Ala Tyr Gly Gly Gly Leu Tyr His Ile Ala Cys Asp Tyr
415 420 425

CGC CTG ATG ATC GAC AAC CTC ATG GAT CTC ACC CAT GAG ACC TAT GTG 480
Arg Leu Met Ile Asp Asn Leu Met Asp Leu Thr His Glu Thr Tyr Val
430 435 440

CAT GCC TCC AGC ATC GGT CAA AAG GAA ATT GAC GAG GCA CCG GTC AGT 528
His Ala Ser Ser Ile Gly Gln Lys Glu Ile Asp Glu Ala Pro Val Ser
445 450 455 460

ACT CGT GTC GAG GGC GAC ACC GTG ATT ACC AGC CGG TAC ATG GAT AAC 576
Thr Arg Val Glu Gly Asp Thr Val Ile Thr Ser Arg Tyr Met Asp Asn
465 470 475

GTC ATG GCC CCT CCG TTC TGG CGT GCT GCG CTT CGT GGC AAC GGC TTG 624
Val Met Ala Pro Pro Phe Trp Arg Ala Ala Leu Arg Gly Asn Gly Leu
480 485 490

GCC GAC GAT GTA CCG GTT GAT CGC TGG CAG ATC TGC CGA TTC GCT CCT 672
Ala Asp Asp Val Pro Val Asp Arg Trp Gln Ile Cys Arg Phe Ala Pro
495 500 505

CCG AGT CAC GTA CTG ATC GAA GTA GGT GTG GCT CAT GCG GGC AAA GGC 720
Pro Ser His Val Leu Ile Glu Val Gly Val Ala His Ala Gly Lys Gly
510 515 520

GGA TAT GAC GCG CCG GCG GAA TAC AAG GCC GGC AGC ATA GTG GTC GAC 768
Gly Tyr Asp Ala Pro Ala Glu Tyr Lys Ala Gly Ser Ile Val Val Asp
525 530 535 540

TTC ATC ACG CCG GAG AGT GAT ACC TCG ATT TGG TAC TTC TGG GGC ATG 816
Phe Ile Thr Pro Glu Ser Asp Thr Ser Ile Trp Tyr Phe Trp Gly Met
545 550 555

GCT CGC AAC TTC CGT CCG CAG GGC ACG GAG CTG ACT GAA ACC ATT CGT 864
Ala Arg Asn Phe Arg Pro Gln Gly Thr Glu Leu Thr Glu Thr Ile Arg
560 565 570

GTT GGT CAG GGC AAG ATT TTT GCC GAG GAC CTG GAC ATG CTG GAG CAG 912
Val Gly Gln Gly Lys Ile Phe Ala Glu Asp Leu Asp Met Leu Glu Gln
575 580 585

CAG CAG CGC AAT CTG CTG GCC TAC CCG GAG CGC CAG TTG CTC AAG CTG 960
Gln Gln Arg Asn Leu Leu Ala Tyr Pro Glu Arg Gln Leu Leu Lys Leu
590 595 600

AAT ATC GAT GCC GGC GGG GTT CAG TCA CGG CGC GTC ATT GAT CGG ATT 1008
Asn Ile Asp Ala Gly Gly Val Gln Ser Arg Arg Val Ile Asp Arg Ile
605 610 615 620

CTC GCA GCT GAA CAA GAG GCC GCA GAC GCA GCG CTG ATC GCG AGA AGT 1056
Leu Ala Ala Glu Gln Glu Ala Ala Asp Ala Ala Leu Ile Ala Arg Ser
625 630 635

GCA TCA TGA 1065
Ala Ser

(2) ANGABEN ZU SEQ ID NO: 4:

(i) SEQUENZKENNZEICHEN:
(A) LÄNGE: 354 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Met Phe Pro Lys Asn Ala Trp Tyr Val Ala Cys Thr Pro Asp Glu Ile
1               5                   10                  15

Ala Asp Lys Pro Leu Gly Arg Gln Ile Cys Asn Glu Lys Ile Val Phe
            20                  25                  30

Tyr Arg Gly Pro Glu Gly Arg Val Ala Ala Val Glu Asp Phe Cys Pro
        35                  40                  45

His Arg Gly Ala Pro Leu Ser Leu Gly Phe Val Arg Asp Gly Lys Leu
    50                  55                  60

Ile Cys Gly Tyr His Gly Leu Glu Met Gly Cys Glu Gly Lys Thr Leu
65                  70                  75                  80

Ala Met Pro Gly Gln Arg Val Gln Gly Phe Pro Cys Ile Lys Ser Tyr
                85                  90                  95

Ala Val Glu Glu Arg Tyr Gly Phe Ile Trp Val Trp Pro Gly Asp Arg
            100                 105                 110

Glu Leu Ala Asp Pro Ala Leu Ile His His Leu Glu Trp Ala Asp Asn
        115                 120                 125

Pro Glu Trp Ala Tyr Gly Gly Gly Leu Tyr His Ile Ala Cys Asp Tyr
    130                 135                 140

Arg Leu Met Ile Asp Asn Leu Met Asp Leu Thr His Glu Thr Tyr Val
145                 150                 155                 160

His Ala Ser Ser Ile Gly Gln Lys Glu Ile Asp Glu Ala Pro Val Ser
                165                 170                 175

Thr Arg Val Glu Gly Asp Thr Val Ile Thr Ser Arg Tyr Met Asp Asn
            180                 185                 190

Val Met Ala Pro Pro Phe Trp Arg Ala Ala Leu Arg Gly Asn Gly Leu
        195                 200                 205

Ala Asp Asp Val Pro Val Asp Arg Trp Gln Ile Cys Arg Phe Ala Pro
    210                 215                 220

Pro Ser His Val Leu Ile Glu Val Gly Val Ala His Ala Gly Lys Gly
225                 230                 235                 240

Gly Tyr Asp Ala Pro Ala Glu Tyr Lys Ala Gly Ser Ile Val Val Asp
                245                 250                 255

Phe Ile Thr Pro Glu Ser Asp Thr Ser Ile Trp Tyr Phe Trp Gly Met
            260                 265                 270

Ala Arg Asn Phe Arg Pro Gln Gly Thr Glu Leu Thr Glu Thr Ile Arg
        275                 280                 285

Val Gly Gln Gly Lys Ile Phe Ala Glu Asp Leu Asp Met Leu Glu Gln
    290                 295                 300

Gln Gln Arg Asn Leu Leu Ala Tyr Pro Glu Arg Gln Leu Leu Lys Leu
305                 310                 315                 320

Asn Ile Asp Ala Gly Gly Val Gln Ser Arg Arg Val Ile Asp Arg Ile
                325                 330                 335

Leu Ala Ala Glu Gln Glu Ala Ala Asp Ala Ala Leu Ile Ala Arg Ser
            340                 345                 350

Ala Ser

(2) ANGABEN ZU SEQ ID NO: 5:

(i) SEQUENZKENNZEICHEN:
(A) LÄNGE: 954 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..951
(D) SONSTIGE ANGABEN:/product= "Vanillin-O-Demethylase"
/gene= "vanB"

(xi) SEQUENZBESCHREIBUNG: SEQ ID NO: 5:

ATG ATT GAG GTA ATC ATT TCG GCG ATG CGC TTG GTT GCT CAG GAC ATC 48
Met Ile Glu Val Ile Ile Ser Ala Met Arg Leu Val Ala Gln Asp Ile
355 360 365 370

ATT AGC CTT GAG TTT GTC CGG GCT GAC GGT GGC TTG CTT CCG CCT GTC 96
Ile Ser Leu Glu Phe Val Arg Ala Asp Gly Gly Leu Leu Pro Pro Val
375 380 385

GAG GCC GGC GCC CAC GTC GAT GTG CAT CTT CCT GGC GGC CTG ATT CGG 144
Glu Ala Gly Ala His Val Asp Val His Leu Pro Gly Gly Leu Ile Arg
390 395 400

CAG TAC TCG CTC TGG AAT CAA CCA GGG GCG CAG AGC CAT TAC TGC ATC 192
Gln Tyr Ser Leu Trp Asn Gln Pro Gly Ala Gln Ser His Tyr Cys Ile
405 410 415

GGT GTT CTG AAG GAC CCG GCG TCT CGT GGT GGT TCG AAG GCG GTG CAC 240
Gly Val Leu Lys Asp Pro Ala Ser Arg Gly Gly Ser Lys Ala Val His
420 425 430

GAG AAT CTT CGC GTC GGG ATG CGC GTG CAA ATT AGC GAG CCG AGG AAC 288
Glu Asn Leu Arg Val Gly Met Arg Val Gln Ile Ser Glu Pro Arg Asn
435 440 445 450

CTA TTC CCA TTG GAA GAG GGG GTG GAG CGG AGT CTG CTG TTC GCG GGC 336
Leu Phe Pro Leu Glu Glu Gly Val Glu Arg Ser Leu Leu Phe Ala Gly
455 460 465

GGG ATT GGC ATT ACG CCG ATT CTG TGT ATG GCT CAA GAA TTA GCA GCA 384

Gly Ile Gly Ile Thr Pro Ile Leu Cys Met Ala Gln Glu Leu Ala Ala
470 475 480

CGC GAG CAA GAT TTC GAG TTG CAT TAT TGC GCG CGT TCG ACC GAC CGA 432
Arg Glu Gln Asp Phe Glu Leu His Tyr Cys Ala Arg Ser Thr Asp Arg
485 490 495

GCG GCG TTC GTT GAA TGG CTT AAG GTT TGC GAC TTT GCT GAT CAC GTA 480
Ala Ala Phe Val Glu Trp Leu Lys Val Cys Asp Phe Ala Asp His Val
500 505 510

CGT TTC CAC TTT GAC AAT GGC CCG GAT CAG CAA AAA CTG AAT GCC GCA 528
Arg Phe His Phe Asp Asn Gly Pro Asp Gln Gln Lys Leu Asn Ala Ala
515 520 525 530

GCG CTG CTA GCG GCC GAG GCC GAA GGT ACC CAC CTT TAT GTC TGT GGG 576
Ala Leu Leu Ala Ala Glu Ala Glu Gly Thr His Leu Tyr Val Cys Gly
535 540 545

CCC GGC GGG TTC ATG GGG CAT GTG CTT GAT ACC GCG AAG GAG CAG GGC 624
Pro Gly Gly Phe Met Gly His Val Leu Asp Thr Ala Lys Glu Gln Gly
550 555 560

TGG GCT GAC AAT CGA CTG CAT CGA GAG TAT TTC GCC GCG GCG CCG AAT 672
Trp Ala Asp Asn Arg Leu His Arg Glu Tyr Phe Ala Ala Ala Pro Asn
565 570 575

GTG AGT GCT GAC GAT GGC AGT TTC GAG GTG CGG ATT CAC AGC ACC GGA 720
Val Ser Ala Asp Asp Gly Ser Phe Glu Val Arg Ile His Ser Thr Gly
580 585 590

CAA GTG CTT CAG GTC CCC GCG GAT CAA ACG GTC TCC CAG GTG CTC GAT 768
Gln Val Leu Gln Val Pro Ala Asp Gln Thr Val Ser Gln Val Leu Asp
595 600 605 610

GCG GCC GGA ATT ATC GTT CCC GTT TCT TGT GAG CAG GGC ATC TGC GGT 816
Ala Ala Gly Ile Ile Val Pro Val Ser Cys Glu Gln Gly Ile Cys Gly
615 620 625

ACT TGC ATC ACT CGG GTG GTA GAC GGA GAG CCT GAT CAT CGT GAC TTC 864
Thr Cys Ile Thr Arg Val Val Asp Gly Glu Pro Asp His Arg Asp Phe
630 635 640

TTC CTC ACG GAT GCG GAG AAG GCA AAG AAC GAC CAG TTC ACC CCC TGT 912
Phe Leu Thr Asp Ala Glu Lys Ala Lys Asn Asp Gln Phe Thr Pro Cys
645 650 655

TGC TCG CGA GCC AAG AGC GCC TGT TTG GTC TTG GAT CTC TAA 954
Cys Ser Arg Ala Lys Ser Ala Cys Leu Val Leu Asp Leu
660 665 670

(2) ANGABEN ZU SEQ ID NO: 6:

(i) SEQUENZKENNZEICHEN:

(A) LÄNGE: 317 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Met Ile Glu Val Ile Ile Ser Ala Met Arg Leu Val Ala Gln Asp Ile
1               5                   10                  15

Ile Ser Leu Glu Phe Val Arg Ala Asp Gly Gly Leu Leu Pro Pro Val
            20                  25                  30

Glu Ala Gly Ala His Val Asp Val His Leu Pro Gly Gly Leu Ile Arg
        35                  40                  45

Gln Tyr Ser Leu Trp Asn Gln Pro Gly Ala Gln Ser His Tyr Cys Ile
    50                  55                  60

Gly Val Leu Lys Asp Pro Ala Ser Arg Gly Gly Ser Lys Ala Val His
65                  70                  75                  80

Glu Asn Leu Arg Val Gly Met Arg Val Gln Ile Ser Glu Pro Arg Asn
                85                  90                  95

Leu Phe Pro Leu Glu Glu Gly Val Glu Arg Ser Leu Leu Phe Ala Gly
            100                 105                 110

Gly Ile Gly Ile Thr Pro Ile Leu Cys Met Ala Gln Glu Leu Ala Ala
        115                 120                 125

Arg Glu Gln Asp Phe Glu Leu His Tyr Cys Ala Arg Ser Thr Asp Arg
    130                 135                 140

Ala Ala Phe Val Glu Trp Leu Lys Val Cys Asp Phe Ala Asp His Val
145                 150                 155                 160

Arg Phe His Phe Asp Asn Gly Pro Asp Gln Gln Lys Leu Asn Ala Ala
                165                 170                 175

Ala Leu Leu Ala Ala Glu Ala Glu Gly Thr His Leu Tyr Val Cys Gly
            180                 185                 190

Pro Gly Gly Phe Met Gly His Val Leu Asp Thr Ala Lys Glu Gln Gly
        195                 200                 205

Trp Ala Asp Asn Arg Leu His Arg Glu Tyr Phe Ala Ala Ala Pro Asn
    210                 215                 220

Val Ser Ala Asp Asp Gly Ser Phe Glu Val Arg Ile His Ser Thr Gly
225                 230                 235                 240

Gln Val Leu Gln Val Pro Ala Asp Gln Thr Val Ser Gln Val Leu Asp
                245                 250                 255

Ala Ala Gly Ile Ile Val Pro Val Ser Cys Glu Gln Gly Ile Cys Gly
            260                 265                 270

Thr Cys Ile Thr Arg Val Val Asp Gly Glu Pro Asp His Arg Asp Phe
        275                 280                 285

Phe Leu Thr Asp Ala Glu Lys Ala Lys Asn Asp Gln Phe Thr Pro Cys
    290                 295                 300

Cys Ser Arg Ala Lys Ser Ala Cys Leu Val Leu Asp Leu
305                 310                 315

(2) ANGABEN ZU SEQ ID NO: 7:

(i) SEQUENZKENNZEICHEN:
(A) LÄNGE: 1119 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1116
(D) SONSTIGE ANGABEN:/product=
"Formaldehyd-Dehydrogenase"
/gene= "fdh"

(xi) SEQUENZBESCHREIBUNG: SEQ ID NO: 7:

ATG ATC AAA TCC CGC GCC GCT GTG GCG TTC GCA CCC AAT CAG CCA TTG      48
Met Ile Lys Ser Arg Ala Ala Val Ala Phe Ala Pro Asn Gln Pro Leu
        320                 325                 330

CAG ATC GTC GAA GTG GAC GTG GCT CCG CCC AAG GCC GGT GAA GTC CTG      96
Gln Ile Val Glu Val Asp Val Ala Pro Pro Lys Ala Gly Glu Val Leu
    335                 340                 345

GTG CGG GTC GTG GCC ACC GGC GTT TGC CAC ACC GAT GCC TAC ACC CTG     144
Val Arg Val Val Ala Thr Gly Val Cys His Thr Asp Ala Tyr Thr Leu
350                 355                 360                 365

TCC GGC GCT GAT TCC GAG GGC GTT TTC CCC TGC ATC CTT GGT CAC GAA     192
Ser Gly Ala Asp Ser Glu Gly Val Phe Pro Cys Ile Leu Gly His Glu
                370                 375                 380

GGC GGC GGC ATT GTC GAA GCG GTG GGC GAG GGC GTC ACC TCG CTG GCG     240
Gly Gly Gly Ile Val Glu Ala Val Gly Glu Gly Val Thr Ser Leu Ala

385 390 395

GTC GGC GAC CAC GTG ATC CCG CTC TAC ACG GCC GAA TGC CGT GAG TGC 288
Val Gly Asp His Val Ile Pro Leu Tyr Thr Ala Glu Cys Arg Glu Cys
400 405 410

AAG TTC TTC AAG TCC GGC AAG ACC AAC CTG TGC CAG AAA GTG CGT GCT 336
Lys Phe Phe Lys Ser Gly Lys Thr Asn Leu Cys Gln Lys Val Arg Ala
415 420 425

ACT CAG GGC AAG GGT CTG ATG CCG GAC GGC ACC TCC CGC TTC AGC TAC 384
Thr Gln Gly Lys Gly Leu Met Pro Asp Gly Thr Ser Arg Phe Ser Tyr
430 435 440 445

AAC GGT CAG CCG ATC TAC CAC TAC ATG GGC TGC TCG ACC TTC TCC GAG 432
Asn Gly Gln Pro Ile Tyr His Tyr Met Gly Cys Ser Thr Phe Ser Glu
450 455 460

TAC ACC GTG CTG CCG GAA ATC TCC CTG GCG AAG ATT CCC AAG AAT GCG 480
Tyr Thr Val Leu Pro Glu Ile Ser Leu Ala Lys Ile Pro Lys Asn Ala
465 470 475

CCG CTG GAG AAA GTC TGC CTG CTG GGC TGC GGC GTG ACC ACC GGC ATT 528
Pro Leu Glu Lys Val Cys Leu Leu Gly Cys Gly Val Thr Thr Gly Ile
480 485 490

GGC GCG GTG CTG AAC ACT GCC AAG GTG GAG GAG GGT GCT ACC GTG GCC 576
Gly Ala Val Leu Asn Thr Ala Lys Val Glu Glu Gly Ala Thr Val Ala
495 500 505

ATC TTC GGC CTG GGC GGC ATC GGC TTG GCG GCG ATC ATC GGC GCG AAG 624
Ile Phe Gly Leu Gly Gly Ile Gly Leu Ala Ala Ile Ile Gly Ala Lys
510 515 520 525

ATG GCC AAG GCC TCG CGC ATC ATC GCC ATC GAC ATC AAT CCG TCC AAG 672
Met Ala Lys Ala Ser Arg Ile Ile Ala Ile Asp Ile Asn Pro Ser Lys
530 535 540

TTC GAT GTG GCT CGC GAG CTG GGC GCC ACT GAC TTC GTC AAT CCG AAC 720
Phe Asp Val Ala Arg Glu Leu Gly Ala Thr Asp Phe Val Asn Pro Asn
545 550 555

GAT CAC GCG AAG CCG ATC CAG GAT GTC ATC GTC GAG ATG ACT GAT GGC 768
Asp His Ala Lys Pro Ile Gln Asp Val Ile Val Glu Met Thr Asp Gly
560 565 570

GGT GTG GAC TAC AGC TTC GAG TGC ATC GGC AAC GTT CGA CTC ATG CGC 816
Gly Val Asp Tyr Ser Phe Glu Cys Ile Gly Asn Val Arg Leu Met Arg
575 580 585

GCA GCA CTC GAG TGC TGC CAC AAG GGC TGG GGC GAA TCC GTG ATC ATC 864
Ala Ala Leu Glu Cys Cys His Lys Gly Trp Gly Glu Ser Val Ile Ile
590 595 600 605

GGC GTG GCG CCG GCG GGG GCC GAA ATC AAC ACC CGT CCG TTC CAC CTG 912

Gly Val Ala Pro Ala Gly Ala Glu Ile Asn Thr Arg Pro Phe His Leu
610 615 620

GTG ACC GGT CGC GTC TGG CGG GGT TCG GCG TTC GGT GGC GTA AAG GGC 960
Val Thr Gly Arg Val Trp Arg Gly Ser Ala Phe Gly Gly Val Lys Gly
625 630 635

CGC ACC GAA CTG CCG AGC TAC GTG GAG AAG GCA CAG CAG GGC GAG ATC 1008
Arg Thr Glu Leu Pro Ser Tyr Val Glu Lys Ala Gln Gln Gly Glu Ile
640 645 650

CCG CTG GAC ACC TTC ATC ACT CAC ACC ATG GGC CTG GAC GAC ATC AAC 1056
Pro Leu Asp Thr Phe Ile Thr His Thr Met Gly Leu Asp Asp Ile Asn
655 660 665

ACG GCC TTC GAC CTG ATG GAC GAA GGG AAG AGC ATC CGC TCT GTT GTT 1104
Thr Ala Phe Asp Leu Met Asp Glu Gly Lys Ser Ile Arg Ser Val Val
670 675 680 685

CAA TTG AGT CGC TAG 1119
Gln Leu Ser Arg

(2) ANGABEN ZU SEQ ID NO: 8:

(i) SEQUENZKENNZEICHEN:
(A) LÄNGE: 372 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 8:

Met Ile Lys Ser Arg Ala Ala Val Ala Phe Ala Pro Asn Gln Pro Leu
1               5                   10                  15

Gln Ile Val Glu Val Asp Val Ala Pro Pro Lys Ala Gly Glu Val Leu
            20                  25                  30

Val Arg Val Val Ala Thr Gly Val Cys His Thr Asp Ala Tyr Thr Leu
        35                  40                  45

Ser Gly Ala Asp Ser Glu Gly Val Phe Pro Cys Ile Leu Gly His Glu
    50                  55                  60

Gly Gly Gly Ile Val Glu Ala Val Gly Glu Gly Val Thr Ser Leu Ala
65                  70                  75                  80

Val Gly Asp His Val Ile Pro Leu Tyr Thr Ala Glu Cys Arg Glu Cys
                85                  90                  95

Lys Phe Phe Lys Ser Gly Lys Thr Asn Leu Cys Gln Lys Val Arg Ala
            100                 105                 110

Thr Gln Gly Lys Gly Leu Met Pro Asp Gly Thr Ser Arg Phe Ser Tyr
        115                 120                 125

Asn Gly Gln Pro Ile Tyr His Tyr Met Gly Cys Ser Thr Phe Ser Glu
    130                 135                 140

Tyr Thr Val Leu Pro Glu Ile Ser Leu Ala Lys Ile Pro Lys Asn Ala
145                 150                 155                 160

Pro Leu Glu Lys Val Cys Leu Leu Gly Cys Gly Val Thr Thr Gly Ile
                165                 170                 175

Gly Ala Val Leu Asn Thr Ala Lys Val Glu Glu Gly Ala Thr Val Ala
            180                 185                 190

Ile Phe Gly Leu Gly Gly Ile Gly Leu Ala Ala Ile Ile Gly Ala Lys
        195                 200                 205

Met Ala Lys Ala Ser Arg Ile Ile Ala Ile Asp Ile Asn Pro Ser Lys
    210                 215                 220

Phe Asp Val Ala Arg Glu Leu Gly Ala Thr Asp Phe Val Asn Pro Asn
225                 230                 235                 240

Asp His Ala Lys Pro Ile Gln Asp Val Ile Val Glu Met Thr Asp Gly
                245                 250                 255

Gly Val Asp Tyr Ser Phe Glu Cys Ile Gly Asn Val Arg Leu Met Arg
            260                 265                 270

Ala Ala Leu Glu Cys Cys His Lys Gly Trp Gly Glu Ser Val Ile Ile
        275                 280                 285

Gly Val Ala Pro Ala Gly Ala Glu Ile Asn Thr Arg Pro Phe His Leu
    290                 295                 300

Val Thr Gly Arg Val Trp Arg Gly Ser Ala Phe Gly Gly Val Lys Gly
305                 310                 315                 320

Arg Thr Glu Leu Pro Ser Tyr Val Glu Lys Ala Gln Gln Gly Glu Ile
                325                 330                 335

Pro Leu Asp Thr Phe Ile Thr His Thr Met Gly Leu Asp Asp Ile Asn
            340                 345                 350

Thr Ala Phe Asp Leu Met Asp Glu Gly Lys Ser Ile Arg Ser Val Val
        355                 360                 365

Gln Leu Ser Arg
    370

(2) ANGABEN ZU SEQ ID NO: 9:

    (i) SEQUENZKENNZEICHEN:
        (A) LÄNGE: 1638 Basenpaare

(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1635
(D) SONSTIGE ANGABEN:/product=
"gamma-Glutamylcystein-Synthetase"
/gene= "gcs"

(xi) SEQUENZBESCHREIBUNG: SEQ ID NO: 9:

ATG CCG CAA ACT CTT GCT GGA CGG TTG AGT CTG TTA TCC GGC ACC GAC 48
Met Pro Gln Thr Leu Ala Gly Arg Leu Ser Leu Leu Ser Gly Thr Asp
375 380 385

GAA TTA ACC CTG CTT CTT CGG GGT GGT CGG GGC ATT GAG CGT GAA GCC 96
Glu Leu Thr Leu Leu Leu Arg Gly Gly Arg Gly Ile Glu Arg Glu Ala
390 395 400

TTG CGG GTC GAT GTT CAA GGT GAA CTG GCG CTG ACG CCT CAC CCG GCG 144
Leu Arg Val Asp Val Gln Gly Glu Leu Ala Leu Thr Pro His Pro Ala
405 410 415 420

GCG CTT GGC TCT GCG TTG ACC CAT CCG ACA ATT ACT ACG GAT TAC GCC 192
Ala Leu Gly Ser Ala Leu Thr His Pro Thr Ile Thr Thr Asp Tyr Ala
425 430 435

GAG GCC CTG CTT GAG TTG ATC ACT CGG CCG GCA ACC GAT TGT GCG CAA 240
Glu Ala Leu Leu Glu Leu Ile Thr Arg Pro Ala Thr Asp Cys Ala Gln
440 445 450

GCC TTG GCT GAG CTG GAG GAG CTT CAC CGT TTC GTT CAT TCG AGA CTT 288
Ala Leu Ala Glu Leu Glu Glu Leu His Arg Phe Val His Ser Arg Leu
455 460 465

GAG GGG GAG TAT CTC TGG AAT CTG TCC ATG CCT GGC AGA TTG CCG GTT 336
Glu Gly Glu Tyr Leu Trp Asn Leu Ser Met Pro Gly Arg Leu Pro Val
470 475 480

GAT GAG CAA ATC CCG ATT GCT TGG TAT GGA CCA TCA AAT CCA GGC ATG 384
Asp Glu Gln Ile Pro Ile Ala Trp Tyr Gly Pro Ser Asn Pro Gly Met
485 490 495 500

TTG CGC CAC GTT TAT CGC CGT GGC CTA GCT CTG CGT TAT GGC AAG CGA 432
Leu Arg His Val Tyr Arg Arg Gly Leu Ala Leu Arg Tyr Gly Lys Arg

505 510 515

ATG CAA TGC ATC GCA GGG ATT CAC TAC AAC TAC TCA CTG CCG CCA GAG 480
Met Gln Cys Ile Ala Gly Ile His Tyr Asn Tyr Ser Leu Pro Pro Glu
520 525 530

CTT TTC GCT GTC CTG ACC AAG GCA GAG GTC GGG TCT CCC AAG TTA CTG 528
Leu Phe Ala Val Leu Thr Lys Ala Glu Val Gly Ser Pro Lys Leu Leu
535 540 545

GAG CGC CAG TCA GCA GCT TAC ATG CGC CAA ATT CGC AAC CTT CGG CAA 576
Glu Arg Gln Ser Ala Ala Tyr Met Arg Gln Ile Arg Asn Leu Arg Gln
550 555 560

TAC GGT TGG TTG CTG GCC TAC TTG TTC GGC GCT TCC CCC GCC ATC TGC 624
Tyr Gly Trp Leu Leu Ala Tyr Leu Phe Gly Ala Ser Pro Ala Ile Cys
565 570 575 580

AAG AGC TTC TTG GGG GGC GAG AGA GAT GAG CTA GCT CGC ATG GGG GGC 672
Lys Ser Phe Leu Gly Gly Glu Arg Asp Glu Leu Ala Arg Met Gly Gly
585 590 595

GAT ACG CTT TAC ATG CCC TAT GCA ACC AGC TTG CGC ATG AGT GAC ATC 720
Asp Thr Leu Tyr Met Pro Tyr Ala Thr Ser Leu Arg Met Ser Asp Ile
600 605 610

GGG TAC CGC AAC CGT GCC ATG GAT GAT CTA TCT CCC AGC CTG AAT GAT 768
Gly Tyr Arg Asn Arg Ala Met Asp Asp Leu Ser Pro Ser Leu Asn Asp
615 620 625

CTG GGT GCC TAT ATT CGC GAT ATT TGC CGT GCT CTT CAC ACT CCC GAT 816
Leu Gly Ala Tyr Ile Arg Asp Ile Cys Arg Ala Leu His Thr Pro Asp
630 635 640

GCC CAG TAC CAG GCG CTG GGT GTG TTT GCA CAG GGC GAG TGG CGG CAG 864
Ala Gln Tyr Gln Ala Leu Gly Val Phe Ala Gln Gly Glu Trp Arg Gln
645 650 655 660

TTA AAC GCC AAT CTA TTG CAG TTG GAT AGT GAG TAC TAC GCA CTG GCG 912
Leu Asn Ala Asn Leu Leu Gln Leu Asp Ser Glu Tyr Tyr Ala Leu Ala
665 670 675

CGA CCG AAG TCA GCG CCC GAG CGG GGG GAG CGA AAC CTG GAT GCT CTC 960
Arg Pro Lys Ser Ala Pro Glu Arg Gly Glu Arg Asn Leu Asp Ala Leu
680 685 690

GCT AGG CGT GGA GTC CAG TAT GTG GAG CTG CGC GCA CTG GAT CTC GAT 1008
Ala Arg Arg Gly Val Gln Tyr Val Glu Leu Arg Ala Leu Asp Leu Asp
695 700 705

CCA TTC TCC CCG TTA GGC ATT GGC CTG ACC TGC GCC AAG TTC CTC GAT 1056
Pro Phe Ser Pro Leu Gly Ile Gly Leu Thr Cys Ala Lys Phe Leu Asp
710 715 720

GGC TTT TTG CTT TTC TGC TTG TTG TCT GAG GCG CCG GTT GAT GAT CGA 1104

Gly Phe Leu Leu Phe Cys Leu Leu Ser Glu Ala Pro Val Asp Asp Arg
725                 730                 735                 740

AAT GCC CAG CGT TCA AGA CCG GGA AAA TCT GAG CCT GGC CGG CAA GTA      1152
Asn Ala Gln Arg Ser Arg Pro Gly Lys Ser Glu Pro Gly Arg Gln Val
                745                 750                 755

CGG GCG TCA CCT GGC TTA AAG CTG CAT CGG AAT GGT CAG TCC ATT CTC      1200
Arg Ala Ser Pro Gly Leu Lys Leu His Arg Asn Gly Gln Ser Ile Leu
            760                 765                 770

CTC AAG GAT TGG GCG CAG GAA GTG TTG ACG GAG GTT CAG GCC TGT GTG      1248
Leu Lys Asp Trp Ala Gln Glu Val Leu Thr Glu Val Gln Ala Cys Val
        775                 780                 785

GAA TTG CTC GAC AGT GCA AAT GGG GGC TCA TCT CAC GCA TTG GCT TGG      1296
Glu Leu Leu Asp Ser Ala Asn Gly Gly Ser Ser His Ala Leu Ala Trp
    790                 795                 800

TCA GCA CAG GAG GAA AAG GTG CTT AAT CCG GAT TGT GCG CCA TCA GCT      1344
Ser Ala Gln Glu Glu Lys Val Leu Asn Pro Asp Cys Ala Pro Ser Ala
805                 810                 815                 820

CAG GTG CTC GCA GAG ATA CAC AGA CAC GGT GGG AGC TTC ACG GCA TTT      1392
Gln Val Leu Ala Glu Ile His Arg His Gly Gly Ser Phe Thr Ala Phe
                825                 830                 835

GGT CGC CAA TTA GCT ATC GAC CAT GCA AAA CAC TTC AGT GCC TCC TCG      1440
Gly Arg Gln Leu Ala Ile Asp His Ala Lys His Phe Ser Ala Ser Ser
            840                 845                 850

CTT GAG GCT GGC GTA GCC AAA GCG CTT GAC CTC CAG GCG ACG TCG TCT      1488
Leu Glu Ala Gly Val Ala Lys Ala Leu Asp Leu Gln Ala Thr Ser Ser
        855                 860                 865

CTG CGC GAG CAG CAT CAA TTG GAG GCC AAC GAC CGT GCG CCA TTT TCT      1536
Leu Arg Glu Gln His Gln Leu Glu Ala Asn Asp Arg Ala Pro Phe Ser
    870                 875                 880

GAC TAC CTT CAG CAA TTC TCC CTG GCT TTC GGT CAA TCC GTC GGC GCC      1584
Asp Tyr Leu Gln Gln Phe Ser Leu Ala Phe Gly Gln Ser Val Gly Ala
885                 890                 895                 900

TCT CGT GCG CCC AAC CCT ACC GCG CAC CTC ATC GAT CTG ACC CCT CCT      1632
Ser Arg Ala Pro Asn Pro Thr Ala His Leu Ile Asp Leu Thr Pro Pro
                905                 910                 915

GTC TAA                                                              1638
Val

(2) ANGABEN ZU SEQ ID NO: 10:

    (i) SEQUENZKENNZEICHEN:

(A) LÄNGE: 545 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 10:

Met Pro Gln Thr Leu Ala Gly Arg Leu Ser Leu Leu Ser Gly Thr Asp
1               5                   10                  15

Glu Leu Thr Leu Leu Leu Arg Gly Gly Arg Gly Ile Glu Arg Glu Ala
            20                  25                  30

Leu Arg Val Asp Val Gln Gly Glu Leu Ala Leu Thr Pro His Pro Ala
        35                  40                  45

Ala Leu Gly Ser Ala Leu Thr His Pro Thr Ile Thr Thr Asp Tyr Ala
    50                  55                  60

Glu Ala Leu Leu Glu Leu Ile Thr Arg Pro Ala Thr Asp Cys Ala Gln
65                  70                  75                  80

Ala Leu Ala Glu Leu Glu Glu Leu His Arg Phe Val His Ser Arg Leu
                85                  90                  95

Glu Gly Glu Tyr Leu Trp Asn Leu Ser Met Pro Gly Arg Leu Pro Val
            100                 105                 110

Asp Glu Gln Ile Pro Ile Ala Trp Tyr Gly Pro Ser Asn Pro Gly Met
        115                 120                 125

Leu Arg His Val Tyr Arg Arg Gly Leu Ala Leu Arg Tyr Gly Lys Arg
    130                 135                 140

Met Gln Cys Ile Ala Gly Ile His Tyr Asn Tyr Ser Leu Pro Pro Glu
145                 150                 155                 160

Leu Phe Ala Val Leu Thr Lys Ala Glu Val Gly Ser Pro Lys Leu Leu
                165                 170                 175

Glu Arg Gln Ser Ala Ala Tyr Met Arg Gln Ile Arg Asn Leu Arg Gln
            180                 185                 190

Tyr Gly Trp Leu Leu Ala Tyr Leu Phe Gly Ala Ser Pro Ala Ile Cys
        195                 200                 205

Lys Ser Phe Leu Gly Gly Glu Arg Asp Glu Leu Ala Arg Met Gly Gly
    210                 215                 220

Asp Thr Leu Tyr Met Pro Tyr Ala Thr Ser Leu Arg Met Ser Asp Ile
225                 230                 235                 240

Gly Tyr Arg Asn Arg Ala Met Asp Asp Leu Ser Pro Ser Leu Asn Asp
                245                 250                 255

Leu Gly Ala Tyr Ile Arg Asp Ile Cys Arg Ala Leu His Thr Pro Asp
            260                 265                 270

Ala Gln Tyr Gln Ala Leu Gly Val Phe Ala Gln Gly Glu Trp Arg Gln
        275                 280                 285

Leu Asn Ala Asn Leu Leu Gln Leu Asp Ser Glu Tyr Tyr Ala Leu Ala
    290                 295                 300

Arg Pro Lys Ser Ala Pro Glu Arg Gly Glu Arg Asn Leu Asp Ala Leu
305                 310                 315                 320

Ala Arg Arg Gly Val Gln Tyr Val Glu Leu Arg Ala Leu Asp Leu Asp
                325                 330                 335

Pro Phe Ser Pro Leu Gly Ile Gly Leu Thr Cys Ala Lys Phe Leu Asp
            340                 345                 350

Gly Phe Leu Leu Phe Cys Leu Leu Ser Glu Ala Pro Val Asp Asp Arg
        355                 360                 365

Asn Ala Gln Arg Ser Arg Pro Gly Lys Ser Glu Pro Gly Arg Gln Val
    370                 375                 380

Arg Ala Ser Pro Gly Leu Lys Leu His Arg Asn Gly Gln Ser Ile Leu
385                 390                 395                 400

Leu Lys Asp Trp Ala Gln Glu Val Leu Thr Glu Val Gln Ala Cys Val
                405                 410                 415

Glu Leu Leu Asp Ser Ala Asn Gly Gly Ser Ser His Ala Leu Ala Trp
            420                 425                 430

Ser Ala Gln Glu Glu Lys Val Leu Asn Pro Asp Cys Ala Pro Ser Ala
        435                 440                 445

Gln Val Leu Ala Glu Ile His Arg His Gly Gly Ser Phe Thr Ala Phe
    450                 455                 460

Gly Arg Gln Leu Ala Ile Asp His Ala Lys His Phe Ser Ala Ser Ser
465                 470                 475                 480

Leu Glu Ala Gly Val Ala Lys Ala Leu Asp Leu Gln Ala Thr Ser Ser
                485                 490                 495

Leu Arg Glu Gln His Gln Leu Glu Ala Asn Asp Arg Ala Pro Phe Ser
            500                 505                 510

Asp Tyr Leu Gln Gln Phe Ser Leu Ala Phe Gly Gln Ser Val Gly Ala
        515                 520                 525

Ser Arg Ala Pro Asn Pro Thr Ala His Leu Ile Asp Leu Thr Pro Pro
    530                 535                 540

Val

545

(2) ANGABEN ZU SEQ ID NO: 11:

(i) SEQUENZKENNZEICHEN:
(A) LÄNGE: 354 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..351
(D) SONSTIGE ANGABEN:/product= "Cytochrom C
UE-Eugenol-Hydroxylase"
/gene= "ehyA"

(xi) SEQUENZBESCHREIBUNG: SEQ ID NO: 11:

ATG ATG AAT GTT AAT TAT AAG GCT GTC GGG GCG AGC CTA CTC CTC GCC 48
Met Met Asn Val Asn Tyr Lys Ala Val Gly Ala Ser Leu Leu Leu Ala
550 555 560

TTC ATC TCT CAG GGA GCT TGG GCA GAG AGC CCC GCA GCC TCT GGC AAT 96
Phe Ile Ser Gln Gly Ala Trp Ala Glu Ser Pro Ala Ala Ser Gly Asn
565 570 575

ACC CCT GAC ATT TAT CGA AAG ACC TGC ACC TAC TGC CAT GAG CCT ACT 144
Thr Pro Asp Ile Tyr Arg Lys Thr Cys Thr Tyr Cys His Glu Pro Thr
580 585 590

GTC AAC AAT GGC CGG GTC ATT GCC CGA AGC CTC GGG CCG ACT CTG CGA 192
Val Asn Asn Gly Arg Val Ile Ala Arg Ser Leu Gly Pro Thr Leu Arg
595 600 605

GGG CGC CAG ATC CCT CCA CAG TAC ACG GAG TAC ATG GTG CGT CAT GGA 240
Gly Arg Gln Ile Pro Pro Gln Tyr Thr Glu Tyr Met Val Arg His Gly
610 615 620 625

CGC GGG GCA ATG CCT GCA TTC TCT GAA GCA GAA GTG CCT CCG GCG GAG 288
Arg Gly Ala Met Pro Ala Phe Ser Glu Ala Glu Val Pro Pro Ala Glu
630 635 640

CTG AAA GTT CTG GGC GAT TGG ATT CAG CAA AGC AGT GCT CCC AAA GAC 336
Leu Lys Val Leu Gly Asp Trp Ile Gln Gln Ser Ser Ala Pro Lys Asp
645 650 655

GCT GGA GTC GCG CCA TGA                                                    354
Ala Gly Val Ala Pro
        660

(2) ANGABEN ZU SEQ ID NO: 12:

(i) SEQUENZKENNZEICHEN:
(A) LÄNGE: 117 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 12:

Met Met Asn Val Asn Tyr Lys Ala Val Gly Ala Ser Leu Leu Leu Ala
  1               5                  10                  15

Phe Ile Ser Gln Gly Ala Trp Ala Glu Ser Pro Ala Ala Ser Gly Asn
             20                  25                  30

Thr Pro Asp Ile Tyr Arg Lys Thr Cys Thr Tyr Cys His Glu Pro Thr
         35                  40                  45

Val Asn Asn Gly Arg Val Ile Ala Arg Ser Leu Gly Pro Thr Leu Arg
     50                  55                  60

Gly Arg Gln Ile Pro Pro Gln Tyr Thr Glu Tyr Met Val Arg His Gly
 65                  70                  75                  80

Arg Gly Ala Met Pro Ala Phe Ser Glu Ala Glu Val Pro Pro Ala Glu
                 85                  90                  95

Leu Lys Val Leu Gly Asp Trp Ile Gln Gln Ser Ser Ala Pro Lys Asp
            100                 105                 110

Ala Gly Val Ala Pro
        115

(2) ANGABEN ZU SEQ ID NO: 13:

(i) SEQUENZKENNZEICHEN:
(A) LÄNGE: 687 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:

(A) NAME/SCHLÜSSEL: CDS
(B) LAGE:1..684
(D) SONSTIGE ANGABEN:/gene= "ORF5"

(xi) SEQUENZBESCHREIBUNG: SEQ ID NO: 13:

ATG ACT ACC CGT CGC AAC TTT CTA ATA GGC GCG TCG CAG GTG GGG GCA 48
Met Thr Thr Arg Arg Asn Phe Leu Ile Gly Ala Ser Gln Val Gly Ala
120 125 130

TTG GTG ATG ATG TCG CCG AAA TTG GTC TTC CGT ACG CCG CTC AAG CAG 96
Leu Val Met Met Ser Pro Lys Leu Val Phe Arg Thr Pro Leu Lys Gln
135 140 145

AAG CCC GTG CGC ATC CTG TCG ACC GGG CTG GCC GGT GAG CAA GAG TTT 144
Lys Pro Val Arg Ile Leu Ser Thr Gly Leu Ala Gly Glu Gln Glu Phe
150 155 160 165

CAC TCG ATG CTT CGC GCG CGA TTG ACC CAT ACG GGT CAG GTC GAC ATC 192
His Ser Met Leu Arg Ala Arg Leu Thr His Thr Gly Gln Val Asp Ile
170 175 180

GCG TCG GTA CCG CTG GAC GCA GCT ATT TGG GCT TCT CCC GCT CGA CTT 240
Ala Ser Val Pro Leu Asp Ala Ala Ile Trp Ala Ser Pro Ala Arg Leu
185 190 195

GCC CAG GCA ATG GAT GCG TTG AAT GGT ACG CGT CTG ATC GCT TTT GTT 288
Ala Gln Ala Met Asp Ala Leu Asn Gly Thr Arg Leu Ile Ala Phe Val
200 205 210

GAG CCC AGG AAC GAA TTG ATA CTG ATG CAA TTC TTG ATG GAT CGC GGG 336
Glu Pro Arg Asn Glu Leu Ile Leu Met Gln Phe Leu Met Asp Arg Gly
215 220 225

GCT GCG GTG CTT ATT CAA GGT GAG CAT GCG GTG GAC AGC AAG GGG GTC 384
Ala Ala Val Leu Ile Gln Gly Glu His Ala Val Asp Ser Lys Gly Val
230 235 240 245

TCT CGG CAC GAC TTT CTG AGT ACC CCA TCC AGT GCG GGA ATT GGA GGG 432
Ser Arg His Asp Phe Leu Ser Thr Pro Ser Ser Ala Gly Ile Gly Gly
250 255 260

GCG CTA GCC GAC AGC CTG GCA AAA GGG GGC TCG CCG TTC TCT ATT TCC 480
Ala Leu Ala Asp Ser Leu Ala Lys Gly Gly Ser Pro Phe Ser Ile Ser
265 270 275

GTC CGA GCG CTT GGC TCG GTA ACT GCT CAG CCA AGA AGT AAT CAG AGT 528
Val Arg Ala Leu Gly Ser Val Thr Ala Gln Pro Arg Ser Asn Gln Ser
280 285 290

GAG GTG GCC ACC CAC TGG ACG ACC GCT CTG GGG ACC TAT TAT GCC GAT 576
Glu Val Ala Thr His Trp Thr Thr Ala Leu Gly Thr Tyr Tyr Ala Asp
295 300 305

ATC GCA GTG GGG CGC TGG GAG CCG CAG CGC GAA GTG GCC AGC TAT GGA 624
Ile Ala Val Gly Arg Trp Glu Pro Gln Arg Glu Val Ala Ser Tyr Gly
310 315 320 325

AGT GGA CTA ATC ATG GCG GAA CGG CTT GAT CGT GTT GCC TCA ACC TTC 672
Ser Gly Leu Ile Met Ala Glu Arg Leu Asp Arg Val Ala Ser Thr Phe
330 335 340

ATT GCA GAT CTC TGA 687
Ile Ala Asp Leu
345

(2) ANGABEN ZU SEQ ID NO: 14:

(i) SEQUENZKENNZEICHEN:
(A) LÄNGE: 228 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 14:

Met Thr Thr Arg Arg Asn Phe Leu Ile Gly Ala Ser Gln Val Gly Ala
1               5                   10                  15

Leu Val Met Met Ser Pro Lys Leu Val Phe Arg Thr Pro Leu Lys Gln
            20                  25                  30

Lys Pro Val Arg Ile Leu Ser Thr Gly Leu Ala Gly Glu Gln Glu Phe
        35                  40                  45

His Ser Met Leu Arg Ala Arg Leu Thr His Thr Gly Gln Val Asp Ile
    50                  55                  60

Ala Ser Val Pro Leu Asp Ala Ala Ile Trp Ala Ser Pro Ala Arg Leu
65                  70                  75                  80

Ala Gln Ala Met Asp Ala Leu Asn Gly Thr Arg Leu Ile Ala Phe Val
                85                  90                  95

Glu Pro Arg Asn Glu Leu Ile Leu Met Gln Phe Leu Met Asp Arg Gly
            100                 105                 110

Ala Ala Val Leu Ile Gln Gly Glu His Ala Val Asp Ser Lys Gly Val
        115                 120                 125

Ser Arg His Asp Phe Leu Ser Thr Pro Ser Ser Ala Gly Ile Gly Gly
    130                 135                 140

Ala Leu Ala Asp Ser Leu Ala Lys Gly Gly Ser Pro Phe Ser Ile Ser
145                 150                 155                 160

Val Arg Ala Leu Gly Ser Val Thr Ala Gln Pro Arg Ser Asn Gln Ser
                165                 170                 175

Glu Val Ala Thr His Trp Thr Thr Ala Leu Gly Thr Tyr Tyr Ala Asp
            180                 185                 190

Ile Ala Val Gly Arg Trp Glu Pro Gln Arg Glu Val Ala Ser Tyr Gly
        195                 200                 205

Ser Gly Leu Ile Met Ala Glu Arg Leu Asp Arg Val Ala Ser Thr Phe
    210                 215                 220

Ile Ala Asp Leu
225

(2) ANGABEN ZU SEQ ID NO: 15:

(i) SEQUENZKENNZEICHEN:
(A) LÄNGE: 1554 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1551
(D) SONSTIGE ANGABEN:/product= "Flavoprotein
UE-Eugenol-Hydroxylase"
/gene= "ehyB"

(xi) SEQUENZBESCHREIBUNG: SEQ ID NO: 15:

ATG GAA AGC ACC GTA GTT CTT CCC GAG GGT GTC ACC CCG GAG CAG TTC 48
Met Glu Ser Thr Val Val Leu Pro Glu Gly Val Thr Pro Glu Gln Phe
230 235 240

ACC AAA GCC ATC AGC GAG TTC CGT CAG GTA TTG GGT GAG GAC AGT GTT 96
Thr Lys Ala Ile Ser Glu Phe Arg Gln Val Leu Gly Glu Asp Ser Val
245 250 255 260

CTT GTC ACT GCT GAA CGA GTT GTT CCC TAT ACG AAA CTC CTC ATT CCT 144
Leu Val Thr Ala Glu Arg Val Val Pro Tyr Thr Lys Leu Leu Ile Pro
265 270 275

ACA CAG GAT GAT GCC CAG TAC ACC CCG GCC GGT GCC TTG ACT CCT TCT 192
Thr Gln Asp Asp Ala Gln Tyr Thr Pro Ala Gly Ala Leu Thr Pro Ser
280 285 290

TCG GTG GAG CAG GTC CAG AAA GTC ATG GGG ATC TGC AAT AAG TAC AAG 240
Ser Val Glu Gln Val Gln Lys Val Met Gly Ile Cys Asn Lys Tyr Lys

295                    300                    305

ATC CCG GTA TGG CCA ATC TCT ACC GGT CGG AAC TGG GGG TAT GGG TCC          288
Ile Pro Val Trp Pro Ile Ser Thr Gly Arg Asn Trp Gly Tyr Gly Ser
    310                    315                    320

GCT TCG CCT GCA ACT CCT GGG CAG ATG ATT CTT GAC CTT CGC AAG ATG          336
Ala Ser Pro Ala Thr Pro Gly Gln Met Ile Leu Asp Leu Arg Lys Met
325                    330                    335                    340

AAC AAG ATC ATT GAG ATC GAT GTT GAG GGG TGT ACT GCC CTG CTC GAG          384
Asn Lys Ile Ile Glu Ile Asp Val Glu Gly Cys Thr Ala Leu Leu Glu
                345                    350                    355

CCG GGC GTT ACC TAC CAG CAG CTT CAC GAT TAC ATC AAG GAG CAC AAT          432
Pro Gly Val Thr Tyr Gln Gln Leu His Asp Tyr Ile Lys Glu His Asn
            360                    365                    370

CTG CCC TTG ATG CTG GAT GTG CCG ACT ATT GGG CCT ATG GTT GGC CCG          480
Leu Pro Leu Met Leu Asp Val Pro Thr Ile Gly Pro Met Val Gly Pro
        375                    380                    385

GTG GGT AAC ACG CTG GAT CGA GGC GTT GGT TAT ACG CCG TAC GGC GAG          528
Val Gly Asn Thr Leu Asp Arg Gly Val Gly Tyr Thr Pro Tyr Gly Glu
    390                    395                    400

CAC TTC ATG ATG CAG TGT GGT ATG GAA GTC GTC ATG GCC GAT GGC GAA          576
His Phe Met Met Gln Cys Gly Met Glu Val Val Met Ala Asp Gly Glu
405                    410                    415                    420

ATC CTC CGT ACT GGT ATG GGC TCG GTG CCC AAA GCC AAG ACT TGG CAG          624
Ile Leu Arg Thr Gly Met Gly Ser Val Pro Lys Ala Lys Thr Trp Gln
                425                    430                    435

GCA TTC AAA TGG GGC TAT GGT CCA TAT CTG GAC GGT ATC TTT ACC CAG          672
Ala Phe Lys Trp Gly Tyr Gly Pro Tyr Leu Asp Gly Ile Phe Thr Gln
            440                    445                    450

TCC AAC TTT GGT GTT GTG ACA AAG CTC GGG ATT TGG TTG ATG CCC AAG          720
Ser Asn Phe Gly Val Val Thr Lys Leu Gly Ile Trp Leu Met Pro Lys
        455                    460                    465

CCG CCA GTG ATC AAG TCG TTT ATG ATC CGT TAT CCC AAT GAA GCT GAT          768
Pro Pro Val Ile Lys Ser Phe Met Ile Arg Tyr Pro Asn Glu Ala Asp
    470                    475                    480

GTG GTT AAG GCA ATT GAT GCT TTT CGC CCG CTG CGT ATT ACT CAG CTG          816
Val Val Lys Ala Ile Asp Ala Phe Arg Pro Leu Arg Ile Thr Gln Leu
485                    490                    495                    500

ATT CCT AAC GTC GTT TTG TTC ATG CAC GGC ATG TAC GAA ACG GCA ATC          864
Ile Pro Asn Val Val Leu Phe Met His Gly Met Tyr Glu Thr Ala Ile
                505                    510                    515

TGC CGG ACG CGT GCT GAG GTT ACT TCG GAC CCA GGT CCT ATT TCT GAA          912

Cys Arg Thr Arg Ala Glu Val Thr Ser Asp Pro Gly Pro Ile Ser Glu
520 525 530

GCG GAC GCC CGC AAA GCA TTC AAA GAG CTA GGC GTT GGC TAC TGG AAC 960
Ala Asp Ala Arg Lys Ala Phe Lys Glu Leu Gly Val Gly Tyr Trp Asn
535 540 545

GTT TAC TTC GCG CTT TAC GGC ACA GAA GAG CAG ATA GCC GTC AAT GAA 1008
Val Tyr Phe Ala Leu Tyr Gly Thr Glu Glu Gln Ile Ala Val Asn Glu
550 555 560

AAG ATC GTC CGC GGC ATC CTC GAA CCG ACG GGG GGT GAG ATC CTC ACC 1056
Lys Ile Val Arg Gly Ile Leu Glu Pro Thr Gly Gly Glu Ile Leu Thr
565 570 575 580

GAA GAG GAG GCT GGA GAT AAC ATT CTT TTC CAT CAC CAT AAG CAG CTC 1104
Glu Glu Glu Ala Gly Asp Asn Ile Leu Phe His His His Lys Gln Leu
585 590 595

ATG AAC GGC GAG ATG ACA TTG GAG GAA ATG AAT ATC TAC CAG TGG CGC 1152
Met Asn Gly Glu Met Thr Leu Glu Glu Met Asn Ile Tyr Gln Trp Arg
600 605 610

GGA GCA GGT GGC GGT GCT TGC TGG TTT GCA CCG GTT GCT CAG GTC AAG 1200
Gly Ala Gly Gly Gly Ala Cys Trp Phe Ala Pro Val Ala Gln Val Lys
615 620 625

GGG CAT GAG GCA GAG CAG CAG GTC AAG CTT GCT CAG AAG GTG CTT GCA 1248
Gly His Glu Ala Glu Gln Gln Val Lys Leu Ala Gln Lys Val Leu Ala
630 635 640

AAG CAT GGG TTC GAT TAC ACG GCG GGC TTT GCG ATT GGT TGG CGC GAT 1296
Lys His Gly Phe Asp Tyr Thr Ala Gly Phe Ala Ile Gly Trp Arg Asp
645 650 655 660

CTT CAC CAT GTG ATC GAT GTG CTG TAC GAC CGT AGC AAT GCC GAC GAG 1344
Leu His His Val Ile Asp Val Leu Tyr Asp Arg Ser Asn Ala Asp Glu
665 670 675

AAA AAG CGC GCT TAC GCT TGC TTT GAT GAA TTG ATC GAC GTC TTT GCG 1392
Lys Lys Arg Ala Tyr Ala Cys Phe Asp Glu Leu Ile Asp Val Phe Ala
680 685 690

GCC GAA GGC TTT GCA AGT TAC AGG ACC AAT ATT GCC TTT ATG GAC AAA 1440
Ala Glu Gly Phe Ala Ser Tyr Arg Thr Asn Ile Ala Phe Met Asp Lys
695 700 705

GTC GCC TCT AAG TTC GGC GCT GAG AAT AAG AGG GTC AAT CAG AAG ATC 1488
Val Ala Ser Lys Phe Gly Ala Glu Asn Lys Arg Val Asn Gln Lys Ile
710 715 720

AAG GCT GCC CTT GAT CCA AAC GGC ATC ATC GCT CCC GGC AAG TCG GGC 1536
Lys Ala Ala Leu Asp Pro Asn Gly Ile Ile Ala Pro Gly Lys Ser Gly
725 730 735 740

ATT CAT CTT CCC AAA TAA                                                    1554
Ile His Leu Pro Lys
                745

(2) ANGABEN ZU SEQ ID NO: 16:

        (i) SEQUENZKENNZEICHEN:
            (A) LÄNGE: 517 Aminosäuren
            (B) ART: Aminosäure
            (D) TOPOLOGIE: linear

    (ii) ART DES MOLEKÜLS: Protein
    (xi) SEQUENZBESCHREIBUNG: SEQ ID NO: 16:

Met Glu Ser Thr Val Val Leu Pro Glu Gly Val Thr Pro Glu Gln Phe
  1               5                  10                  15

Thr Lys Ala Ile Ser Glu Phe Arg Gln Val Leu Gly Glu Asp Ser Val
             20                  25                  30

Leu Val Thr Ala Glu Arg Val Val Pro Tyr Thr Lys Leu Leu Ile Pro
         35                  40                  45

Thr Gln Asp Asp Ala Gln Tyr Thr Pro Ala Gly Ala Leu Thr Pro Ser
     50                  55                  60

Ser Val Glu Gln Val Gln Lys Val Met Gly Ile Cys Asn Lys Tyr Lys
 65                  70                  75                  80

Ile Pro Val Trp Pro Ile Ser Thr Gly Arg Asn Trp Gly Tyr Gly Ser
                 85                  90                  95

Ala Ser Pro Ala Thr Pro Gly Gln Met Ile Leu Asp Leu Arg Lys Met
            100                 105                 110

Asn Lys Ile Ile Glu Ile Asp Val Glu Gly Cys Thr Ala Leu Leu Glu
        115                 120                 125

Pro Gly Val Thr Tyr Gln Gln Leu His Asp Tyr Ile Lys Glu His Asn
    130                 135                 140

Leu Pro Leu Met Leu Asp Val Pro Thr Ile Gly Pro Met Val Gly Pro
145                 150                 155                 160

Val Gly Asn Thr Leu Asp Arg Gly Val Gly Tyr Thr Pro Tyr Gly Glu
                165                 170                 175

His Phe Met Met Gln Cys Gly Met Glu Val Val Met Ala Asp Gly Glu
            180                 185                 190

Ile Leu Arg Thr Gly Met Gly Ser Val Pro Lys Ala Lys Thr Trp Gln
        195                 200                 205

Ala Phe Lys Trp Gly Tyr Gly Pro Tyr Leu Asp Gly Ile Phe Thr Gln

210                 215                 220

Ser Asn Phe Gly Val Val Thr Lys Leu Gly Ile Trp Leu Met Pro Lys
225                 230                 235                 240

Pro Pro Val Ile Lys Ser Phe Met Ile Arg Tyr Pro Asn Glu Ala Asp
                245                 250                 255

Val Val Lys Ala Ile Asp Ala Phe Arg Pro Leu Arg Ile Thr Gln Leu
            260                 265                 270

Ile Pro Asn Val Val Leu Phe Met His Gly Met Tyr Glu Thr Ala Ile
        275                 280                 285

Cys Arg Thr Arg Ala Glu Val Thr Ser Asp Pro Gly Pro Ile Ser Glu
    290                 295                 300

Ala Asp Ala Arg Lys Ala Phe Lys Glu Leu Gly Val Gly Tyr Trp Asn
305                 310                 315                 320

Val Tyr Phe Ala Leu Tyr Gly Thr Glu Glu Gln Ile Ala Val Asn Glu
                325                 330                 335

Lys Ile Val Arg Gly Ile Leu Glu Pro Thr Gly Gly Glu Ile Leu Thr
            340                 345                 350

Glu Glu Glu Ala Gly Asp Asn Ile Leu Phe His His His Lys Gln Leu
        355                 360                 365

Met Asn Gly Glu Met Thr Leu Glu Glu Met Asn Ile Tyr Gln Trp Arg
    370                 375                 380

Gly Ala Gly Gly Gly Ala Cys Trp Phe Ala Pro Val Ala Gln Val Lys
385                 390                 395                 400

Gly His Glu Ala Glu Gln Gln Val Lys Leu Ala Gln Lys Val Leu Ala
                405                 410                 415

Lys His Gly Phe Asp Tyr Thr Ala Gly Phe Ala Ile Gly Trp Arg Asp
            420                 425                 430

Leu His His Val Ile Asp Val Leu Tyr Asp Arg Ser Asn Ala Asp Glu
        435                 440                 445

Lys Lys Arg Ala Tyr Ala Cys Phe Asp Glu Leu Ile Asp Val Phe Ala
    450                 455                 460

Ala Glu Gly Phe Ala Ser Tyr Arg Thr Asn Ile Ala Phe Met Asp Lys
465                 470                 475                 480

Val Ala Ser Lys Phe Gly Ala Glu Asn Lys Arg Val Asn Gln Lys Ile
                485                 490                 495

Lys Ala Ala Leu Asp Pro Asn Gly Ile Ile Ala Pro Gly Lys Ser Gly
            500                 505                 510

Ile His Leu Pro Lys
        515

(2) ANGABEN ZU SEQ ID NO: 17:

(i) SEQUENZKENNZEICHEN:
(A) LÄNGE: 861 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..858
(D) SONSTIGE ANGABEN:/gene= "ORF2"

(xi) SEQUENZBESCHREIBUNG: SEQ ID NO: 17:

ATG ATT GCA ATC ACT GCG GGC ACC GGA AGT CTT GGT CGG GCT ATC GTT 48
Met Ile Ala Ile Thr Ala Gly Thr Gly Ser Leu Gly Arg Ala Ile Val
520 525 530

GAG CGA CTA GGG GAC TGC GGT CTT ATC GGT CAA GTT CGA TTG ACG GCT 96
Glu Arg Leu Gly Asp Cys Gly Leu Ile Gly Gln Val Arg Leu Thr Ala
535 540 545

CGC GAT CCT AAA AGG CTT CGT GCC GCT GCC GAG GAA GGG TTT CAG GTC 144
Arg Asp Pro Lys Arg Leu Arg Ala Ala Ala Glu Glu Gly Phe Gln Val
550 555 560 565

GCT AAG GCG GAT TAC GCC GAT ATT GGG AGT CTT GAC CAG GCA TTA CAG 192
Ala Lys Ala Asp Tyr Ala Asp Ile Gly Ser Leu Asp Gln Ala Leu Gln
570 575 580

GGG GTA GAC GTA TTA CTC CTG ATT TCT GGT ACT GCA CCC AAT GAA ATA 240
Gly Val Asp Val Leu Leu Leu Ile Ser Gly Thr Ala Pro Asn Glu Ile
585 590 595

AGG ATC CAA CAG CAT AAG TCG GTC ATC GAC GCG GCA AAA CGA AAC GGC 288
Arg Ile Gln Gln His Lys Ser Val Ile Asp Ala Ala Lys Arg Asn Gly
600 605 610

GTG TCG CGT ATT GTG TAT ACC AGC TTC ATA AAT CCA AGT ACT CGC AGC 336
Val Ser Arg Ile Val Tyr Thr Ser Phe Ile Asn Pro Ser Thr Arg Ser
615 620 625

AGG TCT ATT TGG GCC TCC ATT CAT CGT GAA ACT GAG ACT TAC CTC AGG 384

Arg Ser Ile Trp Ala Ser Ile His Arg Glu Thr Glu Thr Tyr Leu Arg
630                 635                 640                 645

CAG TCT GGG GTG AAG TTT ACG ATT GTC CGA AAT AAT CAG TAT GCG TCT     432
Gln Ser Gly Val Lys Phe Thr Ile Val Arg Asn Asn Gln Tyr Ala Ser
                650                 655                 660

AAC CTG GAT CTG TTG CTG CTG AGG GCT CAA GAC AGC GGA ATA TTT GCC     480
Asn Leu Asp Leu Leu Leu Leu Arg Ala Gln Asp Ser Gly Ile Phe Ala
            665                 670                 675

ATT CCC GGG GCG AAG GGG CGG GTG GCG TAC GTC TCT CAT CGC GAC GTT     528
Ile Pro Gly Ala Lys Gly Arg Val Ala Tyr Val Ser His Arg Asp Val
        680                 685                 690

GCC GCT GCC ATC TGT AGT GTC CTG ACG ACC GCC GGA CAC GAT AAC AGG     576
Ala Ala Ala Ile Cys Ser Val Leu Thr Thr Ala Gly His Asp Asn Arg
    695                 700                 705

ATC TAC CAG CTC ACA GGC TCT GAG GCT CTC AAT GGG CTC GAG ATC GCG     624
Ile Tyr Gln Leu Thr Gly Ser Glu Ala Leu Asn Gly Leu Glu Ile Ala
710                 715                 720                 725

GAG ATT CTT GGT GGG GTG CTC GGG CGT CCA GTG CGC GCG ATG GAT GCC     672
Glu Ile Leu Gly Gly Val Leu Gly Arg Pro Val Arg Ala Met Asp Ala
                730                 735                 740

TCG CCT GAC GAG TTT GCT GCC AGC TTT CGC GAG GCT GGA TTC CCT GAG     720
Ser Pro Asp Glu Phe Ala Ala Ser Phe Arg Glu Ala Gly Phe Pro Glu
            745                 750                 755

TTT ATG GTT GAA GGC CTA CTA AGC ATT TAT GCC GCT TCA GGT GCT GGG     768
Phe Met Val Glu Gly Leu Leu Ser Ile Tyr Ala Ala Ser Gly Ala Gly
        760                 765                 770

GAG TAC CAA TCC GTC AGT CCT GAT GTT GGG TTG TTG ACG GGA CGA CGT     816
Glu Tyr Gln Ser Val Ser Pro Asp Val Gly Leu Leu Thr Gly Arg Arg
    775                 780                 785

GCC GAA TCG ATG CGA ACT TAC ATA CAG CGT CTA GTT TGG CCT             858
Ala Glu Ser Met Arg Thr Tyr Ile Gln Arg Leu Val Trp Pro
790                 795                 800

TGA                                                                 861

(2) ANGABEN ZU SEQ ID NO: 18:

(i) SEQUENZKENNZEICHEN:
(A) LÄNGE: 286 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 18:

Met Ile Ala Ile Thr Ala Gly Thr Gly Ser Leu Gly Arg Ala Ile Val
1               5                   10                  15

Glu Arg Leu Gly Asp Cys Gly Leu Ile Gly Gln Val Arg Leu Thr Ala
            20                  25                  30

Arg Asp Pro Lys Arg Leu Arg Ala Ala Ala Glu Glu Gly Phe Gln Val
        35                  40                  45

Ala Lys Ala Asp Tyr Ala Asp Ile Gly Ser Leu Asp Gln Ala Leu Gln
    50                  55                  60

Gly Val Asp Val Leu Leu Leu Ile Ser Gly Thr Ala Pro Asn Glu Ile
65                  70                  75                  80

Arg Ile Gln Gln His Lys Ser Val Ile Asp Ala Ala Lys Arg Asn Gly
                85                  90                  95

Val Ser Arg Ile Val Tyr Thr Ser Phe Ile Asn Pro Ser Thr Arg Ser
            100                 105                 110

Arg Ser Ile Trp Ala Ser Ile His Arg Glu Thr Glu Thr Tyr Leu Arg
        115                 120                 125

Gln Ser Gly Val Lys Phe Thr Ile Val Arg Asn Asn Gln Tyr Ala Ser
    130                 135                 140

Asn Leu Asp Leu Leu Leu Leu Arg Ala Gln Asp Ser Gly Ile Phe Ala
145                 150                 155                 160

Ile Pro Gly Ala Lys Gly Arg Val Ala Tyr Val Ser His Arg Asp Val
                165                 170                 175

Ala Ala Ala Ile Cys Ser Val Leu Thr Thr Ala Gly His Asp Asn Arg
            180                 185                 190

Ile Tyr Gln Leu Thr Gly Ser Glu Ala Leu Asn Gly Leu Glu Ile Ala
        195                 200                 205

Glu Ile Leu Gly Gly Val Leu Gly Arg Pro Val Arg Ala Met Asp Ala
    210                 215                 220

Ser Pro Asp Glu Phe Ala Ala Ser Phe Arg Glu Ala Gly Phe Pro Glu
225                 230                 235                 240

Phe Met Val Glu Gly Leu Leu Ser Ile Tyr Ala Ala Ser Gly Ala Gly
                245                 250                 255

Glu Tyr Gln Ser Val Ser Pro Asp Val Gly Leu Leu Thr Gly Arg Arg
            260                 265                 270

Ala Glu Ser Met Arg Thr Tyr Ile Gln Arg Leu Val Trp Pro
        275                 280                 285

(2) ANGABEN ZU SEQ ID NO: 19:

(i) SEQUENZKENNZEICHEN:
(A) LÄNGE: 1011 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1008
(D) SONSTIGE ANGABEN:/product= "Alkohol-Dehydrogenase"
/gene= "adh"

(xi) SEQUENZBESCHREIBUNG: SEQ ID NO: 19:

ATG AAG GCT TAT GAG CTT CAC AAG ATT TCG GAA CAG GTA GAG GTC AGG 48
Met Lys Ala Tyr Glu Leu His Lys Ile Ser Glu Gln Val Glu Val Arg
290 295 300

CTC CAG CCA ACT CGG CCC CGC CCG CAG TTG AAT CAT GGC GAG GTC CTC 96
Leu Gln Pro Thr Arg Pro Arg Pro Gln Leu Asn His Gly Glu Val Leu
305 310 315

ATC AGG GTC CAT GCA GCC TCG CTC AAC TTT CGC GAT TTG ATG ATC TTG 144
Ile Arg Val His Ala Ala Ser Leu Asn Phe Arg Asp Leu Met Ile Leu
320 325 330

GCC GGT CGC TAT CCG GGT CAA ATG AAA CCC GAT GTG ATC CCG CTG TCC 192
Ala Gly Arg Tyr Pro Gly Gln Met Lys Pro Asp Val Ile Pro Leu Ser
335 340 345 350

GAT GGT GCT GGC GAG ATT GTG GAG GTC GGG CCT GGC GTA TCT TCG GAG 240
Asp Gly Ala Gly Glu Ile Val Glu Val Gly Pro Gly Val Ser Ser Glu
355 360 365

GTG CAG GGT CAG CGC GTA GCC AGC ACC TTT TTC CCT AAC TGG CGG GCC 288
Val Gln Gly Gln Arg Val Ala Ser Thr Phe Phe Pro Asn Trp Arg Ala
370 375 380

GGA AAG ATT ACC GAG CCG GCT ATT GAG GTG TCG TTG GGC TTC GGT ATG 336
Gly Lys Ile Thr Glu Pro Ala Ile Glu Val Ser Leu Gly Phe Gly Met
385 390 395

GAC GGG ATG CTC GCG GAA TAC GTT GCT CTG CCC TAT GAG GCA ACG ATA 384
Asp Gly Met Leu Ala Glu Tyr Val Ala Leu Pro Tyr Glu Ala Thr Ile
400 405 410

CCG ATA CCG GAG CAC CTG TCG TAC GAG GAG GCT GCA ACA TTG CCT TGC 432

Pro Ile Pro Glu His Leu Ser Tyr Glu Glu Ala Ala Thr Leu Pro Cys
415                 420                 425                 430

GCG GCG CTA ACC GCT TGG AAT GCG TTG ACC GAA GTG GGG CGT GTC AAG     480
Ala Ala Leu Thr Ala Trp Asn Ala Leu Thr Glu Val Gly Arg Val Lys
                435                 440                 445

GCC GGT GAT ACG GTC TTG TTG CTT GGC ACT GGC GGT GTC TCG ATG TTC     528
Ala Gly Asp Thr Val Leu Leu Leu Gly Thr Gly Gly Val Ser Met Phe
            450                 455                 460

GCG TTG CAG TTC GCC AAG CTC TTG GGG GCG ACG GTC ATT CAC ACC TCG     576
Ala Leu Gln Phe Ala Lys Leu Leu Gly Ala Thr Val Ile His Thr Ser
        465                 470                 475

AGC AGT GAA CAA AAG CTG GAG AGG GTG AAA GCG ATG GGG GCT GAT CAT     624
Ser Ser Glu Gln Lys Leu Glu Arg Val Lys Ala Met Gly Ala Asp His
    480                 485                 490

CTG ATC AAC TAC CGC AAT TCG CCA GGG TGG GAC CGT ACT GTC CTG GAT     672
Leu Ile Asn Tyr Arg Asn Ser Pro Gly Trp Asp Arg Thr Val Leu Asp
495                 500                 505                 510

CTC ACC GCG GGG CGA GGG GTT GAC CTG GTA GTC GAG GTA GGG GGG GCG     720
Leu Thr Ala Gly Arg Gly Val Asp Leu Val Val Glu Val Gly Gly Ala
                515                 520                 525

GGG ACC TTG GAG CGC TCA CTT CGT GCG GTC AAG GTA GGC GGT ATT GTC     768
Gly Thr Leu Glu Arg Ser Leu Arg Ala Val Lys Val Gly Gly Ile Val
            530                 535                 540

GCC ACG ATT GGG CTA GTG GCT GGC GTT GGC CCG ATT GAC CCA TTG CCG     816
Ala Thr Ile Gly Leu Val Ala Gly Val Gly Pro Ile Asp Pro Leu Pro
        545                 550                 555

CTT ATC TCC AGG GCT ATT CAG CTC TCG GGC GTC TAT GTC GGT TCC CGG     864
Leu Ile Ser Arg Ala Ile Gln Leu Ser Gly Val Tyr Val Gly Ser Arg
    560                 565                 570

GAA ATG TTT CTC TCA ATG AAC AAA GCC ATT GCA TCA GCC GAA ATC AAG     912
Glu Met Phe Leu Ser Met Asn Lys Ala Ile Ala Ser Ala Glu Ile Lys
575                 580                 585                 590

CCA GTG ATC GAT TGC TGC TTC CCC ATC GAC GAG GTT GGA GAT GCT TAT     960
Pro Val Ile Asp Cys Cys Phe Pro Ile Asp Glu Val Gly Asp Ala Tyr
                595                 600                 605

GAG TAC ATG CGT AGC GGC AAT CAC CTT GGC AAA GTA GTT ATC ACG ATC    1008
Glu Tyr Met Arg Ser Gly Asn His Leu Gly Lys Val Val Ile Thr Ile
            610                 615                 620

TAA                                                                1011

(2) ANGABEN ZU SEQ ID NO: 20:

(i) SEQUENZKENNZEICHEN:
(A) LÄNGE: 336 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 20:

Met Lys Ala Tyr Glu Leu His Lys Ile Ser Glu Gln Val Glu Val Arg
1               5                   10                  15

Leu Gln Pro Thr Arg Pro Arg Pro Gln Leu Asn His Gly Glu Val Leu
            20                  25                  30

Ile Arg Val His Ala Ala Ser Leu Asn Phe Arg Asp Leu Met Ile Leu
        35                  40                  45

Ala Gly Arg Tyr Pro Gly Gln Met Lys Pro Asp Val Ile Pro Leu Ser
    50                  55                  60

Asp Gly Ala Gly Glu Ile Val Glu Val Gly Pro Gly Val Ser Ser Glu
65                  70                  75                  80

Val Gln Gly Gln Arg Val Ala Ser Thr Phe Phe Pro Asn Trp Arg Ala
                85                  90                  95

Gly Lys Ile Thr Glu Pro Ala Ile Glu Val Ser Leu Gly Phe Gly Met
            100                 105                 110

Asp Gly Met Leu Ala Glu Tyr Val Ala Leu Pro Tyr Glu Ala Thr Ile
        115                 120                 125

Pro Ile Pro Glu His Leu Ser Tyr Glu Glu Ala Ala Thr Leu Pro Cys
    130                 135                 140

Ala Ala Leu Thr Ala Trp Asn Ala Leu Thr Glu Val Gly Arg Val Lys
145                 150                 155                 160

Ala Gly Asp Thr Val Leu Leu Leu Gly Thr Gly Gly Val Ser Met Phe
                165                 170                 175

Ala Leu Gln Phe Ala Lys Leu Leu Gly Ala Thr Val Ile His Thr Ser
            180                 185                 190

Ser Ser Glu Gln Lys Leu Glu Arg Val Lys Ala Met Gly Ala Asp His
        195                 200                 205

Leu Ile Asn Tyr Arg Asn Ser Pro Gly Trp Asp Arg Thr Val Leu Asp
    210                 215                 220

Leu Thr Ala Gly Arg Gly Val Asp Leu Val Val Glu Val Gly Gly Ala
225                 230                 235                 240

Gly Thr Leu Glu Arg Ser Leu Arg Ala Val Lys Val Gly Gly Ile Val
                245                 250                 255

Ala Thr Ile Gly Leu Val Ala Gly Val Gly Pro Ile Asp Pro Leu Pro
            260                 265                 270

Leu Ile Ser Arg Ala Ile Gln Leu Ser Gly Val Tyr Val Gly Ser Arg
        275                 280                 285

Glu Met Phe Leu Ser Met Asn Lys Ala Ile Ala Ser Ala Glu Ile Lys
    290                 295                 300

Pro Val Ile Asp Cys Cys Phe Pro Ile Asp Glu Val Gly Asp Ala Tyr
305                 310                 315                 320

Glu Tyr Met Arg Ser Gly Asn His Leu Gly Lys Val Val Ile Thr Ile
                325                 330                 335

(2) ANGABEN ZU SEQ ID NO: 21:

(i) SEQUENZKENNZEICHEN:
(A) LÄNGE: 1518 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:complement (4..1518)
(D) SONSTIGE ANGABEN:/product=
"Lignostilben-Dioxygenase"
/gene= "lsd"

(xi) SEQUENZBESCHREIBUNG: SEQ ID NO: 21:

TCACCGTCGT GATCGGGATT GGAAATTCGT GCGAGGACAG CGGCCACGTA CCGGCGCCCT      60

GAAGGGCTGG AAGGTTGGAG TTTCGTTAAG GTCTGGTACC CAGCAGCCAT GGAGAGCGGC     120

CCTTAGCCGG AATGGCAGCT TGATGGTTGC CACGGGACCA GACTGGATGT CTTGAGTGTC     180

GAGAATTACC AGATCGCTGC GATTTTCATC GAGGCGACCA ACCACGGTCA GCAAGTACCC     240

GTCACCTTCG GCGGCGGTCG GACTTCTAGG GACGAAGGCC GGCTCCTGGG CCGCCGAGGC     300

TTCGCCGGAG TACCAGAGGT CGTAGTCACC TCGGTGGTTG TCCCAGATGC CGAGTGAGTT     360

GTACGCGAAT ATCTTCTCGG CCTGCTGATG CGCAAGTGGT TTGCGTGGAT CGTCCACCCC     420

CATAAAGCCA TAGCGGTTGC ATTGCAGGGC GAACGAAGAA TCCATGATTG GCATTTCCGC 480

AAAGAAATCG TGTAGCCGGG TTCGCTTGAT CTCGTCGCTG CTGCTATCGA GGTCAATTTC 540

CCAACGAGTC AGGCGTGGTA CGGCTTTCTC AGGGGCGAAG GGTTGGTTTT GTGAGTTGGG 600

GAAGGGGAAC GGCAGGATTT CACTTTCCAT AAGGTCGATA TAAATCTTGG TTCCGACTTC 660

CCAAGCATTC ACAACATGAA ATACCCAGAG CGCCGGTGCC TTGAGCCAGC GAATCAGACT 720

GCCCTGGCGC GGCGCGAGTA CGCCAATGTA GCTGCCCAGT TCCGGCTCCC ACATATAAAT 780

TGGCTGTTTC GCCTTGAGGC GGGACAGGCT GTTGGTGGCC GGCATAATTG GGAAAATGGA 840

CCAATTTCGG GTAATGGCAA AGTCGTGCAT GAATGCGCCA TAGGGCTGCT CAAACCAAGT 900

TTCATGTGTC ACCTTGCCGT GCTTGTCGAC AATGTAATAG GCCATGTCTG GAGTTGCTTC 960

GCCCTTAGCT GCCGAACCGA AGAACAACAA GTCACCCGTT TCCGGGTCAT ATTTTGGATG 1020

GGCGGTGTGG GTTTGGCTGG TAACTTGGCC GTCGTAGTCG AAGTGTCCGC GAGTTTCAAG 1080

TGTACGAGGA TCCAGTTCGT ACGGTAGGCC GTCTTCCTTC ACCGCCAGCA CCTTGCCGTG 1140

ATGGCTAATG ATGCTTGTAT TGGCAACGGT GCGGTCTAGT CCTTTTACAC TGGTGTCGTC 1200

GGTATAGGGG TTTCTGTACA TGCCAAATAG CGATTTTCGC GCTAGTCGTT CGGCCGTGAA 1260

TCGAGCGGTT TTAACCCAGC GACTGATGAA GTCGACATGA CCATCTTCGA AGTGGAAGGC 1320

AGAGGCCATT CCATCTCCAT CTATGAAGGT GTGGAATTTT TGTGGGGTAA CTTGAGGCTC 1380

TGGCGTATTA CGGTAGAACG TTCCATTTAT TGATTTTGGG ATTTCGCCGT CAACCTCTAG 1440

ATCGAACAAG TCTGCCTCTA TACGGGTGGG GAGAAGTGTT CCTACTAATT GCGGGTCGTT 1500

GCGGTTGAAT CTCGCCAT 1518

(2) ANGABEN ZU SEQ ID NO: 22:

(i) SEQUENZKENNZEICHEN:
(A) LÄNGE: 505 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 22:

Met Ala Arg Phe Asn Arg Asn Asp Pro Gln Leu Val Gly Thr Leu Leu
1               5                   10                  15

Pro Thr Arg Ile Glu Ala Asp Leu Phe Asp Leu Glu Val Asp Gly Glu
            20                  25                  30

Ile Pro Lys Ser Ile Asn Gly Thr Phe Tyr Arg Asn Thr Pro Glu Pro
            35                  40                  45

Gln Val Thr Pro Gln Lys Phe His Thr Phe Ile Asp Gly Asp Gly Met
    50                  55                  60

Ala Ser Ala Phe His Phe Glu Asp Gly His Val Asp Phe Ile Ser Arg
65                  70                  75                  80

Trp Val Lys Thr Ala Arg Phe Thr Ala Glu Arg Leu Ala Arg Lys Ser
                85                  90                  95

Leu Phe Gly Met Tyr Arg Asn Pro Tyr Thr Asp Asp Thr Ser Val Lys
            100                 105                 110

Gly Leu Asp Arg Thr Val Ala Asn Thr Ser Ile Ile Ser His His Gly
        115                 120                 125

Lys Val Leu Ala Val Lys Glu Asp Gly Leu Pro Tyr Glu Leu Asp Pro
    130                 135                 140

Arg Thr Leu Glu Thr Arg Gly His Phe Asp Tyr Asp Gly Gln Val Thr
145                 150                 155                 160

Ser Gln Thr His Thr Ala His Pro Lys Tyr Asp Pro Glu Thr Gly Asp
                165                 170                 175

Leu Leu Phe Phe Gly Ser Ala Ala Lys Gly Glu Ala Thr Pro Asp Met
            180                 185                 190

Ala Tyr Tyr Ile Val Asp Lys His Gly Lys Val Thr His Glu Thr Trp
        195                 200                 205

Phe Glu Gln Pro Tyr Gly Ala Phe Met His Asp Phe Ala Ile Thr Arg
    210                 215                 220

Asn Trp Ser Ile Phe Pro Ile Met Pro Ala Thr Asn Ser Leu Ser Arg
225                 230                 235                 240

Leu Lys Ala Lys Gln Pro Ile Tyr Met Trp Glu Pro Glu Leu Gly Ser
                245                 250                 255

Tyr Ile Gly Val Leu Ala Pro Arg Gln Gly Ser Leu Ile Arg Trp Leu
            260                 265                 270

Lys Ala Pro Ala Leu Trp Val Phe His Val Val Asn Ala Trp Glu Val
        275                 280                 285

Gly Thr Lys Ile Tyr Ile Asp Leu Met Glu Ser Glu Ile Leu Pro Phe
    290                 295                 300

Pro Phe Pro Asn Ser Gln Asn Gln Pro Phe Ala Pro Glu Lys Ala Val
305                 310                 315                 320

Pro Arg Leu Thr Arg Trp Glu Ile Asp Leu Asp Ser Ser Ser Asp Glu

                325                 330                 335

Ile Lys Arg Thr Arg Leu His Asp Phe Phe Ala Glu Met Pro Ile Met
            340                 345                 350

Asp Ser Ser Phe Ala Leu Gln Cys Asn Arg Tyr Gly Phe Met Gly Val
        355                 360                 365

Asp Asp Pro Arg Lys Pro Leu Ala His Gln Gln Ala Glu Lys Ile Phe
    370                 375                 380

Ala Tyr Asn Ser Leu Gly Ile Trp Asp Asn His Arg Gly Asp Tyr Asp
385                 390                 395                 400

Leu Trp Tyr Ser Gly Glu Ala Ser Ala Ala Gln Glu Pro Ala Phe Val
                405                 410                 415

Pro Arg Ser Pro Thr Ala Ala Glu Gly Asp Gly Tyr Leu Leu Thr Val
            420                 425                 430

Val Gly Arg Leu Asp Glu Asn Arg Ser Asp Leu Val Ile Leu Asp Thr
        435                 440                 445

Gln Asp Ile Gln Ser Gly Pro Val Ala Thr Ile Lys Leu Pro Phe Arg
    450                 455                 460

Leu Arg Ala Ala Leu His Gly Cys Trp Val Pro Asp Leu Asn Glu Thr
465                 470                 475                 480

Pro Thr Phe Gln Pro Phe Arg Ala Pro Val Arg Gly Arg Cys Pro Arg
                485                 490                 495

Thr Asn Phe Gln Ser Arg Ser Arg Arg
            500                 505

(2) ANGABEN ZU SEQ ID NO: 23:

     (i) SEQUENZKENNZEICHEN:
          (A) LÄNGE: 951 Basenpaare
          (B) ART: Nucleotid
          (C) STRANGFORM: Doppelstrang
          (D) TOPOLOGIE: linear

    (ii) ART DES MOLEKÜLS: Genom-DNA

   (iii) HYPOTHETISCH: NEIN

    (iv) ANTISENSE: NEIN


    (ix) MERKMAL:
          (A) NAME/SCHLÜSSEL: CDS
          (B) LAGE:1..948
          (D) SONSTIGE ANGABEN:/gene= "ORF3"

(xi) SEQUENZBESCHREIBUNG: SEQ ID NO: 23:

ATG ACA ACT ATT CGG TGG CGG CGT ATG TCC ATT CAC TCT GAG GGG ATC 48
Met Thr Thr Ile Arg Trp Arg Arg Met Ser Ile His Ser Glu Gly Ile
510 515 520

ACT CTC GCG GAT TCG CCG CTG CAT TGG GCG CAT ACC CTG AAT GGA TCA 96
Thr Leu Ala Asp Ser Pro Leu His Trp Ala His Thr Leu Asn Gly Ser
525 530 535

ATG CGT ACT CAT TTC GAA GTC CAG CGT CTT GAG CGG GGT AGA GGT GCC 144
Met Arg Thr His Phe Glu Val Gln Arg Leu Glu Arg Gly Arg Gly Ala
540 545 550

TCC CTT GCC CGA TCT AGA TTT GGC GCG GGT GAG CTG TAC AGT GCC ATT 192
Ser Leu Ala Arg Ser Arg Phe Gly Ala Gly Glu Leu Tyr Ser Ala Ile
555 560 565

GCA CCA AGC CAG GTA CTT CGC CAC TTC AAC GAC CAG CGA AAT GCT GAT 240
Ala Pro Ser Gln Val Leu Arg His Phe Asn Asp Gln Arg Asn Ala Asp
570 575 580 585

GAG GCT GAG CAC AGC TAT TTG ATT CAG ATA CGA AGT GGC GCT TTG GGC 288
Glu Ala Glu His Ser Tyr Leu Ile Gln Ile Arg Ser Gly Ala Leu Gly
590 595 600

GTT GCA TCC GGC GGA AGA AAG GTG ATC TTG GCA AAT GGT GAT TGC TCC 336
Val Ala Ser Gly Gly Arg Lys Val Ile Leu Ala Asn Gly Asp Cys Ser
605 610 615

ATA GTT GAT AGT CGC CAA GAC TTC ACA CTT TCC TCG AAC TCT TCG ACC 384
Ile Val Asp Ser Arg Gln Asp Phe Thr Leu Ser Ser Asn Ser Ser Thr
620 625 630

CAA GGT GTC GTA ATA CGC TTT CCG GTG AGT TGG CTG GGA GCG TGG GTG 432
Gln Gly Val Val Ile Arg Phe Pro Val Ser Trp Leu Gly Ala Trp Val
635 640 645

TCC AAT CCG GAG GAT CTT ATC GCC CGA CGA GTT GAT GCT GAG GTA GGG 480
Ser Asn Pro Glu Asp Leu Ile Ala Arg Arg Val Asp Ala Glu Val Gly
650 655 660 665

TGG GGT AGG GCG CTA AGC GCA TCG GTT TCT AAT CTA GAT CCA TTG CGC 528
Trp Gly Arg Ala Leu Ser Ala Ser Val Ser Asn Leu Asp Pro Leu Arg
670 675 680

ATC GAC GAT TTA GGT AGC AAT GTA AAT GGC ATT GCA GAG CAT GTT GCT 576
Ile Asp Asp Leu Gly Ser Asn Val Asn Gly Ile Ala Glu His Val Ala
685 690 695

ATG TTA ATT TCA CTA GCA AGT TCT GCG GTT AGT TCT GAA GAT GGG GGT 624
Met Leu Ile Ser Leu Ala Ser Ser Ala Val Ser Ser Glu Asp Gly Gly
700 705 710

GTG GCT CTT CGG AAA ATG AGG GAA GTG AAG AGA GTA CTC GAG CAG AGT 672

Val Ala Leu Arg Lys Met Arg Glu Val Lys Arg Val Leu Glu Gln Ser
    715                 720                 725

TTC GCA GAC GCT AAT CTC GGG CCG GAA AGT GTT TCA AGT CAA TTA GGA         720
Phe Ala Asp Ala Asn Leu Gly Pro Glu Ser Val Ser Ser Gln Leu Gly
730                 735                 740                 745

ATT TCG AAA CGC TAT TTG CAT TAT GTC TTT GCT GCG TGC GGT ACG ACC         768
Ile Ser Lys Arg Tyr Leu His Tyr Val Phe Ala Ala Cys Gly Thr Thr
                750                 755                 760

TTT GGT CGC GAG CTG TTG GAA ATA CGC CTG GGC AAA GCT TAT CGA ATG         816
Phe Gly Arg Glu Leu Leu Glu Ile Arg Leu Gly Lys Ala Tyr Arg Met
            765                 770                 775

CTC TGT GCG GCG AGT GAC TCG GGT GCT GTG CTG AAG GTG GCC ATG TCC         864
Leu Cys Ala Ala Ser Asp Ser Gly Ala Val Leu Lys Val Ala Met Ser
        780                 785                 790

TCA GGT TTT TCG GAT TCA AGC CAT TTC AGC AAG AAA TTT AAG GAA AGA         912
Ser Gly Phe Ser Asp Ser Ser His Phe Ser Lys Lys Phe Lys Glu Arg
    795                 800                 805

TAC GGT GTT TCG CCT GTC TCC TTG GTG AGG CAG GCT TGA                     951
Tyr Gly Val Ser Pro Val Ser Leu Val Arg Gln Ala
810                 815                 820

(2) ANGABEN ZU SEQ ID NO: 24:

    (i) SEQUENZKENNZEICHEN:
        (A) LÄNGE: 316 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 24:

Met Thr Thr Ile Arg Trp Arg Arg Met Ser Ile His Ser Glu Gly Ile
  1               5                  10                  15

Thr Leu Ala Asp Ser Pro Leu His Trp Ala His Thr Leu Asn Gly Ser
             20                  25                  30

Met Arg Thr His Phe Glu Val Gln Arg Leu Glu Arg Gly Arg Gly Ala
         35                  40                  45

Ser Leu Ala Arg Ser Arg Phe Gly Ala Gly Glu Leu Tyr Ser Ala Ile
     50                  55                  60

Ala Pro Ser Gln Val Leu Arg His Phe Asn Asp Gln Arg Asn Ala Asp
 65                  70                  75                  80

Glu Ala Glu His Ser Tyr Leu Ile Gln Ile Arg Ser Gly Ala Leu Gly
                 85                  90                  95

Val Ala Ser Gly Gly Arg Lys Val Ile Leu Ala Asn Gly Asp Cys Ser
            100                 105                 110

Ile Val Asp Ser Arg Gln Asp Phe Thr Leu Ser Ser Asn Ser Ser Thr
        115                 120                 125

Gln Gly Val Val Ile Arg Phe Pro Val Ser Trp Leu Gly Ala Trp Val
    130                 135                 140

Ser Asn Pro Glu Asp Leu Ile Ala Arg Arg Val Asp Ala Glu Val Gly
145                 150                 155                 160

Trp Gly Arg Ala Leu Ser Ala Ser Val Ser Asn Leu Asp Pro Leu Arg
                165                 170                 175

Ile Asp Asp Leu Gly Ser Asn Val Asn Gly Ile Ala Glu His Val Ala
            180                 185                 190

Met Leu Ile Ser Leu Ala Ser Ser Ala Val Ser Ser Glu Asp Gly Gly
        195                 200                 205

Val Ala Leu Arg Lys Met Arg Glu Val Lys Arg Val Leu Glu Gln Ser
    210                 215                 220

Phe Ala Asp Ala Asn Leu Gly Pro Glu Ser Val Ser Ser Gln Leu Gly
225                 230                 235                 240

Ile Ser Lys Arg Tyr Leu His Tyr Val Phe Ala Ala Cys Gly Thr Thr
                245                 250                 255

Phe Gly Arg Glu Leu Leu Glu Ile Arg Leu Gly Lys Ala Tyr Arg Met
            260                 265                 270

Leu Cys Ala Ala Ser Asp Ser Gly Ala Val Leu Lys Val Ala Met Ser
        275                 280                 285

Ser Gly Phe Ser Asp Ser Ser His Phe Ser Lys Lys Phe Lys Glu Arg
    290                 295                 300

Tyr Gly Val Ser Pro Val Ser Leu Val Arg Gln Ala
305                 310                 315

(2) ANGABEN ZU SEQ ID NO: 25:

     (i) SEQUENZKENNZEICHEN:
          (A) LÄNGE: 735 Basenpaare
          (B) ART: Nucleotid
          (C) STRANGFORM: Doppelstrang
          (D) TOPOLOGIE: linear

    (ii) ART DES MOLEKÜLS: Genom-DNA

   (iii) HYPOTHETISCH: NEIN

    (iv) ANTISENSE: NEIN

(ix) MERKMAL:
(A) NAME/SCHLÜSSEL: CDS
(B) LAGE:1..732
(D) SONSTIGE ANGABEN:/product= "Enoyl-CoA-Hydratase"
/gene= "ech"

(xi) SEQUENZBESCHREIBUNG: SEQ ID NO: 25:

ATG AGC CCA ACT CTC AAT CGA GAG ATG GTC GAG GTT CTG GAG GTG CTG 48
Met Ser Pro Thr Leu Asn Arg Glu Met Val Glu Val Leu Glu Val Leu
320 325 330

GAG CAG GAC GCA GAT GCT CGC GTG CTT GTT CTG ACT GGT GCA GGC GAA 96
Glu Gln Asp Ala Asp Ala Arg Val Leu Val Leu Thr Gly Ala Gly Glu
335 340 345

TCC TGG ACC GCG GGC ATG GAC CTG AAG GAG TAT TTC CGC GAG ACC GAT 144
Ser Trp Thr Ala Gly Met Asp Leu Lys Glu Tyr Phe Arg Glu Thr Asp
350 355 360

GCT GGC CCC GAA ATT CTG CAA GAG AAG ATT CGT CGC GAA GCG TCG ACC 192
Ala Gly Pro Glu Ile Leu Gln Glu Lys Ile Arg Arg Glu Ala Ser Thr
365 370 375 380

TGG CAG TGG AAG CTC CTG CGG ATG TAC ACC AAG CCG ACC ATC GCG ATG 240
Trp Gln Trp Lys Leu Leu Arg Met Tyr Thr Lys Pro Thr Ile Ala Met
385 390 395

GTC AAT GGC TGG TGC TTC GGC GGC GGC TTC AGC CCG CTG GTG GCC TGT 288
Val Asn Gly Trp Cys Phe Gly Gly Gly Phe Ser Pro Leu Val Ala Cys
400 405 410

GAT CTG GCC ATC TGT GCC GAC GAG GCC ACC TTT GGC CTG TCC GAG ATC 336
Asp Leu Ala Ile Cys Ala Asp Glu Ala Thr Phe Gly Leu Ser Glu Ile
415 420 425

AAC TGG GGC ATC CCG CCG GGC AAC CTG GTG AGT AAG GCT ATG GCC GAC 384
Asn Trp Gly Ile Pro Pro Gly Asn Leu Val Ser Lys Ala Met Ala Asp
430 435 440

ACC GTG GGT CAC CGC GAG TCC CTT TAC TAC ATC ATG ACT GGC AAG ACA 432
Thr Val Gly His Arg Glu Ser Leu Tyr Tyr Ile Met Thr Gly Lys Thr
445 450 455 460

TTT GGC GGT CAG CAG GCC GCC AAG ATG GGG CTT GTG AAC CAG AGT GTT 480
Phe Gly Gly Gln Gln Ala Ala Lys Met Gly Leu Val Asn Gln Ser Val
465 470 475

CCG CTG GCC GAG CTG CGC AGT GTC ACT GTA GAG CTG GCT CAG AAC CTG 528
Pro Leu Ala Glu Leu Arg Ser Val Thr Val Glu Leu Ala Gln Asn Leu
480 485 490

CTG GAC AAG AAC CCC GTA GTG CTG CGT GCC GCC AAA ATA GGC TTC AAG 576

Leu Asp Lys Asn Pro Val Val Leu Arg Ala Ala Lys Ile Gly Phe Lys
495 500 505

CGT TGC CGC GAG CTG ACT TGG GAG CAG AAC GAG GAC TAC CTG TAC GCC 624
Arg Cys Arg Glu Leu Thr Trp Glu Gln Asn Glu Asp Tyr Leu Tyr Ala
510 515 520

AAG CTC GAC CAA TCC CGT TTG CTC GAT CCG GAA GGC GGT CGC GAG CAG 672
Lys Leu Asp Gln Ser Arg Leu Leu Asp Pro Glu Gly Gly Arg Glu Gln
525 530 535 540

GGC ATG AAG CAG TTC CTT GAC GAG AAA AGC ATC AAG CCG GGC TTG CAG 720
Gly Met Lys Gln Phe Leu Asp Glu Lys Ser Ile Lys Pro Gly Leu Gln
545 550 555

ACC TAC AAG CGC TGA 735
Thr Tyr Lys Arg
560

(2) ANGABEN ZU SEQ ID NO: 26:

(i) SEQUENZKENNZEICHEN:
(A) LÄNGE: 244 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 26:

Met Ser Pro Thr Leu Asn Arg Glu Met Val Glu Val Leu Glu Val Leu
1               5                   10                  15

Glu Gln Asp Ala Asp Ala Arg Val Leu Val Leu Thr Gly Ala Gly Glu
            20                  25                  30

Ser Trp Thr Ala Gly Met Asp Leu Lys Glu Tyr Phe Arg Glu Thr Asp
        35                  40                  45

Ala Gly Pro Glu Ile Leu Gln Glu Lys Ile Arg Arg Glu Ala Ser Thr
    50                  55                  60

Trp Gln Trp Lys Leu Leu Arg Met Tyr Thr Lys Pro Thr Ile Ala Met
65                  70                  75                  80

Val Asn Gly Trp Cys Phe Gly Gly Gly Phe Ser Pro Leu Val Ala Cys
                85                  90                  95

Asp Leu Ala Ile Cys Ala Asp Glu Ala Thr Phe Gly Leu Ser Glu Ile
            100                 105                 110

Asn Trp Gly Ile Pro Pro Gly Asn Leu Val Ser Lys Ala Met Ala Asp
        115                 120                 125

Thr Val Gly His Arg Glu Ser Leu Tyr Tyr Ile Met Thr Gly Lys Thr

130                 135                 140

Phe Gly Gly Gln Gln Ala Ala Lys Met Gly Leu Val Asn Gln Ser Val
145                 150                 155                 160

Pro Leu Ala Glu Leu Arg Ser Val Thr Val Glu Leu Ala Gln Asn Leu
                165                 170                 175

Leu Asp Lys Asn Pro Val Val Leu Arg Ala Ala Lys Ile Gly Phe Lys
            180                 185                 190

Arg Cys Arg Glu Leu Thr Trp Glu Gln Asn Glu Asp Tyr Leu Tyr Ala
        195                 200                 205

Lys Leu Asp Gln Ser Arg Leu Leu Asp Pro Glu Gly Gly Arg Glu Gln
    210                 215                 220

Gly Met Lys Gln Phe Leu Asp Glu Lys Ser Ile Lys Pro Gly Leu Gln
225                 230                 235                 240

Thr Tyr Lys Arg

(2) ANGABEN ZU SEQ ID NO: 27:

(i) SEQUENZKENNZEICHEN:
(A) LÄNGE: 1446 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1443
(D) SONSTIGE ANGABEN:/product= "Vanillin-Dehydrogenase"
/gene= "vdh"

(xi) SEQUENZBESCHREIBUNG: SEQ ID NO: 27:

ATG TTT CAC GTG CCC CTG CTT ATT GGT GGT AAG CCT TGT TCA GCA TCT      48
Met Phe His Val Pro Leu Leu Ile Gly Gly Lys Pro Cys Ser Ala Ser
245                 250                 255                 260

GAT GAG CGC ACC TTC GAG CGT CGT AGC CCG CTG ACC GGA GAA GTG GTA      96
Asp Glu Arg Thr Phe Glu Arg Arg Ser Pro Leu Thr Gly Glu Val Val
                265                 270                 275

TCG CGC GTC GCT GCT GCC AGT TTG GAA GAT GCG GAC GCC GCA GTG GCC 144
Ser Arg Val Ala Ala Ala Ser Leu Glu Asp Ala Asp Ala Ala Val Ala
280 285 290

GCT GCA CAG GCT GCG TTT CCT GAA TGG GCG GCG CTT GCT CCG AGC GAA 192
Ala Ala Gln Ala Ala Phe Pro Glu Trp Ala Ala Leu Ala Pro Ser Glu
295 300 305

CGC CGT GCC CGA CTG CTG CGA GCG GCG GAT CTT CTA GAG GAC CGT TCT 240
Arg Arg Ala Arg Leu Leu Arg Ala Ala Asp Leu Leu Glu Asp Arg Ser
310 315 320

TCC GAG TTC ACC GCC GCA GCG AGT GAA ACT GGC GCA GCG GGA AAC TGG 288
Ser Glu Phe Thr Ala Ala Ala Ser Glu Thr Gly Ala Ala Gly Asn Trp
325 330 335 340

TAT GGG TTT AAC GTT TAC CTG GCG GCG GGC ATG TTG CGG GAA GCC GCG 336
Tyr Gly Phe Asn Val Tyr Leu Ala Ala Gly Met Leu Arg Glu Ala Ala
345 350 355

GCC ATG ACC ACA CAG ATT CAG GGC GAT GTC ATT CCG TCC AAT GTG CCC 384
Ala Met Thr Thr Gln Ile Gln Gly Asp Val Ile Pro Ser Asn Val Pro
360 365 370

GGT AGC TTT GCC ATG GCG GTT CGA CAG CCA TGT GGC GTG GTG CTC GGT 432
Gly Ser Phe Ala Met Ala Val Arg Gln Pro Cys Gly Val Val Leu Gly
375 380 385

ATT GCG CCT TGG AAT GCT CCG GTA ATC CTT GGC GTA CGG GCT GTT GCG 480
Ile Ala Pro Trp Asn Ala Pro Val Ile Leu Gly Val Arg Ala Val Ala
390 395 400

ATG CCG TTG GCA TGC GGC AAT ACC GTG GTG TTG AAA AGC TCT GAG CTG 528
Met Pro Leu Ala Cys Gly Asn Thr Val Val Leu Lys Ser Ser Glu Leu
405 410 415 420

AGT CCC TTT ACC CAT CGC CTG ATT GGT CAG GTG TTG CAT GAT GCT GGT 576
Ser Pro Phe Thr His Arg Leu Ile Gly Gln Val Leu His Asp Ala Gly
425 430 435

CTG GGG GAT GGC GTG GTG AAT GTC ATC AGC AAT GCC CCG CAA GAC GCT 624
Leu Gly Asp Gly Val Val Asn Val Ile Ser Asn Ala Pro Gln Asp Ala
440 445 450

CCT GCG GTG GTG GAG CGA CTG ATT GCA AAT CCT GCG GTA CGT CGA GTG 672
Pro Ala Val Val Glu Arg Leu Ile Ala Asn Pro Ala Val Arg Arg Val
455 460 465

AAC TTC ACC GGT TCG ACC CAC GTT GGA CGG ATC ATT GGT GAG CTG TCT 720
Asn Phe Thr Gly Ser Thr His Val Gly Arg Ile Ile Gly Glu Leu Ser
470 475 480

GCG CGT CAT CTG AAG CCT GCT GTG CTG GAA TTA GGT GGT AAG GCT CCG 768
Ala Arg His Leu Lys Pro Ala Val Leu Glu Leu Gly Gly Lys Ala Pro
485 490 495 500

TTC TTG GTC TTG GAC GAT GCC GAC CTC GAT GCG GCG GTC GAA GCG GCG 816
Phe Leu Val Leu Asp Asp Ala Asp Leu Asp Ala Ala Val Glu Ala Ala
505 510 515

GCC TTT GGT GCC TAC TTC AAT CAG GGT CAA ATC TGC ATG TCC ACT GAG 864
Ala Phe Gly Ala Tyr Phe Asn Gln Gly Gln Ile Cys Met Ser Thr Glu
520 525 530

CGT CTG ATT GTG ACA GCA GTC GCA GAC GCC TTT GTT GAA AAG CTG GCG 912
Arg Leu Ile Val Thr Ala Val Ala Asp Ala Phe Val Glu Lys Leu Ala
535 540 545

AGG AAG GTC GCC ACA CTG CGT GCT GGC GAT CCT AAT GAT CCG CAA TCG 960
Arg Lys Val Ala Thr Leu Arg Ala Gly Asp Pro Asn Asp Pro Gln Ser
550 555 560

GTC TTG GGT TCG TTG ATT GAT GCC AAT GCA GGT CAA CGC ATC CAG GTT 1008
Val Leu Gly Ser Leu Ile Asp Ala Asn Ala Gly Gln Arg Ile Gln Val
565 570 575 580

CTG GTC GAT GAT GCG CTC GCA AAA GGC GCG CGG CAG GTC GTC GGT GGT 1056
Leu Val Asp Asp Ala Leu Ala Lys Gly Ala Arg Gln Val Val Gly Gly
585 590 595

GGC TTA GAT GGC AGC ATC ATG CAG CCG ATG CTG CTT GAT CAG GTC ACT 1104
Gly Leu Asp Gly Ser Ile Met Gln Pro Met Leu Leu Asp Gln Val Thr
600 605 610

GAA GAG ATG CGG CTC TAC CGT GAG GAG TCC TTT GGC CCT GTT GCC GTT 1152
Glu Glu Met Arg Leu Tyr Arg Glu Glu Ser Phe Gly Pro Val Ala Val
615 620 625

GTC TTG CGC GGC GAT GGT GAT GAA GAA CTG CTG CGT CTT GCC AAC GAT 1200
Val Leu Arg Gly Asp Gly Asp Glu Glu Leu Leu Arg Leu Ala Asn Asp
630 635 640

TCG GAG TTT GGT CTT TCG GCC GCC ATT TTC AGC CGT GAC GTC TCG CGC 1248
Ser Glu Phe Gly Leu Ser Ala Ala Ile Phe Ser Arg Asp Val Ser Arg
645 650 655 660

GCA ATG GAA TTG GCC CAG CGC GTC GAT TCG GGC ATT TGC CAT ATC AAT 1296
Ala Met Glu Leu Ala Gln Arg Val Asp Ser Gly Ile Cys His Ile Asn
665 670 675

GGA CCG ACT GTG CAT GAC GAG GCT CAG ATG CCA TTC GGT GGG GTG AAG 1344
Gly Pro Thr Val His Asp Glu Ala Gln Met Pro Phe Gly Gly Val Lys
680 685 690

TCC AGC GGC TAC GGC AGC TTC GGC AGT CGA GCA TCG ATT GAG CAC TTT 1392
Ser Ser Gly Tyr Gly Ser Phe Gly Ser Arg Ala Ser Ile Glu His Phe
695 700 705

ACC CAG CTG CGC TGG CTG ACC ATT CAG AAT GGC CCG CGG CAC TAT CCA 1440
Thr Gln Leu Arg Trp Leu Thr Ile Gln Asn Gly Pro Arg His Tyr Pro
710 715 720

ATC TAA                                                    1446
Ile
725

(2) ANGABEN ZU SEQ ID NO: 28:

(i) SEQUENZKENNZEICHEN:
(A) LÄNGE: 481 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 28:

Met Phe His Val Pro Leu Leu Ile Gly Gly Lys Pro Cys Ser Ala Ser
1               5                   10                  15

Asp Glu Arg Thr Phe Glu Arg Arg Ser Pro Leu Thr Gly Glu Val Val
            20                  25                  30

Ser Arg Val Ala Ala Ala Ser Leu Glu Asp Ala Asp Ala Ala Val Ala
        35                  40                  45

Ala Ala Gln Ala Ala Phe Pro Glu Trp Ala Ala Leu Ala Pro Ser Glu
    50                  55                  60

Arg Arg Ala Arg Leu Leu Arg Ala Ala Asp Leu Leu Glu Asp Arg Ser
65                  70                  75                  80

Ser Glu Phe Thr Ala Ala Ala Ser Glu Thr Gly Ala Ala Gly Asn Trp
                85                  90                  95

Tyr Gly Phe Asn Val Tyr Leu Ala Ala Gly Met Leu Arg Glu Ala Ala
            100                 105                 110

Ala Met Thr Thr Gln Ile Gln Gly Asp Val Ile Pro Ser Asn Val Pro
        115                 120                 125

Gly Ser Phe Ala Met Ala Val Arg Gln Pro Cys Gly Val Val Leu Gly
    130                 135                 140

Ile Ala Pro Trp Asn Ala Pro Val Ile Leu Gly Val Arg Ala Val Ala
145                 150                 155                 160

Met Pro Leu Ala Cys Gly Asn Thr Val Val Leu Lys Ser Ser Glu Leu
                165                 170                 175

Ser Pro Phe Thr His Arg Leu Ile Gly Gln Val Leu His Asp Ala Gly
            180                 185                 190

Leu Gly Asp Gly Val Val Asn Val Ile Ser Asn Ala Pro Gln Asp Ala
        195                 200                 205

Pro Ala Val Val Glu Arg Leu Ile Ala Asn Pro Ala Val Arg Arg Val

210                 215                 220

Asn Phe Thr Gly Ser Thr His Val Gly Arg Ile Ile Gly Glu Leu Ser
225                 230                 235                 240

Ala Arg His Leu Lys Pro Ala Val Leu Glu Leu Gly Gly Lys Ala Pro
                245                 250                 255

Phe Leu Val Leu Asp Asp Ala Asp Leu Asp Ala Ala Val Glu Ala Ala
            260                 265                 270

Ala Phe Gly Ala Tyr Phe Asn Gln Gly Gln Ile Cys Met Ser Thr Glu
        275                 280                 285

Arg Leu Ile Val Thr Ala Val Ala Asp Ala Phe Val Glu Lys Leu Ala
    290                 295                 300

Arg Lys Val Ala Thr Leu Arg Ala Gly Asp Pro Asn Asp Pro Gln Ser
305                 310                 315                 320

Val Leu Gly Ser Leu Ile Asp Ala Asn Ala Gly Gln Arg Ile Gln Val
                325                 330                 335

Leu Val Asp Asp Ala Leu Ala Lys Gly Ala Arg Gln Val Val Gly Gly
            340                 345                 350

Gly Leu Asp Gly Ser Ile Met Gln Pro Met Leu Leu Asp Gln Val Thr
        355                 360                 365

Glu Glu Met Arg Leu Tyr Arg Glu Glu Ser Phe Gly Pro Val Ala Val
    370                 375                 380

Val Leu Arg Gly Asp Gly Asp Glu Glu Leu Leu Arg Leu Ala Asn Asp
385                 390                 395                 400

Ser Glu Phe Gly Leu Ser Ala Ala Ile Phe Ser Arg Asp Val Ser Arg
                405                 410                 415

Ala Met Glu Leu Ala Gln Arg Val Asp Ser Gly Ile Cys His Ile Asn
            420                 425                 430

Gly Pro Thr Val His Asp Glu Ala Gln Met Pro Phe Gly Gly Val Lys
        435                 440                 445

Ser Ser Gly Tyr Gly Ser Phe Gly Ser Arg Ala Ser Ile Glu His Phe
    450                 455                 460

Thr Gln Leu Arg Trp Leu Thr Ile Gln Asn Gly Pro Arg His Tyr Pro
465                 470                 475                 480

Ile

(2) ANGABEN ZU SEQ ID NO: 29:

(i) SEQUENZKENNZEICHEN:
(A) LÄNGE: 1770 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1767
(D) SONSTIGE ANGABEN:/product=
"Ferulasaeure-CoA-Synthetase"
/gene= "fcs"

(xi) SEQUENZBESCHREIBUNG: SEQ ID NO: 29:

ATG CGT TCT CTC GAG GCG CTT CTT CCC TTC CCG GGT CGA ATT CTT GAG 48
Met Arg Ser Leu Glu Ala Leu Leu Pro Phe Pro Gly Arg Ile Leu Glu
485 490 495

CGT CTC GAG CAT TGG GCT AAG ACC CGT CCA GAA CAA ACC TGC GTT GCT 96
Arg Leu Glu His Trp Ala Lys Thr Arg Pro Glu Gln Thr Cys Val Ala
500 505 510

GCC AGG GCG GCA AAT GGG GAA TGG CGT CGT ATC AGC TAC GCG GAA ATG 144
Ala Arg Ala Ala Asn Gly Glu Trp Arg Arg Ile Ser Tyr Ala Glu Met
515 520 525

TTC CAC AAC GTC CGC GCC ATC GCA CAG AGC TTG CTT CCT TAC GGA CTA 192
Phe His Asn Val Arg Ala Ile Ala Gln Ser Leu Leu Pro Tyr Gly Leu
530 535 540 545

TCG GCA GAG CGT CCG CTG CTT ATC GTC TCT GGA AAT GAC CTG GAA CAT 240
Ser Ala Glu Arg Pro Leu Leu Ile Val Ser Gly Asn Asp Leu Glu His
550 555 560

CTT CAG CTG GCA TTT GGG GCT ATG TAT GCG GGC ATT CCC TAT TGC CCG 288
Leu Gln Leu Ala Phe Gly Ala Met Tyr Ala Gly Ile Pro Tyr Cys Pro
565 570 575

GTG TCT CCT GCT TAT TCA CTG CTG TCG CAA GAT TTG GCG AAG CTG CGT 336
Val Ser Pro Ala Tyr Ser Leu Leu Ser Gln Asp Leu Ala Lys Leu Arg
580 585 590

CAC ATC GTA GGT CTT CTG CAA CCG GGA CTG GTC TTT GCT GCC GAT GCA 384
His Ile Val Gly Leu Leu Gln Pro Gly Leu Val Phe Ala Ala Asp Ala
595 600 605

GCA CCT TTC CAG CGC GCA ATT GAG ACC ATT CTG CCG GAC GAC GTG CCC 432
Ala Pro Phe Gln Arg Ala Ile Glu Thr Ile Leu Pro Asp Asp Val Pro
610 615 620 625

GCA ATC TTC ACT CGA GGC GAA TTG GCC GGG CGG CGC ACG GTG AGT TTT 480
Ala Ile Phe Thr Arg Gly Glu Leu Ala Gly Arg Arg Thr Val Ser Phe
630 635 640

GAC AGC CTG CTG GAG CAG CCT GGT GGG ATT GAG GCA GAT AAT GCC TTT 528
Asp Ser Leu Leu Glu Gln Pro Gly Gly Ile Glu Ala Asp Asn Ala Phe
645 650 655

GCG GCA ACT GGC CCC GAT ACG ATT GCC AAG TTC TTG TTC ACT TCT GGC 576
Ala Ala Thr Gly Pro Asp Thr Ile Ala Lys Phe Leu Phe Thr Ser Gly
660 665 670

TCT ACC AAA CTG CCT AAG GCG GTG CCG ACT ACT CAG CGA ATG CTC TGC 624
Ser Thr Lys Leu Pro Lys Ala Val Pro Thr Thr Gln Arg Met Leu Cys
675 680 685

GCC AAT CAG CAG ATG CTT CTG CAA ACT TTC CCG GTT TTT GGT GAA GAG 672
Ala Asn Gln Gln Met Leu Leu Gln Thr Phe Pro Val Phe Gly Glu Glu
690 695 700 705

CCG CCG GTG CTG GTG GAC TGG TTG CCG TGG AAC CAC ACC TTC GGC GGC 720
Pro Pro Val Leu Val Asp Trp Leu Pro Trp Asn His Thr Phe Gly Gly
710 715 720

AGC CAC AAC ATC GGC ATC GTG TTG TAC AAC GGC GGC ACG TAC TAC CTT 768
Ser His Asn Ile Gly Ile Val Leu Tyr Asn Gly Gly Thr Tyr Tyr Leu
725 730 735

GAC GAC GGT AAA CCA ACC GCC CAA GGG TTC GCC GAG ACG CTT CGC AAC 816
Asp Asp Gly Lys Pro Thr Ala Gln Gly Phe Ala Glu Thr Leu Arg Asn
740 745 750

TTG AGC GAA ATC TCT CCC ACT GCG TAC CTC ACT GTG CCG AAA GGC TGG 864
Leu Ser Glu Ile Ser Pro Thr Ala Tyr Leu Thr Val Pro Lys Gly Trp
755 760 765

GAG GAA TTA GTG GGT GCC CTT GAG CGA GAC AGT ACC CTG CGC GAA CGC 912
Glu Glu Leu Val Gly Ala Leu Glu Arg Asp Ser Thr Leu Arg Glu Arg
770 775 780 785

TTC TTC GCT CGC ATG AAG CTG TTC TTC TTC GCG GCG GCT GGG TTG TCG 960
Phe Phe Ala Arg Met Lys Leu Phe Phe Phe Ala Ala Ala Gly Leu Ser
790 795 800

CAA GGG ATC TGG GAT CGT TTG GAC CGG GTC GCT GAA CAG CAC TGT GGT 1008
Gln Gly Ile Trp Asp Arg Leu Asp Arg Val Ala Glu Gln His Cys Gly
805 810 815

GAG CGC ATT CGC ATG ATG GCG GGT CTG GGC ATG ACG GAG ACT GCT CCT 1056
Glu Arg Ile Arg Met Met Ala Gly Leu Gly Met Thr Glu Thr Ala Pro
820 825 830

TCC TGC ACT TTT ACC ACC GGA CCG CTG TCG ATG GCT GGT TAC ATT GGG 1104
Ser Cys Thr Phe Thr Thr Gly Pro Leu Ser Met Ala Gly Tyr Ile Gly
835 840 845

CTG CCA GCG CCT GGC TGC GAG GTC AAG CTC GTT CCG GTC GAT GGG AAA 1152
Leu Pro Ala Pro Gly Cys Glu Val Lys Leu Val Pro Val Asp Gly Lys
850 855 860 865

TTG GAA GGG CGT TTC CAT GGT CCG CAC GTC ATG AGC GGC TAC TGG CGT 1200
Leu Glu Gly Arg Phe His Gly Pro His Val Met Ser Gly Tyr Trp Arg
870 875 880

GCT CCT GAA CAA AAT GCC CAA GCG TTC GAC GAG GAA GGC TAT TAC TGC 1248
Ala Pro Glu Gln Asn Ala Gln Ala Phe Asp Glu Glu Gly Tyr Tyr Cys
885 890 895

TCC GGT GAT GCC ATC AAA TTG GCA GAT CCT GCC GAT CCT CAG AAA GGT 1296
Ser Gly Asp Ala Ile Lys Leu Ala Asp Pro Ala Asp Pro Gln Lys Gly
900 905 910

CTG ATG TTT GAC GGT CGA ATT GCT GAA GAC TTC AAG CTG TCC TCA GGG 1344
Leu Met Phe Asp Gly Arg Ile Ala Glu Asp Phe Lys Leu Ser Ser Gly
915 920 925

GTA TTT GTC AGC GTT GGG CCA TTG CGC ACG CGG GCG GTT CTG GAA GGC 1392
Val Phe Val Ser Val Gly Pro Leu Arg Thr Arg Ala Val Leu Glu Gly
930 935 940 945

GGC TCT TAC GTC CTG GAC GTA GTG GTT GCT GCT CCT GAT CGT GAA TGC 1440
Gly Ser Tyr Val Leu Asp Val Val Val Ala Ala Pro Asp Arg Glu Cys
950 955 960

CTT GGA TTG CTC GTG TTT CCG CGT CTT CTC GAC TGC CGT GCC TTG TCG 1488
Leu Gly Leu Leu Val Phe Pro Arg Leu Leu Asp Cys Arg Ala Leu Ser
965 970 975

GGG CTA GGA AAA GAG GCG TCG GAC GCC GAG GTG CTT GCC AGT GAG CCG 1536
Gly Leu Gly Lys Glu Ala Ser Asp Ala Glu Val Leu Ala Ser Glu Pro
980 985 990

GTT CGG GCC TGG TTT GCT GAC TGG CTC AAA CGA CTC AAT CGA GAA GCA 1584
Val Arg Ala Trp Phe Ala Asp Trp Leu Lys Arg Leu Asn Arg Glu Ala
995 1000 1005

ACT GGC AAT GCC AGT CGC ATC ATG TGG GTA GGG CTC CTC GAT ACG CCG 1632
Thr Gly Asn Ala Ser Arg Ile Met Trp Val Gly Leu Leu Asp Thr Pro
1010 1015 1020 1025

CCG TCG ATT GAT AAG GGC GAG GTC ACT GAC AAG GGC TCG ATC AAC CAG 1680
Pro Ser Ile Asp Lys Gly Glu Val Thr Asp Lys Gly Ser Ile Asn Gln
1030 1035 1040

CGC GCT GTT TTG CAA TGG CGG TCG GCG AAA GTT GAT GCG CTG TAT CGT 1728
Arg Ala Val Leu Gln Trp Arg Ser Ala Lys Val Asp Ala Leu Tyr Arg
1045 1050 1055

GGT GAA GAT CAA TCC ATG CTG CGT GAC GAG GCC ACA CTG TGA 1770
Gly Glu Asp Gln Ser Met Leu Arg Asp Glu Ala Thr Leu
1060 1065 1070

(2) ANGABEN ZU SEQ ID NO: 30:

(i) SEQUENZKENNZEICHEN:
(A) LÄNGE: 589 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 30:

Met Arg Ser Leu Glu Ala Leu Leu Pro Phe Pro Gly Arg Ile Leu Glu
1               5                   10                  15

Arg Leu Glu His Trp Ala Lys Thr Arg Pro Glu Gln Thr Cys Val Ala
            20                  25                  30

Ala Arg Ala Ala Asn Gly Glu Trp Arg Arg Ile Ser Tyr Ala Glu Met
        35                  40                  45

Phe His Asn Val Arg Ala Ile Ala Gln Ser Leu Leu Pro Tyr Gly Leu
    50                  55                  60

Ser Ala Glu Arg Pro Leu Leu Ile Val Ser Gly Asn Asp Leu Glu His
65                  70                  75                  80

Leu Gln Leu Ala Phe Gly Ala Met Tyr Ala Gly Ile Pro Tyr Cys Pro
                85                  90                  95

Val Ser Pro Ala Tyr Ser Leu Leu Ser Gln Asp Leu Ala Lys Leu Arg
            100                 105                 110

His Ile Val Gly Leu Leu Gln Pro Gly Leu Val Phe Ala Ala Asp Ala
        115                 120                 125

Ala Pro Phe Gln Arg Ala Ile Glu Thr Ile Leu Pro Asp Asp Val Pro
    130                 135                 140

Ala Ile Phe Thr Arg Gly Glu Leu Ala Gly Arg Arg Thr Val Ser Phe
145                 150                 155                 160

Asp Ser Leu Leu Glu Gln Pro Gly Gly Ile Glu Ala Asp Asn Ala Phe
                165                 170                 175

Ala Ala Thr Gly Pro Asp Thr Ile Ala Lys Phe Leu Phe Thr Ser Gly
            180                 185                 190

Ser Thr Lys Leu Pro Lys Ala Val Pro Thr Thr Gln Arg Met Leu Cys
        195                 200                 205

Ala Asn Gln Gln Met Leu Leu Gln Thr Phe Pro Val Phe Gly Glu Glu

210                 215                 220

Pro Pro Val Leu Val Asp Trp Leu Pro Trp Asn His Thr Phe Gly Gly
225                 230                 235                 240

Ser His Asn Ile Gly Ile Val Leu Tyr Asn Gly Gly Thr Tyr Tyr Leu
                245                 250                 255

Asp Asp Gly Lys Pro Thr Ala Gln Gly Phe Ala Glu Thr Leu Arg Asn
            260                 265                 270

Leu Ser Glu Ile Ser Pro Thr Ala Tyr Leu Thr Val Pro Lys Gly Trp
        275                 280                 285

Glu Glu Leu Val Gly Ala Leu Glu Arg Asp Ser Thr Leu Arg Glu Arg
    290                 295                 300

Phe Phe Ala Arg Met Lys Leu Phe Phe Phe Ala Ala Ala Gly Leu Ser
305                 310                 315                 320

Gln Gly Ile Trp Asp Arg Leu Asp Arg Val Ala Glu Gln His Cys Gly
                325                 330                 335

Glu Arg Ile Arg Met Met Ala Gly Leu Gly Met Thr Glu Thr Ala Pro
            340                 345                 350

Ser Cys Thr Phe Thr Thr Gly Pro Leu Ser Met Ala Gly Tyr Ile Gly
        355                 360                 365

Leu Pro Ala Pro Gly Cys Glu Val Lys Leu Val Pro Val Asp Gly Lys
    370                 375                 380

Leu Glu Gly Arg Phe His Gly Pro His Val Met Ser Gly Tyr Trp Arg
385                 390                 395                 400

Ala Pro Glu Gln Asn Ala Gln Ala Phe Asp Glu Glu Gly Tyr Tyr Cys
                405                 410                 415

Ser Gly Asp Ala Ile Lys Leu Ala Asp Pro Ala Asp Pro Gln Lys Gly
            420                 425                 430

Leu Met Phe Asp Gly Arg Ile Ala Glu Asp Phe Lys Leu Ser Ser Gly
        435                 440                 445

Val Phe Val Ser Val Gly Pro Leu Arg Thr Arg Ala Val Leu Glu Gly
    450                 455                 460

Gly Ser Tyr Val Leu Asp Val Val Val Ala Ala Pro Asp Arg Glu Cys
465                 470                 475                 480

Leu Gly Leu Leu Val Phe Pro Arg Leu Leu Asp Cys Arg Ala Leu Ser
                485                 490                 495

Gly Leu Gly Lys Glu Ala Ser Asp Ala Glu Val Leu Ala Ser Glu Pro
            500                 505                 510

Val Arg Ala Trp Phe Ala Asp Trp Leu Lys Arg Leu Asn Arg Glu Ala
        515                 520                 525

Thr Gly Asn Ala Ser Arg Ile Met Trp Val Gly Leu Leu Asp Thr Pro
    530                 535                 540

Pro Ser Ile Asp Lys Gly Glu Val Thr Asp Lys Gly Ser Ile Asn Gln
545                 550                 555                 560

Arg Ala Val Leu Gln Trp Arg Ser Ala Lys Val Asp Ala Leu Tyr Arg
                565                 570                 575

Gly Glu Asp Gln Ser Met Leu Arg Asp Glu Ala Thr Leu
            580                 585

(2) ANGABEN ZU SEQ ID NO: 31:

(i) SEQUENZKENNZEICHEN:
(A) LÄNGE: 1296 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1293
(D) SONSTIGE ANGABEN:/product= "beta-Ketothiolase"
/gene= "aat"

(xi) SEQUENZBESCHREIBUNG: SEQ ID NO: 31:

ATG AGT TGG TCA GGG GGG GCT TAC TCG GCG TTT TCC GAC ACT GCG TTG 48
Met Ser Trp Ser Gly Gly Ala Tyr Ser Ala Phe Ser Asp Thr Ala Leu
590 595 600 605

GTT GCG GCA GTG CGC ACC CCC TGG ATT GAT TGC GGG GGT GCC CTG TCG 96
Val Ala Ala Val Arg Thr Pro Trp Ile Asp Cys Gly Gly Ala Leu Ser
610 615 620

CTG GTG TCG CCT ATC GAC TTA GGG GTA AAG GTC GCT CGC GAA GTT CTG 144
Leu Val Ser Pro Ile Asp Leu Gly Val Lys Val Ala Arg Glu Val Leu
625 630 635

ATG CGT GCG TCG CTT GAA CCA CAA ATG GTC GAT AGC GTA CTC GCA GGC 192
Met Arg Ala Ser Leu Glu Pro Gln Met Val Asp Ser Val Leu Ala Gly
640 645 650

TCT ATG GCT CAA GCA AGC TTT GAT GCT TAC CTG CTC CCG CGG CAC ATT 240
Ser Met Ala Gln Ala Ser Phe Asp Ala Tyr Leu Leu Pro Arg His Ile
655 660 665

GGC TTG TAC AGC GGT GTT CCC AAG TCG GTT CCG GCC TTG GGG GTG CAG 288
Gly Leu Tyr Ser Gly Val Pro Lys Ser Val Pro Ala Leu Gly Val Gln
670 675 680 685

CGC ATT TGC GGC ACA GGC TTC GAA CTG CTT CGG CAG GCC GGC GAG CAG 336
Arg Ile Cys Gly Thr Gly Phe Glu Leu Leu Arg Gln Ala Gly Glu Gln
690 695 700

ATT TCC CAA GGC GCT GAT CAC GTG CTG TGT GTC GCG GCA GAG TCC ATG 384
Ile Ser Gln Gly Ala Asp His Val Leu Cys Val Ala Ala Glu Ser Met
705 710 715

TCG CGT AAC CCC ATC GCG TCG TAT ACA CAC CGG GGC GGG TTC CGC CTC 432
Ser Arg Asn Pro Ile Ala Ser Tyr Thr His Arg Gly Gly Phe Arg Leu
720 725 730

GGT GCG CCC GTT GAG TTC AAG GAT TTT TTG TGG GAG GCA TTG TTT GAT 480
Gly Ala Pro Val Glu Phe Lys Asp Phe Leu Trp Glu Ala Leu Phe Asp
735 740 745

CCT GCT CCA GGA CTC GAC ATG ATC GCT ACC GCA GAA AAC CTG GCG CGC 528
Pro Ala Pro Gly Leu Asp Met Ile Ala Thr Ala Glu Asn Leu Ala Arg
750 755 760 765

CTG TAC GGA ATC ACC AGG GGA GAA GCT AAT TCC TAC GCG GTA AGC AGC 576
Leu Tyr Gly Ile Thr Arg Gly Glu Ala Asn Ser Tyr Ala Val Ser Ser
770 775 780

TTC GAG CGC GCA TTG AGG GCG CAA GAG GAG AAA TGG ATT GAC CAA GAG 624
Phe Glu Arg Ala Leu Arg Ala Gln Glu Glu Lys Trp Ile Asp Gln Glu
785 790 795

ATC GTG GCT GTT ACG GAT GAA CAG TTC GAT TTA GAG GGC TAC AAC AGT 672
Ile Val Ala Val Thr Asp Glu Gln Phe Asp Leu Glu Gly Tyr Asn Ser
800 805 810

CGA GCA ATT GAA CTG CCT CGG AAG GCA AAA TTG TTG ATC GTG ACA GTC 720
Arg Ala Ile Glu Leu Pro Arg Lys Ala Lys Leu Leu Ile Val Thr Val
815 820 825

ATC CGC GGC CTA GCA GTC TTT GAA GCC CTT TCC CGA TTG AAG CCT GTT 768
Ile Arg Gly Leu Ala Val Phe Glu Ala Leu Ser Arg Leu Lys Pro Val
830 835 840 845

CAT TCT GGC GGG GTG CAG ACT GCG GGC AAC AGC TGT GCC GTA GTG GAC 816
His Ser Gly Gly Val Gln Thr Ala Gly Asn Ser Cys Ala Val Val Asp
850 855 860

GGC GCC GCG GCG GCT TTG GTG GCT CGA GAG TCG TCT GCG ACA CAG CCG 864
Gly Ala Ala Ala Ala Leu Val Ala Arg Glu Ser Ser Ala Thr Gln Pro
865 870 875

GTC TTG GCT AGG ATA CTG GCT ACC TCC GTA GTC GGG ATC GAG CCC GAG 912
Val Leu Ala Arg Ile Leu Ala Thr Ser Val Val Gly Ile Glu Pro Glu
880 885 890

CAT ATG GGG CTC GGC CCT GCG CCC GCG ATT CGC CTG CTG CTT GCG CGT 960
His Met Gly Leu Gly Pro Ala Pro Ala Ile Arg Leu Leu Leu Ala Arg
895 900 905

AGT GAT CTT AGT TTG AGG GAT ATC GAC CTC TTT GAG ATA AAC GAG GCG 1008
Ser Asp Leu Ser Leu Arg Asp Ile Asp Leu Phe Glu Ile Asn Glu Ala
910 915 920 925

CAG GCC GCC CAA GTT CTA GCG GTA CAG CAT GAA TTG GGT ATT GAG CAC 1056
Gln Ala Ala Gln Val Leu Ala Val Gln His Glu Leu Gly Ile Glu His
930 935 940

TCA AAA CTT AAT ATT TGG GGC GGG GCC ATT GCA CTT GGA CAC CCG CTT 1104
Ser Lys Leu Asn Ile Trp Gly Gly Ala Ile Ala Leu Gly His Pro Leu
945 950 955

GCC GCG ACC GGA TTG CGT CTC TGC ATG ACC CTC GCT CAC CAA TTG CAA 1152
Ala Ala Thr Gly Leu Arg Leu Cys Met Thr Leu Ala His Gln Leu Gln
960 965 970

GCT AAT AAC TTT CGA TAT GGA ATT GCC TCG GCA TGC ATT GGT GGG GGA 1200
Ala Asn Asn Phe Arg Tyr Gly Ile Ala Ser Ala Cys Ile Gly Gly Gly
975 980 985

CAG GGG ATG GCG GTT CTT TTA GAG AAT CCC CAC TTC GGT TCG TCC TCT 1248
Gln Gly Met Ala Val Leu Leu Glu Asn Pro His Phe Gly Ser Ser Ser
990 995 1000 1005

GCA CGA AGT TCG ATG ATT AAC AGA GTT GAC CAC TAT CCA CTG AGC 1293
Ala Arg Ser Ser Met Ile Asn Arg Val Asp His Tyr Pro Leu Ser
1010 1015 1020

TAA 1296

(2) ANGABEN ZU SEQ ID NO: 32:

(i) SEQUENZKENNZEICHEN:
(A) LÄNGE: 431 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 32:

Met Ser Trp Ser Gly Gly Ala Tyr Ser Ala Phe Ser Asp Thr Ala Leu
1               5                   10                  15

Val Ala Ala Val Arg Thr Pro Trp Ile Asp Cys Gly Gly Ala Leu Ser
            20                  25                  30

Leu Val Ser Pro Ile Asp Leu Gly Val Lys Val Ala Arg Glu Val Leu
        35                  40                  45

Met Arg Ala Ser Leu Glu Pro Gln Met Val Asp Ser Val Leu Ala Gly
    50                  55                  60

Ser Met Ala Gln Ala Ser Phe Asp Ala Tyr Leu Leu Pro Arg His Ile
65                  70                  75                  80

Gly Leu Tyr Ser Gly Val Pro Lys Ser Val Pro Ala Leu Gly Val Gln
                85                  90                  95

Arg Ile Cys Gly Thr Gly Phe Glu Leu Leu Arg Gln Ala Gly Glu Gln
            100                 105                 110

Ile Ser Gln Gly Ala Asp His Val Leu Cys Val Ala Ala Glu Ser Met
        115                 120                 125

Ser Arg Asn Pro Ile Ala Ser Tyr Thr His Arg Gly Gly Phe Arg Leu
    130                 135                 140

Gly Ala Pro Val Glu Phe Lys Asp Phe Leu Trp Glu Ala Leu Phe Asp
145                 150                 155                 160

Pro Ala Pro Gly Leu Asp Met Ile Ala Thr Ala Glu Asn Leu Ala Arg
                165                 170                 175

Leu Tyr Gly Ile Thr Arg Gly Glu Ala Asn Ser Tyr Ala Val Ser Ser
            180                 185                 190

Phe Glu Arg Ala Leu Arg Ala Gln Glu Glu Lys Trp Ile Asp Gln Glu
        195                 200                 205

Ile Val Ala Val Thr Asp Glu Gln Phe Asp Leu Glu Gly Tyr Asn Ser
    210                 215                 220

Arg Ala Ile Glu Leu Pro Arg Lys Ala Lys Leu Leu Ile Val Thr Val
225                 230                 235                 240

Ile Arg Gly Leu Ala Val Phe Glu Ala Leu Ser Arg Leu Lys Pro Val
                245                 250                 255

His Ser Gly Gly Val Gln Thr Ala Gly Asn Ser Cys Ala Val Val Asp
            260                 265                 270

Gly Ala Ala Ala Ala Leu Val Ala Arg Glu Ser Ser Ala Thr Gln Pro
        275                 280                 285

Val Leu Ala Arg Ile Leu Ala Thr Ser Val Val Gly Ile Glu Pro Glu
    290                 295                 300

His Met Gly Leu Gly Pro Ala Pro Ala Ile Arg Leu Leu Leu Ala Arg
305                 310                 315                 320

Ser Asp Leu Ser Leu Arg Asp Ile Asp Leu Phe Glu Ile Asn Glu Ala

325                 330                 335

Gln Ala Ala Gln Val Leu Ala Val Gln His Glu Leu Gly Ile Glu His
        340                 345                 350

Ser Lys Leu Asn Ile Trp Gly Gly Ala Ile Ala Leu Gly His Pro Leu
    355                 360                 365

Ala Ala Thr Gly Leu Arg Leu Cys Met Thr Leu Ala His Gln Leu Gln
    370                 375                 380

Ala Asn Asn Phe Arg Tyr Gly Ile Ala Ser Ala Cys Ile Gly Gly Gly
385                 390                 395                 400

Gln Gly Met Ala Val Leu Leu Glu Asn Pro His Phe Gly Ser Ser Ser
                405                 410                 415

Ala Arg Ser Ser Met Ile Asn Arg Val Asp His Tyr Pro Leu Ser
            420                 425                 430

(2) ANGABEN ZU SEQ ID NO: 33:

(i) SEQUENZKENNZEICHEN:
(A) LÄNGE: 1596 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1593
(D) SONSTIGE ANGABEN:/product= "Chemotaxis-Protein"
/gene= "mac"

(xi) SEQUENZBESCHREIBUNG: SEQ ID NO: 33:

ATG ATT AGT TTC GCT CGT ATG GCA GAA AGT TTA GGA GTC CAG GCT AAA      48
Met Ile Ser Phe Ala Arg Met Ala Glu Ser Leu Gly Val Gln Ala Lys
            435                 440                 445

CTT GCC CTT GCC TTC GCA CTC GTA TTA TGT GTC GGG CTG ATT GTT ACC      96
Leu Ala Leu Ala Phe Ala Leu Val Leu Cys Val Gly Leu Ile Val Thr
        450                 455                 460

GGC ACG GGT TTC TAC AGT GTA CAT ACC TTG TCA GGG TTG GTG GAA AAG     144
Gly Thr Gly Phe Tyr Ser Val His Thr Leu Ser Gly Leu Val Glu Lys
    465                 470                 475

AGC GCG ATA GCT GGT GAG TTG CGG GCG AAA ATT CAG GAA CTG AAG GTT 192
Ser Ala Ile Ala Gly Glu Leu Arg Ala Lys Ile Gln Glu Leu Lys Val
480 485 490 495

CTG GAG CAG CGC GCC TTA TTC ATC GCC GAT GAA GGG TCG CTG AAG CAG 240
Leu Glu Gln Arg Ala Leu Phe Ile Ala Asp Glu Gly Ser Leu Lys Gln
500 505 510

CGC TCG ATC CTC CTA AGT CAG GTG ATA GCT GAA GTT AAT GAT GCT ATA 288
Arg Ser Ile Leu Leu Ser Gln Val Ile Ala Glu Val Asn Asp Ala Ile
515 520 525

GAT ATT TTT GAC TTT CAG CGC GGA CGA TCT GAG TTA CTT AAA TTC GCT 336
Asp Ile Phe Asp Phe Gln Arg Gly Arg Ser Glu Leu Leu Lys Phe Ala
530 535 540

GCT TCT TCG CGC GAA GCA AGT TAC TCC ATT GAG GTC GGT AGT AAC GCT 384
Ala Ser Ser Arg Glu Ala Ser Tyr Ser Ile Glu Val Gly Ser Asn Ala
545 550 555

GCG GCC GAT AAG TTG CAG TCG GGC GAA CCA AGT GAC GCA TTG ATG GTT 432
Ala Ala Asp Lys Leu Gln Ser Gly Glu Pro Ser Asp Ala Leu Met Val
560 565 570 575

GCC GAT AAA AAG CTG AAT GTT GAG TAT GAG CAA TTG AGT TCT GCT GTG 480
Ala Asp Lys Lys Leu Asn Val Glu Tyr Glu Gln Leu Ser Ser Ala Val
580 585 590

AAT GCA CTG ATG GGG CAT TTA ATT GAG GAT CAG AAT GAA AAA GTT CCA 528
Asn Ala Leu Met Gly His Leu Ile Glu Asp Gln Asn Glu Lys Val Pro
595 600 605

CTA ATC TAC TAT ATG CTT GGC GGC GTA ACT TTG TTT ACG ATG CTC ATG 576
Leu Ile Tyr Tyr Met Leu Gly Gly Val Thr Leu Phe Thr Met Leu Met
610 615 620

AGT GCT TAT TCG GTC TGG TTC ATT TCG CGT CAG TTA GTT CCG CCA TTA 624
Ser Ala Tyr Ser Val Trp Phe Ile Ser Arg Gln Leu Val Pro Pro Leu
625 630 635

AAG TCG ACG GTG CAG CTT GCC GAG CGG ATT GCA TCA GGC GAC TTG GCT 672
Lys Ser Thr Val Gln Leu Ala Glu Arg Ile Ala Ser Gly Asp Leu Ala
640 645 650 655

GAT GTC GGG GAC AGC AGG CGC AAG GAT GAA ATC GGT CAG TTG CAA AGT 720
Asp Val Gly Asp Ser Arg Arg Lys Asp Glu Ile Gly Gln Leu Gln Ser
660 665 670

GCA ACT AGG CGG ATG GCG ATT GGA CTG CGT AAT CTG GTC GGT GAT ATT 768
Ala Thr Arg Arg Met Ala Ile Gly Leu Arg Asn Leu Val Gly Asp Ile
675 680 685

GGT CAA AGT CGT GCG CAA CTG GTT TCA TCG TCC AGC GAC CTT TCG GCC 816
Gly Gln Ser Arg Ala Gln Leu Val Ser Ser Ser Ser Asp Leu Ser Ala
690 695 700

ATC TGT GCT CAG GCT CAG ATT GAT GTC GAG TGC CAG AAG CTT TCG GTC 864
Ile Cys Ala Gln Ala Gln Ile Asp Val Glu Cys Gln Lys Leu Ser Val
705 710 715

GCC CAG GTC TCT ACC GCC GTG AAC GAG TTG GTT GAA ACC GTC CAG GCA 912
Ala Gln Val Ser Thr Ala Val Asn Glu Leu Val Glu Thr Val Gln Ala
720 725 730 735

ATA GCA AAA AGC ACC GAA GAG GCA GCA ACA GTC GCC GTC TTG GCC GAT 960
Ile Ala Lys Ser Thr Glu Glu Ala Ala Thr Val Ala Val Leu Ala Asp
740 745 750

GAA AAG GCA CGC GGT GGT GAA AGT GTC GTT AAC AAG GCC GTT GAT TTC 1008
Glu Lys Ala Arg Gly Gly Glu Ser Val Val Asn Lys Ala Val Asp Phe
755 760 765

ATT GAG CAC CTC TCC GGA GAT ATG GCG GAA CTG GGA GAC GCA ATG GAG 1056
Ile Glu His Leu Ser Gly Asp Met Ala Glu Leu Gly Asp Ala Met Glu
770 775 780

CGG CTT CAG AAC GAC AGT GCG CAG ATC AAT AAG GTA GTA GAC GTC ATT 1104
Arg Leu Gln Asn Asp Ser Ala Gln Ile Asn Lys Val Val Asp Val Ile
785 790 795

AAG GCT GTG GCG GAG CAG ACC AAT CTG CTA GCC CTG AAT GCG GCG ATA 1152
Lys Ala Val Ala Glu Gln Thr Asn Leu Leu Ala Leu Asn Ala Ala Ile
800 805 810 815

GAG GCG GCC CGT GCA GGA GAG CAG GGC AGG GGC TTT GCG GTC GTG GCG 1200
Glu Ala Ala Arg Ala Gly Glu Gln Gly Arg Gly Phe Ala Val Val Ala
820 825 830

GAT GAG GTT CGT GCT TTG GCG ATG CGC ACC CAA CAA TCG ACC AAA GAA 1248
Asp Glu Val Arg Ala Leu Ala Met Arg Thr Gln Gln Ser Thr Lys Glu
835 840 845

ATT GAG AGG CTA GTG GTT TCA TTG CAG CAG GGA AGT GAA GCT GCG GGC 1296
Ile Glu Arg Leu Val Val Ser Leu Gln Gln Gly Ser Glu Ala Ala Gly
850 855 860

GAG TTG ATG CGG CGT GGC AAG GTC CGG ACG CAT GAC GTC GTT GGA TTG 1344
Glu Leu Met Arg Arg Gly Lys Val Arg Thr His Asp Val Val Gly Leu
865 870 875

GCC CAG CAA GCC GCG CGC CGC GCT ACT CGA AAT TAC CCA GCT GTC GCC 1392
Ala Gln Gln Ala Ala Arg Arg Ala Thr Arg Asn Tyr Pro Ala Val Ala
880 885 890 895

GGC ATC CAA GCG ATG AAC TAT CAG ATC GCC GCT GGA GCA GAG CAG CAA 1440
Gly Ile Gln Ala Met Asn Tyr Gln Ile Ala Ala Gly Ala Glu Gln Gln
900 905 910

GGG GCT GCT GTG GTT CAA ATC AAC CAG AAT ATG CTT GAA GTG CAT AAG 1488
Gly Ala Ala Val Val Gln Ile Asn Gln Asn Met Leu Glu Val His Lys
915 920 925

ATG GCT GAC GAG TCC GCC ATT AAA GCG GGA CAG ACC ATG AAG TCA TCG 1536
Met Ala Asp Glu Ser Ala Ile Lys Ala Gly Gln Thr Met Lys Ser Ser
930 935 940

AAG GAG CTT GCT CAC CTC GGC AGT GCG CTA CAA AAA TCC GTT GAT CGA 1584
Lys Glu Leu Ala His Leu Gly Ser Ala Leu Gln Lys Ser Val Asp Arg
945 950 955

TTC CAG CTG TAG 1596
Phe Gln Leu
960

(2) ANGABEN ZU SEQ ID NO: 34:

(i) SEQUENZKENNZEICHEN:
(A) LÄNGE: 531 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 34:

Met Ile Ser Phe Ala Arg Met Ala Glu Ser Leu Gly Val Gln Ala Lys
1               5                   10                  15

Leu Ala Leu Ala Phe Ala Leu Val Leu Cys Val Gly Leu Ile Val Thr
            20                  25                  30

Gly Thr Gly Phe Tyr Ser Val His Thr Leu Ser Gly Leu Val Glu Lys
        35                  40                  45

Ser Ala Ile Ala Gly Glu Leu Arg Ala Lys Ile Gln Glu Leu Lys Val
    50                  55                  60

Leu Glu Gln Arg Ala Leu Phe Ile Ala Asp Glu Gly Ser Leu Lys Gln
65                  70                  75                  80

Arg Ser Ile Leu Leu Ser Gln Val Ile Ala Glu Val Asn Asp Ala Ile
                85                  90                  95

Asp Ile Phe Asp Phe Gln Arg Gly Arg Ser Glu Leu Leu Lys Phe Ala
            100                 105                 110

Ala Ser Ser Arg Glu Ala Ser Tyr Ser Ile Glu Val Gly Ser Asn Ala
        115                 120                 125

Ala Ala Asp Lys Leu Gln Ser Gly Glu Pro Ser Asp Ala Leu Met Val
    130                 135                 140

Ala Asp Lys Lys Leu Asn Val Glu Tyr Glu Gln Leu Ser Ser Ala Val
145                 150                 155                 160

Asn Ala Leu Met Gly His Leu Ile Glu Asp Gln Asn Glu Lys Val Pro
                165                 170                 175

Leu Ile Tyr Tyr Met Leu Gly Gly Val Thr Leu Phe Thr Met Leu Met
            180                 185                 190

Ser Ala Tyr Ser Val Trp Phe Ile Ser Arg Gln Leu Val Pro Pro Leu
        195                 200                 205

Lys Ser Thr Val Gln Leu Ala Glu Arg Ile Ala Ser Gly Asp Leu Ala
    210                 215                 220

Asp Val Gly Asp Ser Arg Arg Lys Asp Glu Ile Gly Gln Leu Gln Ser
225                 230                 235                 240

Ala Thr Arg Arg Met Ala Ile Gly Leu Arg Asn Leu Val Gly Asp Ile
                245                 250                 255

Gly Gln Ser Arg Ala Gln Leu Val Ser Ser Ser Ser Asp Leu Ser Ala
            260                 265                 270

Ile Cys Ala Gln Ala Gln Ile Asp Val Glu Cys Gln Lys Leu Ser Val
        275                 280                 285

Ala Gln Val Ser Thr Ala Val Asn Glu Leu Val Glu Thr Val Gln Ala
    290                 295                 300

Ile Ala Lys Ser Thr Glu Glu Ala Ala Thr Val Ala Val Leu Ala Asp
305                 310                 315                 320

Glu Lys Ala Arg Gly Gly Glu Ser Val Val Asn Lys Ala Val Asp Phe
                325                 330                 335

Ile Glu His Leu Ser Gly Asp Met Ala Glu Leu Gly Asp Ala Met Glu
            340                 345                 350

Arg Leu Gln Asn Asp Ser Ala Gln Ile Asn Lys Val Val Asp Val Ile
        355                 360                 365

Lys Ala Val Ala Glu Gln Thr Asn Leu Leu Ala Leu Asn Ala Ala Ile
    370                 375                 380

Glu Ala Ala Arg Ala Gly Glu Gln Gly Arg Gly Phe Ala Val Val Ala
385                 390                 395                 400

Asp Glu Val Arg Ala Leu Ala Met Arg Thr Gln Gln Ser Thr Lys Glu
                405                 410                 415

Ile Glu Arg Leu Val Val Ser Leu Gln Gln Gly Ser Glu Ala Ala Gly
            420                 425                 430

Glu Leu Met Arg Arg Gly Lys Val Arg Thr His Asp Val Val Gly Leu
        435                 440                 445

Ala Gln Gln Ala Ala Arg Arg Ala Thr Arg Asn Tyr Pro Ala Val Ala
    450                 455                 460

Gly Ile Gln Ala Met Asn Tyr Gln Ile Ala Ala Gly Ala Glu Gln Gln

465                 470                 475                 480

Gly Ala Ala Val Val Gln Ile Asn Gln Asn Met Leu Glu Val His Lys
                485                 490                 495

Met Ala Asp Glu Ser Ala Ile Lys Ala Gly Gln Thr Met Lys Ser Ser
            500                 505                 510

Lys Glu Leu Ala His Leu Gly Ser Ala Leu Gln Lys Ser Val Asp Arg
        515                 520                 525

Phe Gln Leu
    530

(2) ANGABEN ZU SEQ ID NO: 35:

(i) SEQUENZKENNZEICHEN:
(A) LÄNGE: 411 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:complement (4..411)
(D) SONSTIGE ANGABEN:/product=
"Transkriptions-Regulator-Protein"
/gene= "trp"

(xi) SEQUENZBESCHREIBUNG: SEQ ID NO: 35:

CTAGCCTAAC TGTTGCGCTT CAGGCTCCGC ATGGATCTTG TGCAGCAGCA ATAGCAATTG 60

TTCACGTTCG TCATCACTCA GCATCGACGT CGCGTCTTGG TCGCTCTGTA CCACGATCTT 120

CTTCAGCTCT TTGAGCTGCG TCTCCCCAGC TTTGCTGAGA AATATCCCAT AGGAACGCTT 180

GTCCGGCTTG CAGCGCACGC GCACAGCAAG GCCGAGCTTC TCGAGCTTGT TCAGCAAGGG 240

AACCAGTTGT GGTGGTTCGA TTGCGAGCAT CCGCGCTAGG TCAGCCTGCA TAAGCCCAGG 300

GCTCGCTTCG ATGATTAGAA GTGCCGACAG CTGCGCCGGG CGTAGGTCAT ATGGCGTCAG 360

GGCTTCAATC AGGCCCTGAG CGAGCTTCAG CTGTGAGCCG GCGTAAGGCA T 411

(2) ANGABEN ZU SEQ ID NO: 36:

(i) SEQUENZKENNZEICHEN:
(A) LÄNGE: 136 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 36:

Met Pro Tyr Ala Gly Ser Gln Leu Lys Leu Ala Gln Gly Leu Ile Glu
1               5                   10                  15

Ala Leu Thr Pro Tyr Asp Leu Arg Pro Ala Gln Leu Ser Ala Leu Leu
            20                  25                  30

Ile Ile Glu Ala Ser Pro Gly Leu Met Gln Ala Asp Leu Ala Arg Met
        35                  40                  45

Leu Ala Ile Glu Pro Pro Gln Leu Val Pro Leu Leu Asn Lys Leu Glu
    50                  55                  60

Lys Leu Gly Leu Ala Val Arg Val Arg Cys Lys Pro Asp Lys Arg Ser
65                  70                  75                  80

Tyr Gly Ile Phe Leu Ser Lys Ala Gly Glu Thr Gln Leu Lys Glu Leu
                85                  90                  95

Lys Lys Ile Val Val Gln Ser Asp Gln Asp Ala Thr Ser Met Leu Ser
            100                 105                 110

Asp Asp Glu Arg Glu Gln Leu Leu Leu Leu Leu His Lys Ile His Ala
        115                 120                 125

Glu Pro Glu Ala Gln Gln Leu Gly
    130                 135

(2) ANGABEN ZU SEQ ID NO: 37:

(i) SEQUENZKENNZEICHEN:
(A) LÄNGE: 1446 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:1..1443
(D) SONSTIGE ANGABEN:/product=
"Coniferylaldehyd-Dehydrogenase"

/gene= "caldh"

(xi) SEQUENZBESCHREIBUNG: SEQ ID NO: 37:

ATG AGC ATT CTT GGT TTG AAT GGT GCC CCG GTC GGA GCT GAG CAG CTG 48
Met Ser Ile Leu Gly Leu Asn Gly Ala Pro Val Gly Ala Glu Gln Leu
140 145 150

GGC TCG GCT CTT GAT CGC ATG AAG AAG GCG CAC CTG GAG CAG GGG CCT 96
Gly Ser Ala Leu Asp Arg Met Lys Lys Ala His Leu Glu Gln Gly Pro
155 160 165

GCA AAC TTG GAG CTG CGT CTG AGT AGG CTG GAT CGT GCG ATT GCA ATG 144
Ala Asn Leu Glu Leu Arg Leu Ser Arg Leu Asp Arg Ala Ile Ala Met
170 175 180

CTT CTG GAA AAT CGT GAA GCA ATT GCC GAC GCG GTT TCT GCT GAC TTT 192
Leu Leu Glu Asn Arg Glu Ala Ile Ala Asp Ala Val Ser Ala Asp Phe
185 190 195 200

GGC AAT CGC AGC CGT GAG CAA ACA CTG CTT TGC GAC ATT GCT GGC TCG 240
Gly Asn Arg Ser Arg Glu Gln Thr Leu Leu Cys Asp Ile Ala Gly Ser
205 210 215

GTG GCA AGC CTG AAG GAT AGC CGC GAG CAC GTG GCC AAA TGG ATG GAG 288
Val Ala Ser Leu Lys Asp Ser Arg Glu His Val Ala Lys Trp Met Glu
220 225 230

CCC GAA CAT CAC AAG GCG ATG TTT CCA GGG GCG GAG GCA CGC GTT GAG 336
Pro Glu His His Lys Ala Met Phe Pro Gly Ala Glu Ala Arg Val Glu
235 240 245

TTT CAG CCG CTG GGT GTC GTT GGG GTC ATT AGT CCC TGG AAC TTC CCT 384
Phe Gln Pro Leu Gly Val Val Gly Val Ile Ser Pro Trp Asn Phe Pro
250 255 260

ATC GTA CTG GCC TTT GGG CCG CTG GCC GGC ATA TTC GCA GCA GGT AAT 432
Ile Val Leu Ala Phe Gly Pro Leu Ala Gly Ile Phe Ala Ala Gly Asn
265 270 275 280

CGC GCC ATG CTC AAG CCG TCC GAG CTT ACC CCG CGG ACT TCT GCC CTG 480
Arg Ala Met Leu Lys Pro Ser Glu Leu Thr Pro Arg Thr Ser Ala Leu
285 290 295

CTT GCG GAG CTA ATT GCT CGT TAC TTC GAT GAA ACT GAG CTG ACT ACA 528
Leu Ala Glu Leu Ile Ala Arg Tyr Phe Asp Glu Thr Glu Leu Thr Thr
300 305 310

GTG CTG GGC GAC GCT GAA GTC GGT GCG CTG TTC AGT GCT CAG CCT TTC 576
Val Leu Gly Asp Ala Glu Val Gly Ala Leu Phe Ser Ala Gln Pro Phe
315 320 325

GAT CAT CTG ATC TTC ACC GGC GGC ACT GCC GTG GCC AAG CAC ATC ATG 624
Asp His Leu Ile Phe Thr Gly Gly Thr Ala Val Ala Lys His Ile Met

330                 335                 340

CGT GCC GCG GCG GAT AAC CTA GTG CCC GTT ACC CTG GAA TTG GGT GGC        672
Arg Ala Ala Ala Asp Asn Leu Val Pro Val Thr Leu Glu Leu Gly Gly
345                 350                 355                 360

AAA TCG CCG GTG ATC GTT TCC CGC AGT GCA GAT ATG GCG GAC GTT GCA        720
Lys Ser Pro Val Ile Val Ser Arg Ser Ala Asp Met Ala Asp Val Ala
                365                 370                 375

CAA CGG GTG TTG ACG GTG AAA ACC TTC AAT GCC GGG CAA ATC TGT CTG        768
Gln Arg Val Leu Thr Val Lys Thr Phe Asn Ala Gly Gln Ile Cys Leu
            380                 385                 390

GCA CCG GAC TAT GTG CTG CTG CCG GAA GAA TCG CTG GAT AGC TTT GTC        816
Ala Pro Asp Tyr Val Leu Leu Pro Glu Glu Ser Leu Asp Ser Phe Val
        395                 400                 405

GCC GAG GCG ACG CGC TTC GTG GCC GCA ATG TAT CCC TCG CTT CTA GAT        864
Ala Glu Ala Thr Arg Phe Val Ala Ala Met Tyr Pro Ser Leu Leu Asp
    410                 415                 420

AAT CCG GAT TAC ACG TCG ATC ATC AAT GCC CGA AAT TTC GAC CGT CTG        912
Asn Pro Asp Tyr Thr Ser Ile Ile Asn Ala Arg Asn Phe Asp Arg Leu
425                 430                 435                 440

CAT CGC TAC CTG ACT GAT GCG CAG GCA AAG GGA GGG CGC GTC ATT GAA        960
His Arg Tyr Leu Thr Asp Ala Gln Ala Lys Gly Gly Arg Val Ile Glu
                445                 450                 455

ATC AAT CCT GCG GCC GAA GAG TTG GGG GAT AGT GGT ATC AGG AAG ATC       1008
Ile Asn Pro Ala Ala Glu Glu Leu Gly Asp Ser Gly Ile Arg Lys Ile
            460                 465                 470

GCG CCC ACT TTG ATC GTG AAT GTG TCG GAT GAA ATG CTG GTC TTG AAC       1056
Ala Pro Thr Leu Ile Val Asn Val Ser Asp Glu Met Leu Val Leu Asn
        475                 480                 485

GAG GAG ATC TTT GGT CCG CTG CTC CCG ATC AAG ACT TAT CGT GAT TTC       1104
Glu Glu Ile Phe Gly Pro Leu Leu Pro Ile Lys Thr Tyr Arg Asp Phe
    490                 495                 500

GAC TCG GCT ATC GAC TAC GTC AAC AGC AAG CAG CGA CCA CTT GCC TCG       1152
Asp Ser Ala Ile Asp Tyr Val Asn Ser Lys Gln Arg Pro Leu Ala Ser
505                 510                 515                 520

TAC TTC TTC GGC GAA GAT GCG GTT GAG CGT GAG CAA GTG CTT AAG CGT       1200
Tyr Phe Phe Gly Glu Asp Ala Val Glu Arg Glu Gln Val Leu Lys Arg
                525                 530                 535

ACG GTT TCG GGC GCC GTG GTC GTG AAC GAT GTC ATG AGC CAT GTG ATG       1248
Thr Val Ser Gly Ala Val Val Val Asn Asp Val Met Ser His Val Met
            540                 545                 550

ATG GAT ACG CTT CCA TTT GGT GGT GTG GGG CAC TCG GGG ATG GGG GCA       1296

Met Asp Thr Leu Pro Phe Gly Gly Val Gly His Ser Gly Met Gly Ala
          555                 560                 565

TAT CAC GGC ATT TAT GGT TTC CGA ACC TTC AGC CAT GCC AAG CCT GTT      1344
Tyr His Gly Ile Tyr Gly Phe Arg Thr Phe Ser His Ala Lys Pro Val
    570                 575                 580

CTC GTG CAA AGT CCT GTG GGT GAG TCG AAC TTG GCG ATG CGC GCA CCC      1392
Leu Val Gln Ser Pro Val Gly Glu Ser Asn Leu Ala Met Arg Ala Pro
585                 590                 595                 600

TAC GGA GAA GCG ATC CAC GGA CTG CTC TCT GTC CTC CTT TCA ACG GAG      1440
Tyr Gly Glu Ala Ile His Gly Leu Leu Ser Val Leu Leu Ser Thr Glu
                605                 610                 615

TGT TAG                                                              1446
Cys

(2) ANGABEN ZU SEQ ID NO: 38:

        (i) SEQUENZKENNZEICHEN:
           (A) LÄNGE: 481 Aminosäuren
           (B) ART: Aminosäure
           (D) TOPOLOGIE: linear

     (ii) ART DES MOLEKÜLS: Protein
     (xi) SEQUENZBESCHREIBUNG: SEQ ID NO: 38:

Met Ser Ile Leu Gly Leu Asn Gly Ala Pro Val Gly Ala Glu Gln Leu
  1               5                  10                  15

Gly Ser Ala Leu Asp Arg Met Lys Lys Ala His Leu Glu Gln Gly Pro
             20                  25                  30

Ala Asn Leu Glu Leu Arg Leu Ser Arg Leu Asp Arg Ala Ile Ala Met
         35                  40                  45

Leu Leu Glu Asn Arg Glu Ala Ile Ala Asp Ala Val Ser Ala Asp Phe
     50                  55                  60

Gly Asn Arg Ser Arg Glu Gln Thr Leu Leu Cys Asp Ile Ala Gly Ser
 65                  70                  75                  80

Val Ala Ser Leu Lys Asp Ser Arg Glu His Val Ala Lys Trp Met Glu
                 85                  90                  95

Pro Glu His His Lys Ala Met Phe Pro Gly Ala Glu Ala Arg Val Glu
            100                 105                 110

Phe Gln Pro Leu Gly Val Val Gly Val Ile Ser Pro Trp Asn Phe Pro
        115                 120                 125

Ile Val Leu Ala Phe Gly Pro Leu Ala Gly Ile Phe Ala Ala Gly Asn

130                 135                 140

Arg Ala Met Leu Lys Pro Ser Glu Leu Thr Pro Arg Thr Ser Ala Leu
145                 150                 155                 160

Leu Ala Glu Leu Ile Ala Arg Tyr Phe Asp Glu Thr Glu Leu Thr Thr
                165                 170                 175

Val Leu Gly Asp Ala Glu Val Gly Ala Leu Phe Ser Ala Gln Pro Phe
            180                 185                 190

Asp His Leu Ile Phe Thr Gly Gly Thr Ala Val Ala Lys His Ile Met
        195                 200                 205

Arg Ala Ala Ala Asp Asn Leu Val Pro Val Thr Leu Glu Leu Gly Gly
    210                 215                 220

Lys Ser Pro Val Ile Val Ser Arg Ser Ala Asp Met Ala Asp Val Ala
225                 230                 235                 240

Gln Arg Val Leu Thr Val Lys Thr Phe Asn Ala Gly Gln Ile Cys Leu
                245                 250                 255

Ala Pro Asp Tyr Val Leu Leu Pro Glu Glu Ser Leu Asp Ser Phe Val
            260                 265                 270

Ala Glu Ala Thr Arg Phe Val Ala Ala Met Tyr Pro Ser Leu Leu Asp
        275                 280                 285

Asn Pro Asp Tyr Thr Ser Ile Ile Asn Ala Arg Asn Phe Asp Arg Leu
    290                 295                 300

His Arg Tyr Leu Thr Asp Ala Gln Ala Lys Gly Gly Arg Val Ile Glu
305                 310                 315                 320

Ile Asn Pro Ala Ala Glu Glu Leu Gly Asp Ser Gly Ile Arg Lys Ile
                325                 330                 335

Ala Pro Thr Leu Ile Val Asn Val Ser Asp Glu Met Leu Val Leu Asn
            340                 345                 350

Glu Glu Ile Phe Gly Pro Leu Leu Pro Ile Lys Thr Tyr Arg Asp Phe
        355                 360                 365

Asp Ser Ala Ile Asp Tyr Val Asn Ser Lys Gln Arg Pro Leu Ala Ser
    370                 375                 380

Tyr Phe Phe Gly Glu Asp Ala Val Glu Arg Glu Gln Val Leu Lys Arg
385                 390                 395                 400

Thr Val Ser Gly Ala Val Val Val Asn Asp Val Met Ser His Val Met
                405                 410                 415

Met Asp Thr Leu Pro Phe Gly Gly Val Gly His Ser Gly Met Gly Ala
            420                 425                 430

Tyr His Gly Ile Tyr Gly Phe Arg Thr Phe Ser His Ala Lys Pro Val
        435                 440                 445

Leu Val Gln Ser Pro Val Gly Glu Ser Asn Leu Ala Met Arg Ala Pro
    450                 455                 460

Tyr Gly Glu Ala Ile His Gly Leu Leu Ser Val Leu Leu Ser Thr Glu
465                 470                 475                 480

Cys

(2) ANGABEN ZU SEQ ID NO: 39:

(i) SEQUENZKENNZEICHEN:
(A) LÄNGE: 1827 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:
(A) NAME/SCHLÜSSEL: CDS
(B) LAGE:complement (4..1827)
(D) SONSTIGE ANGABEN:/product=
"Transkriptions-Aktivator-Protein"
/gene= "tap"

(xi) SEQUENZBESCHREIBUNG: SEQ ID NO: 39:

CTATTTGTCT AGTGGTCGGC GCGAAATTCG ATAAGAAAGC TGGGCGCGAG TGAGGCCGAG      60

CCGGCGGGCA GCTTCCGAGA CATTGCCTTT CACCTGGCCC AGAGCATGGC TAATCATCGC     120

GTCCTCCACT TCTTGCAGCG TCATCGCGCT CAGGTCCTTT GAGTCAAGCG GCGAGTCGAT     180

TGTGCTGGTC GGTTTGGAGA AGGAAGTACT TGGGCTGCCA GTTTCCTGTG GCTGATTATC     240

TTGAGCGGTG GCCAGGATGC CGCTGGCCCC AATGGAGAAC ATCGGTTGAG TCAGTCGTTC     300

ACCGCTAGTG AAGAGGTGGC TCACGTCAAT GGCTCCATCC TCCGGAGCGC TGATGACTCC     360

GCGCTCCACC AAATTTTGAA GCTCCCGGAT GTTTCCTGGA AAGTCGTAGC CAAGCAGGGC     420

ATTGGCTGCA CGTGGAGTGA ATCCGCTGAC CACCCGGCTA TGACGCTGAT TGAAGCGGTG     480

CAGGAAATAG GTCATCAGGA GGGGAATGTC TTCCTTCCTC TCTCGAAGCG GCGGGAGGTG     540

GATCGGGTAA ACATTGAGGC GGAAAAAAAG GTCCTCGCGG AACTCGCCGC GCTGGACGCC 600

TGCGCGAAGA TCGACATTGG TTGCGGCTAC CACACGGACG TCAACCTTGA GTGTCCTGCT 660

TCCGCCAACC CGTTCGACCT CCGACTCTTG CAGGGCGCGA AGTAACTTCC CTTGGGCCAC 720

GAGGCTTAGC GTCCCTATCT CGTCAAGGAA TAGTGTGCCG CCCGAAGCGC GCTCGAACCG 780

TCCTGCTCGA GATTGGGTGG CGCCGGTAAA CGCCCCCCGT TCGACGCCGA ACAACTCGGA 840

CTCCATCAGG GTTTCGGGAA TACGTGCGCA ATTGACCGCA ACAAACGGGC CGTCGTGTCT 900

GGGGCTGATG CGGTGAAGCA TGCGGGCGAA CATCTCCTTG CCCACACCTG ATTCACCCGT 960

AAACAGTACC GTCGCCTCCG TGGGTGCTAC GCGCTTCAGC ATGTGGCAGG CAGCATTGAA 1020

TGCCGAGGAA ATTCCCACCA TGTCGTGTTC CGATGCAGTG CTTGAGTCTG CGGCGGAGTG 1080

ATGGGGAGTG TTCCTTTGTC CCTGCTGCGT TCTTCGTCTC TGCGGCGTGC TTGGTTGCCG 1140

ACAAATGGTT GCGCTAAGCG CCGCCAAGTC CTCTTCGGCG TCTTCCCATT CTTCCGCTGG 1200

CTTGCCGATC ATGCGGCAGA TCTGCGAACC CGTGGAGCGG CATTCCACCT CTCGGTAAAG 1260

GATGAGGCGA CCAACCAGCG CGGACGTATA GCCAATGGCA TAACCCGTCT GCGTCCAGCA 1320

CGCGGGCTCG GTGCCGATGC CGTAGTGCGC AATATGTTCA TCATCTTCGC TCGAATGGTG 1380

CCAGAGGAAT TCGCCGTAGT AGGTCCCCAA ATCCATGTCG AAGTCGAAGT GGATCGGCTC 1440

CACGCGTACT GCGCCTTCCA GAGAGTGCAA GTTCGGGCCG GCGGCAAATA GGGAGAGCGG 1500

ATCGGCGTTG CTGAAGCGCT CCTTCAGAAG GGCGGCATCT TTGGCGCCGC AGTGGTAACC 1560

GGTTCGCAGC ATGATTCCGC GGGCGCGGGC GAAGCCCACG CTTTCAATTA ATTCGCGTCG 1620

CAATGCACCC AGTCCGCTGC TGTGGAGGAG CAGCATTCGC GCGCCGTTCA ACCAGATGCG 1680

TCCATCGCCA GGGCTGAAAA GGAGGGATTC AGTGAGGTCA TGAAGGGAGG GGACGGCGCC 1740

TGGCTCCAAT TGCTCGATGG CGCCGCGATT GAGTGTCTTG GGCGCGGTCT TGGAGAGTTC 1800

GGCTAGGGAG ATAAATTTGC TGGCCAT 1827

(2) ANGABEN ZU SEQ ID NO: 40:

(i) SEQUENZKENNZEICHEN:
(A) LÄNGE: 608 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 40:

Met Ala Ser Lys Phe Ile Ser Leu Ala Glu Leu Ser Lys Thr Ala Pro
1               5                   10                  15

Lys Thr Leu Asn Arg Gly Ala Ile Glu Gln Leu Glu Pro Gly Ala Val
            20                  25                  30

Pro Ser Leu His Asp Leu Thr Glu Ser Leu Leu Phe Ser Pro Gly Asp
        35                  40                  45

Gly Arg Ile Trp Leu Asn Gly Ala Arg Met Leu Leu Leu His Ser Ser
    50                  55                  60

Gly Leu Gly Ala Leu Arg Arg Glu Leu Ile Glu Ser Val Gly Phe Ala
65                  70                  75                  80

Arg Ala Arg Gly Ile Met Leu Arg Thr Gly Tyr His Cys Gly Ala Lys
                85                  90                  95

Asp Ala Ala Leu Leu Lys Glu Arg Phe Ser Asn Ala Asp Pro Leu Ser
            100                 105                 110

Leu Phe Ala Ala Gly Pro Asn Leu His Ser Leu Glu Gly Ala Val Arg
        115                 120                 125

Val Glu Pro Ile His Phe Asp Phe Asp Met Asp Leu Gly Thr Tyr Tyr
    130                 135                 140

Gly Glu Phe Leu Trp His His Ser Ser Glu Asp Asp Glu His Ile Ala
145                 150                 155                 160

His Tyr Gly Ile Gly Thr Glu Pro Ala Cys Trp Thr Gln Thr Gly Tyr
                165                 170                 175

Ala Ile Gly Tyr Thr Ser Ala Leu Val Gly Arg Leu Ile Leu Tyr Arg
            180                 185                 190

Glu Val Glu Cys Arg Ser Thr Gly Ser Gln Ile Cys Arg Met Ile Gly
        195                 200                 205

Lys Pro Ala Glu Glu Trp Glu Asp Ala Glu Glu Asp Leu Ala Ala Leu
    210                 215                 220

Ser Ala Thr Ile Cys Arg Gln Pro Ser Thr Pro Gln Arg Arg Arg Thr
225                 230                 235                 240

Gln Gln Gly Gln Arg Asn Thr Pro His His Ser Ala Ala Asp Ser Ser
                245                 250                 255

Thr Ala Ser Glu His Asp Met Val Gly Ile Ser Ser Ala Phe Asn Ala
            260                 265                 270

Ala Cys His Met Leu Lys Arg Val Ala Pro Thr Glu Ala Thr Val Leu
        275                 280                 285

Phe Thr Gly Glu Ser Gly Val Gly Lys Glu Met Phe Ala Arg Met Leu

290                 295                 300

His Arg Ile Ser Pro Arg His Asp Gly Pro Phe Val Ala Val Asn Cys
305                 310                 315                 320

Ala Arg Ile Pro Glu Thr Leu Met Glu Ser Glu Leu Phe Gly Val Glu
                325                 330                 335

Arg Gly Ala Phe Thr Gly Ala Thr Gln Ser Arg Ala Gly Arg Phe Glu
            340                 345                 350

Arg Ala Ser Gly Gly Thr Leu Phe Leu Asp Glu Ile Gly Thr Leu Ser
        355                 360                 365

Leu Val Ala Gln Gly Lys Leu Leu Arg Ala Leu Gln Glu Ser Glu Val
    370                 375                 380

Glu Arg Val Gly Gly Ser Arg Thr Leu Lys Val Asp Val Arg Val Val
385                 390                 395                 400

Ala Ala Thr Asn Val Asp Leu Arg Ala Gly Val Gln Arg Gly Glu Phe
                405                 410                 415

Arg Glu Asp Leu Phe Phe Arg Leu Asn Val Tyr Pro Ile His Leu Pro
            420                 425                 430

Pro Leu Arg Glu Arg Lys Glu Asp Ile Pro Leu Leu Met Thr Tyr Phe
        435                 440                 445

Leu His Arg Phe Asn Gln Arg His Ser Arg Val Val Ser Gly Phe Thr
    450                 455                 460

Pro Arg Ala Ala Asn Ala Leu Leu Gly Tyr Asp Phe Pro Gly Asn Ile
465                 470                 475                 480

Arg Glu Leu Gln Asn Leu Val Glu Arg Gly Val Ile Ser Ala Pro Glu
                485                 490                 495

Asp Gly Ala Ile Asp Val Ser His Leu Phe Thr Ser Gly Glu Arg Leu
            500                 505                 510

Thr Gln Pro Met Phe Ser Ile Gly Ala Ser Gly Ile Leu Ala Thr Ala
        515                 520                 525

Gln Asp Asn Gln Pro Gln Glu Thr Gly Ser Pro Ser Thr Ser Phe Ser
    530                 535                 540

Lys Pro Thr Ser Thr Ile Asp Ser Pro Leu Asp Ser Lys Asp Leu Ser
545                 550                 555                 560

Ala Met Thr Leu Gln Glu Val Glu Asp Ala Met Ile Ser His Ala Leu
                565                 570                 575

Gly Gln Val Lys Gly Asn Val Ser Glu Ala Ala Arg Arg Leu Gly Leu
            580                 585                 590

Thr Arg Ala Gln Leu Ser Tyr Arg Ile Ser Arg Arg Pro Leu Asp Lys
        595                 600                 605

(2) ANGABEN ZU SEQ ID NO: 41:

    (i) SEQUENZKENNZEICHEN:
        (A) LÄNGE: 768 Basenpaare
        (B) ART: Nucleotid
        (C) STRANGFORM: Doppelstrang
        (D) TOPOLOGIE: linear

    (ii) ART DES MOLEKÜLS: Genom-DNA

    (iii) HYPOTHETISCH: NEIN

    (iv) ANTISENSE: NEIN

    (ix) MERKMAL:
        (A) NAME/SCHLÜSSEL: CDS
        (B) LAGE:1..765
        (D) SONSTIGE ANGABEN:/product=
            "Coniferylalkohol-Dehydrogenase"
            /gene= "cadh"

    (xi) SEQUENZBESCHREIBUNG: SEQ ID NO: 41:

ATG CAA CTG ACC AAC AAG AAA ATC GTC GTC ACC GGA GTG TCC TCC GGT      48
Met Gln Leu Thr Asn Lys Lys Ile Val Val Thr Gly Val Ser Ser Gly
    610                 615                 620

ATC GGT GCC GAA ACT GCC CGC GTT CTG CGC TCT CAC GGC GCC ACA GTG      96
Ile Gly Ala Glu Thr Ala Arg Val Leu Arg Ser His Gly Ala Thr Val
625                 630                 635                 640

ATT GGC GTA GAT CGC AAC ATG CCG AGC CTG ACT CTG GAT GCT TTC GTT     144
Ile Gly Val Asp Arg Asn Met Pro Ser Leu Thr Leu Asp Ala Phe Val
                645                 650                 655

CAG GCT GAC CTG AGC CAT CCT GAA GGC ATC GAT AAG GCC ATC TCT CAG     192
Gln Ala Asp Leu Ser His Pro Glu Gly Ile Asp Lys Ala Ile Ser Gln
            660                 665                 670

CTG CCG GAG AAA ATT GAC GGA CTC TGC AAT ATC GCC GGG GTG CCC GGC     240
Leu Pro Glu Lys Ile Asp Gly Leu Cys Asn Ile Ala Gly Val Pro Gly
        675                 680                 685

ACT GCC GAT CCT CAG CTC GTC GCA AAC GTG AAC TAC CTG GGT CTA AAG     288
Thr Ala Asp Pro Gln Leu Val Ala Asn Val Asn Tyr Leu Gly Leu Lys
    690                 695                 700

TAT CTG ACC GAG GCA GTC CTG TCG CGC ATT CAA CCC GGT GGT TCG ATT     336
Tyr Leu Thr Glu Ala Val Leu Ser Arg Ile Gln Pro Gly Gly Ser Ile

705                 710                 715                 720

GTC AAC GTG TCC TCT GTG CTT GGC GCC GAG TGG CCG GCC CGC CTT CAG        384
Val Asn Val Ser Ser Val Leu Gly Ala Glu Trp Pro Ala Arg Leu Gln
                725                 730                 735

TTG CAT AAG GAG CTG GGG AGT GTT GTT GGA TTC TCC GAA GGC CAG GCA        432
Leu His Lys Glu Leu Gly Ser Val Val Gly Phe Ser Glu Gly Gln Ala
            740                 745                 750

TGG CTT AAG CAG AAT CCA GTG GCC CCC GAA TTC TGC TAC CAG TAT TTC        480
Trp Leu Lys Gln Asn Pro Val Ala Pro Glu Phe Cys Tyr Gln Tyr Phe
        755                 760                 765

AAA GAA GCA CTG ATC GTT TGG TCT CAA GTT CAG GCG CAG GAA TGG TTC        528
Lys Glu Ala Leu Ile Val Trp Ser Gln Val Gln Ala Gln Glu Trp Phe
    770                 775                 780

ATG AGG ACG TCT GTA CGC ATG AAC TGC ATC GCC CCC GGC CCT GTA TTC        576
Met Arg Thr Ser Val Arg Met Asn Cys Ile Ala Pro Gly Pro Val Phe
785                 790                 795                 800

ACT CCC ATT CTC AAT GAG TTC GTC ACC ATG CTG GGT CAA GAG CGG ACT        624
Thr Pro Ile Leu Asn Glu Phe Val Thr Met Leu Gly Gln Glu Arg Thr
                805                 810                 815

CAG GCG GAC GCT CAT CGT ATT AAG CGC CCA GCA TAT GCC GAT GAA GTG        672
Gln Ala Asp Ala His Arg Ile Lys Arg Pro Ala Tyr Ala Asp Glu Val
            820                 825                 830

GCC GCG GTG ATT GCA TTC ATG TGT GCT GAG GAG TCA CGT TGG ATC AAC        720
Ala Ala Val Ile Ala Phe Met Cys Ala Glu Glu Ser Arg Trp Ile Asn
        835                 840                 845

GGC ATA AAT ATT CCA GTG GAC GGA GGT TTG GCA TCG ACC TAC GTG            765
Gly Ile Asn Ile Pro Val Asp Gly Gly Leu Ala Ser Thr Tyr Val
    850                 855                 860

TAA                                                                    768

(2) ANGABEN ZU SEQ ID NO: 42:

(i) SEQUENZKENNZEICHEN:
(A) LÄNGE: 255 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 42:

Met Gln Leu Thr Asn Lys Lys Ile Val Val Thr Gly Val Ser Ser Gly
  1               5                  10                  15

Ile Gly Ala Glu Thr Ala Arg Val Leu Arg Ser His Gly Ala Thr Val

            20                  25                  30

Ile Gly Val Asp Arg Asn Met Pro Ser Leu Thr Leu Asp Ala Phe Val
        35                  40                  45

Gln Ala Asp Leu Ser His Pro Glu Gly Ile Asp Lys Ala Ile Ser Gln
    50                  55                  60

Leu Pro Glu Lys Ile Asp Gly Leu Cys Asn Ile Ala Gly Val Pro Gly
65                  70                  75                  80

Thr Ala Asp Pro Gln Leu Val Ala Asn Val Asn Tyr Leu Gly Leu Lys
                85                  90                  95

Tyr Leu Thr Glu Ala Val Leu Ser Arg Ile Gln Pro Gly Gly Ser Ile
            100                 105                 110

Val Asn Val Ser Ser Val Leu Gly Ala Glu Trp Pro Ala Arg Leu Gln
        115                 120                 125

Leu His Lys Glu Leu Gly Ser Val Val Gly Phe Ser Glu Gly Gln Ala
    130                 135                 140

Trp Leu Lys Gln Asn Pro Val Ala Pro Glu Phe Cys Tyr Gln Tyr Phe
145                 150                 155                 160

Lys Glu Ala Leu Ile Val Trp Ser Gln Val Gln Ala Gln Glu Trp Phe
                165                 170                 175

Met Arg Thr Ser Val Arg Met Asn Cys Ile Ala Pro Gly Pro Val Phe
            180                 185                 190

Thr Pro Ile Leu Asn Glu Phe Val Thr Met Leu Gly Gln Glu Arg Thr
        195                 200                 205

Gln Ala Asp Ala His Arg Ile Lys Arg Pro Ala Tyr Ala Asp Glu Val
    210                 215                 220

Ala Ala Val Ile Ala Phe Met Cys Ala Glu Glu Ser Arg Trp Ile Asn
225                 230                 235                 240

Gly Ile Asn Ile Pro Val Asp Gly Gly Leu Ala Ser Thr Tyr Val
                245                 250                 255

Anlage zum Sequenzprotokoll

SEQ ID NO | 2: | ORF 1
| 4: | Van A
| 6: | Van B
| 8: | FDH
| 10: | GCS
| 12: | CytC
| 14: | ORF 5
| 16: | Ehy B
| 18: | ORF 2
| 20: | ADH
| 22: | LSD
| 24: | ORF 3
| 26: | Ech
| 28: | VDH
| 30: | FCS
| 32: | Aat
| 34: | Mac
| 36: | Trp
| 38: | Caldh
| 40: | Tap
| 42: | Cadh

## Patentansprüche

1. Syntheseenzyme für Coniferylalkohol, Coniferylaldehyd, Ferulasäure, Vanillin und Vanillinsäure aus Eugenol.

2. Syntheseenzyme gemäß Anspruch 1 ausgewählt aus der Gruppe
a) Eugenol-Hydroxylase
b) Coniferylalkohol-Dehydrogenase
c) Coniferylaldehyd-Dehydrogenase
d) Ferulasäuredeacylase.
e) Vanillin-Dehydrogenase

3. DNA, codierend für die Enzyme gemäß Anspruch 1 und 2 sowie Teilsequenzen und funktionelle Äquivalente davon.

4. Cosmidklone, enthaltend die DNA gemäß Anspruch 3.

5. Vektoren, enthaltend DNA nach Anspruch 3.

6. Mikroorganismen, transformiert mit DNA gemäß Anspruch 3.

7. Verwendung von DNA nach Anspruch 3 zur Transformation von Mikroorganismen.

8. Verwendung von Mikroorganismen gemäß Anspruch 6 zur Herstellung von Coniferylalkohol, Coniferylaldehyd, Ferulasäure, Vanillin und Vanillinsäure.

9. Verfahren zur Herstellung von Coniferylalkohol aus Eugenol, dadurch gekennzeichnet, daß die Reaktion in Anwesenheit von Eugenol-Hydroxylase stattfindet.

10. Verfahren zur Herstellung von Coniferylaldehyd aus Coniferylalkohol, dadurch gekennzeichnet, daß die Reaktion in Anwesenheit von Coniferylalkohol-Dehydrogenase stattfindet.

11. Verfahren zur Herstellung von Ferulasäure aus Coniferylaldehyd, dadurch gekennzeichnet, daß die Reaktion in Anwesenheit von Coniferylaldehyd-Dehydrogenase stattfindet.

12. Verfahren zur Herstellung von Vanillin aus Ferulasäure, dadurch gekennzeichnet, daß die Reaktion in Anwesenheit von Ferulasäuredeacylase stattfindet.

13. Verfahren zur Herstellung von Vanillinsäure aus Vanillin, dadurch gekennzeichnet, daß die Reaktion in Anwesenheit von Vanillin-Dehydrogenase stattfindet.
